# EUROPEAN PATENT APPLICATION

(11) **EP 4 253 530 A1**
(43) Date of publication of application: **04.10.2023**
(21) Application number: 21897071.3
(22) Date of filing: 25.11.2021
(51) Int. Cl.: C12N 5/0783, C12N 5/09

(54) **TUMOR INFILTRATION LYMPHOCYTE CULTURE MEDIUM AND APPLICATION THEREOF**

(30) Priority: 25.11.2020 CN 202011339183
(71) Applicant: Shanghai Juncell Therapeutics Co., Ltd., Shanghai 201800 (CN)
(72) Inventor: JIN, Huajun, Shanghai 201800 (CN); HE, Zhou, Shanghai 201800 (CN); LI, Tiantian, Shanghai 201800 (CN); SHEN, Yongchao, Shanghai 201800 (CN); CHEN, Haibin, Shanghai 201800 (CN); XU, Fuhui, Shanghai 201800 (CN); HUANG, Chen, Shanghai 201800 (CN)
(74) Representative: Nederlandsch Octrooibureau
(86) International application number: PCT/CN2021/133059
(87) International publication number: WO 2022/111571

(57) **Abstract**

A culture composition for expanding a TIL, containing IL-2, IL-7, IL-15, and an immune checkpoint antibody or an antigen-binding fragment thereof. The immune checkpoint antibody comprises any one or more of a PD-1 antibody, a CTLA-4 antibody, a LAG-3 antibody, a TIM-3 antibody, a TIGIT antibody, and a BTLA antibody. The present invention further relates to a TIL amplification culture medium, a method for preparing a TIL population, and a pharmaceutical composition. In the culture composition, cell proliferation and activity are ensured while greatly reducing the use amount of IL-2, and IL-2 treatment is removed after infusing the culture composition to a patient so as to reduce side effects; moreover, no feeder cells are used in an amplification process, exogenous pollution is reduced and a production process is simplified, production costs are reduced, and the production time is shortened.

## Description

### Technical Field

The invention relates to the field of biotechnology, in particular to a tumor infiltrating lymphocyte culture medium and application thereof.

### Background

Tumor immunotherapy has become the mainstream of cancer treatment in the present day due to the progress in the field of tumor immunotherapy. In recent years, various strategies to stimulate and enhance anti-tumor immunity have been developed and evaluated in different clinical studies. Immunotherapy for malignant tumors has achieved remarkable clinical efficacy in recent years, mainly including immune checkpoint (such as CTLA4, PD1/PDL1) monoclonal antibody therapy, chimeric antigen receptor T (CAR-T) cell therapy, TCR-T therapy etc. Significant curative effect of hematological tumor immunotherapy has been achieved by CAR-T cell therapy, but for solid tumor immunotherapy, CAR-T, TCR-T and/or immune checkpoint inhibitor therapy are all encounter difficulties of varying degrees.

In 1998, the Surgery Branch of the National Cancer Institute (NCI) reported the first case in which tumor-infiltrating lymphocytes (TIL) were used for treatment. Extensive studies on TILs have shown that cells with antitumor activity can be isolated from tumor tissues of melanoma patients. In recent years, it has been found that TIL has a very significant effect on tumors, especially solid tumors, which are bulky and refractory for adoptive transfer therapy (ACT). Iovance Therapeutics company from the United States currently holds a number of TIL cell drugs that have entered clinical phase II or pivotal phase III, and the indications of its TIL drugs include melanoma, cervical cancer, head and neck cancer, non-small cell lung cancer, ovarian cancer, colon cancer, pancreatic cancer and sarcoma. Among these drugs, LN145 obtained the fast track designation from the FDA for the indication of cervical cancer in 2019 and was identified as a breakthrough therapy for the treatment of advanced cervical cancer that progressed during or after chemotherapy.

The use of TIL in tumor immunotherapy requires a large number of cells, which poses a great challenge to the technical and process control of TIL expansion. A common practice in the past was to use an IL-2-based pre-Rapid Expansion Procedure (pre-REP) followed by a "Rapid Expansion Procedure" (REP). REP was able to further expand TILs at 1000-fold within 14 days, a process that required a large excess of irradiated allogeneic PBMCs (also known as mononuclear cells, MNCs) derived from multiple donors as feeder cells, anti-CD3 antibody (OKT3) and high-dose of IL-2. TILs that have undergone the REP process can successfully be used in immunological adoptive therapy (ACT) in melanoma patients after they have received immunosuppressive therapy. On the basis of the aforementioned TIL expansion culture method, WO2018182817A1 and WO2019190579A1 disclosed an improved TIL manufacturing method suitable for large-scale commercial production and compliant with pharmaceutical regulations, and this method can produce TIL at the order of magnitude of 10¹¹, and shorten the culture time. However, this culture method still requires irradiated allogeneic PBMCs derived from multiple donors as feeder cells. Feeder cells cause potential contamination risks, and the preparation of GMP-level feeder cells also complicates of the entire TIL preparation process. Moreover, a relatively high concentration of IL-2 (6000 IU/mL, 3000 IU/mL) is still needed in this method, and there may be a risk of cell exhaustion in the later infusion.

Therefore, there is an urgent need in the field for a safer and more efficient TIL cell expansion system in vitro.

### Summary

Based on the current state of the prior art, the present invention develops a safer and more efficient *in vitro* TIL expansion system by optimizing the components of the culture medium, and expands the scope of its clinical application.

One aspect of the present invention provides a culture composition for expanding TILs, comprising IL-2, IL-7, IL-15 and an immune checkpoint antibody or an antigen-binding fragment thereof, preferably, the immune checkpoint antibody including PD-1 antibody.

In one or more embodiments, the culture composition further comprises any one, two, three or four selected from the group consisting of: IL-21, IL-12, GM-CSF, and small molecules that increase the proportion of memory T cells. Preferably, the culture composition further comprises IL-21 and IL-12; or IL-21, IL-12 and GM-CSF; or GM-CSF and small molecules that increase the proportion of memory T cells. Preferably, the small molecule that increases the proportion of memory T cells is TWS119.

In one or more embodiments, the immune checkpoint antibody comprises any one or more selected from the group consisting of: a PD-1 antibody, a CTLA-4 antibody, a LAG3 antibody, a TIM3 antibody, a TIGIT antibody and a BTLA antibody.

In one or more embodiments, the culture composition further comprises any one or more selected from the group consisting of: a CD3 antibody, a CD28 antibody, a CD137 antibody, a OX40 antibody, a GITR antibody, a ICOS antibody, a CD206 antibody and a CD40 antibody. Preferably, the culture composition further comprises any one, two, three or four selected from the group consisting of a CD3 antibody, a CD28 antibody, a CD137 antibody and a GITR antibody. For example, the culture composition further comprises (1) a CD3 antibody and a CD28 antibody, or (2) a CD3 antibody, a CD28 antibody, a CD137 antibody and a GITR antibody.

In one or more embodiments, the CD3 antibody, the CD28 antibody, the CD137 antibody, the OX40 antibody, the GITR antibody, the ICOS antibody, the CD206 antibody and the CD40 antibody are activating antibodies

In one or more embodiments, the culture composition further comprises autologous platelets or allogeneic platelets.

In one or more embodiments, each antibody is a monoclonal antibody (mAb).

In one or more embodiments, the one or more antibodies are coupled to a matrix. In one or more embodiments, the matrix is a solid matrix, such as a microsphere, a well-plate, a dish, a cell culture flask, or a cell culture bag. Preferably, the microsphere is a magnetic a bead. Preferably, said coupling is the coupling via a covalent bond or a non-covalent bond. In one or more embodiments, the culture composition comprises IL-2, IL-7, IL-15, a PD-1 antibody, IL-21, IL-12, a CD3 antibody, a CD28 antibody, and GM-CSF.

In one or more embodiments, the culture composition comprises IL-2, IL-7, IL-15, PD-1 antibody, IL-21, IL-12, a CD3 antibody, a CD28 antibody, a CD137 antibody and a GITR antibody.

In one or more embodiments, the culture composition comprises IL-2, IL-7, IL-15, a PD-1 antibody, a CD3 antibody and a CD28 antibody. Preferably, said CD3 antibody and CD28 antibody are coupled to a matrix.

In one or more embodiments, the culture composition comprises IL-2, IL-7, IL-15, a PD-1 antibody, IL-21, IL-12, a CD3 antibody, a CD28 antibody and GM-CSF. Preferably, said CD3 antibody and CD28 antibody are coupled to a matrix.

In one or more embodiments, the culture composition comprises IL-2, IL-7, IL-15, a PD-1 antibody, a CD3 antibody, a CD28 antibody and a CD137 antibody. Preferably, said CD3 antibody, CD137 antibody and CD28 antibody are coupled to a matrix.

In one or more embodiments, the culture composition comprises IL-2, IL-7, IL-15, a PD-1 antibody, a GITR antibody, a CD3 antibody, a CD28 antibody, TWS119 and a CD137 antibody. Preferably, said CD3 antibody, CD137 antibody and CD28 antibody are coupled to a matrix.

In one or more embodiments, the culture composition comprises IL-2, IL-7, IL-15, a PD-1 antibody, a TIGIT antibody, a CD3 antibody, a CD28 antibody, a CD137 antibody and GM-CSF. Preferably, said CD3 antibody, CD137 antibody and CD28 antibody are coupled to a matrix.

In one or more embodiments, the culture composition comprises IL-2, IL-7, IL-15, LAG3 antibody, IL-21, IL-12, a CD3 antibody, a CD28 antibody, a CD137 antibody and a GITR antibody. Preferably, said CD3 antibody, CD137 antibody and CD28 antibody are coupled to a matrix.

In one or more embodiments, the culture composition comprises IL-2, IL-7, IL-15, a PD-1 antibody, a TIGIT antibody, a CD3 antibody, a CD28 antibody, a CD137 antibody, a CD40 antibody, a OX-40 antibody and autologous platelets. Preferably, said CD3 antibody, CD137 antibody and CD28 antibody are coupled to a matrix.

In one or more embodiments, the culture composition comprises IL-2, IL-7, IL-15, a PD-1 antibody, a CTLA-4 antibody, a CD3 antibody, a CD28 antibody, a CD137 antibody, a GITR antibody, GM-CSF, TWS119 and allogeneic platelets. Preferably, said CD3 antibody and CD28 antibody are coupled to a matrix.

In one or more of the above embodiments, the concentration of IL-2 in the culture composition is 200-6000 IU/mL, such as 200, 300, 400, 500, 600, 700, 800, 900, 1000, 1500, 2000, 2500, 3000, 3500, 4000, 4500, 5000, 5500, or 6000 IU/mL, or in a range between any two of these values. Preferably, the concentration of IL-2 in the culture composition is 200-950, 200-850, 200-750, 200-650, 200-550, or 200-500 IU/mL.

In one or more of the above embodiments, the concentration of IL-7 in the culture composition is 5-100 ng/mL, such as 5, 10, 20, 30, 40, 50, 60, 70, 80, 90, 100 ng/mL, or in a range between any two of these values. Preferably, the concentration of IL-7 in the culture composition is 5-65, 5-55, 5-45, 5-35, 5-25, or 5-15 ng/mL.

In one or more of the above embodiments, the concentration of IL-15 in the culture composition is 5-100 ng/mL, such as 5, 10, 20, 30, 40, 50, 60, 70, 80, 90, or 100 ng/mL, or in a range between any two of these values. Preferably, the concentration of IL-15 in the culture composition is 5-65, 5-55, 5-45, 5-35, 5-25, 5-15, or 10-50 ng/mL.

In one or more of the above embodiments, the concentration of PD-1 antibodies in the culture composition is 1-100 µg/mL, such as 1, 2, 3, 5, 7, 9, 10, 20, 30, 40, 50, 60, 70, 80, 90, 100 µg/mL, or in a range between any two of these values. Preferably, the concentration of the PD-1 antibodies in the culture composition is 1-65, 1-55, 1-45, 1-35, 1-25, 1-15, 1-8, 1-6, 1-4, or 1-3 µg/mL.

In one or more of the above embodiments, the concentration of IL-21 in the culture composition is 5-100 ng/mL, such as 5, 10, 20, 30, 40, 50, 60, 70, 80, 90, 100 ng/mL, or in a range between any two of these values. Preferably, the concentration of IL-21 in the culture composition is 5-65, 5-55, 5-45, 5-35, 5-25, or 5-15 ng/mL.

In one or more of the above embodiments, the concentration of IL-12 in the culture composition is 5-100 ng/mL, such as 5, 10, 20, 30, 40, 50, 60, 70, 80, 90, 100 ng/mL, or in a range between any two of these values. Preferably, the concentration of IL-12 in the culture composition is 5-65, 10-55, 10-45, 10-35, 10-25, 15-25, or 10-15 ng/mL.

In one or more of the above embodiments, the concentration of CD3 antibodies in the culture composition is 0.5-10 µg/mL, such as 0.5, 1, 2, 3, 4, 5, 6, 7, 8, 9, 10 µg/mL, or in a range between any two of these values. Preferably, the concentration of CD3 antibodies in the culture composition is 0.5-9.5, 0.5-8.5, 0.5-7.5, 0.5-6.5, 0.5-5.5, 0.5-3, 1.5-4.5, or 2.5-3.5 µg/mL.

In one or more of the above embodiments, the concentration of CD28 antibodies in the culture composition is 0.5-10 µg/mL, such as 0.5, 1, 2, 3, 4, 5, 6, 7, 8, 9, 10 µg/mL, or in a range between any two of these values. Preferably, the concentration of CD28 antibodies in the culture composition is 0.5-9.5, 0.5-8.5, 0.5-7.5, 0.5-6.5, 0.5-5.5, 0.5-1, 1.5-4.5, or 2.5-3.5 µg/mL.

In one or more of the above embodiments, the concentration of CD137 antibodies in the culture composition is 1-100 µg/mL, such as 1, 5, 10, 20, 30, 40, 50, 60, 70, 80, 90, 100 µg/mL, or in a range between any two of these values. Preferably, the concentration of the CD137 antibodies in the culture composition is 1-95, 1-85, 1-75, 1-65, 1-55, 1-45, 1-35, 1-25, 1-15, 1- 9, 1.5-8, 2-7, 2.5-6, 2.5-5, or 3-5 µg/mL.

In one or more of the above embodiments, the concentration of GITR antibodies in the culture composition is 1-100 µg/mL, such as 1, 5, 10, 20, 30, 40, 50, 60, 70, 80, 90, 100 µg/mL, or in a range between any two of these values. Preferably, the concentration of the GITR antibodies in the culture composition is 1-95, 1-85, 1-75, 1-65, 1-55, 1-45, 1-35, 1-25, 1-15, 1-9, 1.5-8, 2-7, 2.5-6, 3-5, or 2-3 µg/mL.

In one or more of the above embodiments, the concentration of GM-CSF in the culture composition is 200-5000 U/mL, such as 200, 300, 400, 500, 600, 700, 800, 900, 1000, 1500, 2000, 2500 , 3000, 3500, 4000, 4500, 5000 U/mL, or in a range between any two of these values. Preferably, the concentration of GM-CSF in the culture composition is 200-1500, 200-1200, 200-950, 200-850, 200-750, 200-650, 200-550, or 200-500 U/mL.

In one or more of the above embodiments, the concentration of TWS119 in the culture composition is 1-500 µM, such as 1, 5, 10, 15, 20, 30, 40, 50, 60, 70, 80, 90, 100, 200, 300, 400, 500 µM, or in a range between any two of these values. Preferably, the concentration of the TWS119 in the culture composition is 1-250, 2-150, 3-95, 4-85, 5-75, 6-65, 7-55, 8-45, 9-35, 10-25, or 10-15 µM.

In one or more of the above embodiments, the concentration of autologous platelets or allogeneic platelets in the culture composition is 0.5×10⁸/mL-1.0×10⁹/mL, such as 0.5×10⁸, 1.0×10⁸, 1.5×10⁸, 2.0×10⁸ , 2.5×10⁸, 3.0×10⁸, 3.5×10⁸, 4.0×10⁸, 4.5×10⁸, 5.0×10⁸, 5.5×10⁸, 6.0×10⁸, 6.5×10⁸, 7.0×10⁸, 7.5×10⁸, 8.0×10⁸, 8.5 ×10⁸, 9.0×10⁸, 9.5×10⁸, 1.0×10⁹/mL, or in a range between any two of these values. Preferably, the concentration of autologous platelets or allogeneic platelets in the culture composition is 0.5×10⁸-9.5×10⁸, 0.5×10⁸-7.5×10⁸, 0.5×10⁸-5.5×10⁸, 1.0×10⁸-5.5×10⁸, 1.0×10⁸-5.0×10⁸, 1.0×10⁸-4.5×10⁸, 1.0×10⁸-4.0×10⁸, 1.0×10⁸-3.5×10⁸, 1.5×10⁸-3.0×10⁸, or 1.5×10⁸-2.5×10⁸/mL.

In one or more embodiments, the culture composition comprises components in the following proportion: 200-6000 IU of IL-2: 5-100 ng/mL of IL-7: 5-100 ng/mL of IL-15: 3-100 µg/mL of PD-1 antibodies. In one or more embodiments, the culture composition comprises components: 200-6000 IU/mL of IL-2, 5-100 ng/mL of IL-7, 5-100 ng/mL of IL-15, 3-100 µg/mL of PD-1 antibodies, or the proportional concentration of these components.

Preferably, the culture composition comprises components in the following proportion: 200-500 IU/mL of IL-2: 5-50 ng/mL of IL-7: 5-50 ng/mL of IL-15: 3-50 µg/mL of PD-1 antibodies. In one or more embodiments, the culture composition comprises components: 200-500 IU/mL of IL-2, 5-50 ng/mL of IL-7, 5-50 ng/mL of IL-15, 3-50 µg/mL of PD-1 antibodies, or the proportional concentration of these components.

More preferably, the culture composition comprises components in the following proportion: 500 IU/mL of IL-2: 10 ng/mL of IL-7: 10 ng/mL of IL-15: 3 µg/mL of PD-1 antibodies. In one or more embodiments, the culture composition comprises components: 500 IU/mL of IL-2, 10 ng/mL of IL-7, 10 ng/mL of IL-15, 3µg/mL of PD-1 antibodies, or the proportional concentration of these components.

In one or more embodiments, the culture composition comprises components in the following proportion: 200-6000 IU of IL-2: 5-100 ng/mL of IL-7: 5-100 ng/mL of IL-15: 1-100 µg/mL of PD-1 antibodies. In one or more embodiments, the culture composition comprises components: 200-6000 IU/mL of IL-2, 5-100 ng/mL of IL-7, 5-100 ng/mL of IL-15, and 1-100 µg/mL of PD-1 antibodies, or the proportional concentration of these components.

Preferably, the culture composition comprises components in the following proportion: 200-500 IU/mL of IL-2: 5-50 ng/mL of IL-7: 5-50 ng/mL of IL-15: 3-50 µg/mL of PD-1 antibodies. In one or more embodiments, the culture composition comprises components: 200-500 IU/mL of IL-2, 5-50 ng/mL of IL-7, 5-50 ng/mL of IL-15, 3-50 µg/mL of PD-1 antibodies, or the proportional concentration of these components.

More preferably, the culture composition comprises components in the following proportion: 500 IU/mL of IL-2: 5 ng/mL of IL-7: 10 ng/mL of IL-15:1µg/mL of PD-1 antibodies. In one or more embodiments, the culture composition comprises components: 500 IU/mL of IL-2, 5 ng/mL of IL-7, 10 ng/mL of IL-15, and 1 µg/mL of PD-1 antibodies, or the proportional concentration of these components.

In another or more embodiments, the culture composition comprises components in the following proportion: 200-6000 IU/mL of IL-2: 5-100 ng/mL of IL-7: 5-100 ng/mL of IL-15: 3-100 µg/mL of PD-1 antibodies: 5-100ng/mL of IL-21: 5-100ng/mL of IL-12: 10 µg/mL of CD3 antibodies: 1-10 µg/mL of CD28 antibodies: 200-5000 U/mL of GM-CSF. In one or more embodiments, the culture composition comprises components: 200-6000 IU/mL of IL-2, 5-100 ng/mL of IL-7, 5-100 ng/mL of IL-15, 3-100 µg/mL of PD-1 antibodies, 5-100 ng/mL of IL-21, 5-100ng/mL of IL-12, 1-10 µg/mL of CD3 antibodies, 1-10 µg/mL of CD28 antibodies and 200-5000 U/mL of GM-CSF, or the proportional concentration of these components.

Preferably, the culture composition comprises components in the following proportion: 200-500 IU/mL of IL-2: 5-50 ng/mL of IL-7: 5-50 ng/mL of IL-15: 3-50 µg/mL of PD-1 antibodies: 5-50ng/mL of IL-21: 5-50ng/mL of IL-12: 1-5 µg/mL of CD3 antibodies: 1-5 µg/mL of CD28 antibodies: 200-1000 U/mL of GM-CSF. In one or more embodiments, the culture composition comprises components: 200-500 IU/mL of IL-2, 5-50 ng/mL of IL-7, 5-50 ng/mL of IL-15, 3-50µg/mL ofPD-1 antibodies, 5-50ng/mL of IL-21, 5-50ng/mL of IL-12, 1-5µg/mL of CD3 antibodies, 1-5µg/mL of CD28 antibodies and 200-1000 U/mL of GM-CSF, or the proportional concentration of these components.

More preferably, the culture composition comprises components in the following proportion: 200 IU /mL of IL-2: 10 ng/mL of IL-7: 10 ng/mL of IL-15: 3 µg/mL of PD-1 antibodies: 10ng/mL of IL-21: 20 ng/mL of IL-12: 3 µg/mL of CD3 antibodies: 3 µg/mL of CD28 antibodies: 500 U/mL of GM-CSF. In one or more embodiments, the culture composition comprises components: 200 IU /mL of IL-2, 10 ng/mL of IL-7, 10 ng/mL of IL-15, 3 µg/mL of PD-1 antibodies, 10 ng/mL of IL-21, 20 ng/mL of IL-12, 3 µg/mL of CD3 antibodies, 3 µg/mL of CD28 antibodies and 500 U/mL of GM-CSF, or the proportional concentration of these components.

In another or more embodiments, the culture composition comprises components in the following proportion: 200-6000 IU/mL of IL-2: 5-100 ng/mL of IL-7: 5-100 ng/mL of IL-15: 3-100 µg/mL of PD-1 antibodies: 5-100 ng/mL of IL-21: 5-100ng/mL of IL-12: 1-10 µg/mL of CD3 antibodies: 0.5-10 µg/mL of CD28 antibodies: 200-5000 U/mL of GM-CSF. In one or more embodiments, the culture composition comprises components: 200-6000 IU/mL of IL-2, 5-100 ng/mL of IL-7, 5-100 ng/mL of IL-15, 3-100 µg/mL of PD-1 antibodies, 5-100ng/mL of IL-21, 5-100ng/mL of IL-12, 1-10 µg/mL of CD3 antibodies, 0.5-10 µg/mL of CD28 antibodies and 200-5000 U/mL of GM-CSF, or the proportional concentration of these components.

Preferably, the culture composition comprises components in the following proportion: 200-500 IU/mL of IL-2: 5-50 ng/mL of IL-7: 5-50 ng/mL of IL-15: 3-50 µg/mL of PD-1 antibodies: 5-50ng/mL of IL-21: 5-50ng/mL of IL-12: 1-5 µg/mL of CD3 antibodies: 0.5-5 µg/mL of CD28 antibodies: 200-1000 U/mL of GM-CSF. In one or more embodiments, the culture composition comprises components: 200-500 IU/mL of IL-2, 5-50 ng/mL of IL-7, 5-50 ng/mL of IL-15, 3-50 µg/mL of PD-1 antibodies, 5-50 ng/mL of IL-21, 5-50 ng/mL of IL-12, 1-5 µg/mL of CD3 antibodies, 0.5-5 µg/mL of CD28 antibodies and 200-1000 U /mL of GM-CSF, or the proportional concentration of these components.

More preferably, the culture composition comprises components in the following proportion: 200 IU /mL of IL-2: 50 ng/mL of IL-7: 50 ng/mL of IL-15: 3 µg/mL of PD-1 antibodies: 10 ng/mL of IL-21: 10 ng/mL of IL-12: 3 µg/mL of CD3 antibodies: 0.5 µg/mL of CD28 antibodies: 500 U/mL of GM-CSF. In one or more embodiments, the culture composition comprises components: 200 IU /mL of IL-2, 50 ng/mL of IL-7, 50 ng/mL of IL-15, 3 µg/mL of PD-1 antibodies, 10 ng/mL of IL-21, 10 ng/mL of IL-12, 3 µg/mL of CD3 antibodies, 0.5 µg/mL of CD28 antibodies and 500 U/mL of GM-CSF, or the proportional concentration of these components.

In another or more embodiments, the culture composition comprises components in the following proportion: 200-6000 IU/mL of IL-2: 5-100 ng/mL of IL-7: 5-100 ng/mL of IL-15: 3-100 µg/mL of PD-1 antibodies: 5-100 ng/mL of IL-21: 5-100 ng/mL of IL-12: 1-10 µg/mL of CD3 antibodies: 1-10 µg/mL of CD28 antibodies: 1-100 µg/mL of CD137 antibodies: 1-100 µg/mL of GITR antibodies. In one or more embodiments, the culture composition comprises components: 200-6000 IU/mL of IL-2, 5-100 ng/mL of IL-7, 5-100 ng/mL of IL-15, 3-100 µg/mL of PD-1 antibodies, 5-100ng/mL of IL-21, 5-100ng/mL of IL-12, 1-10 µg/mL of CD3 antibodies, 1-10 µg/mL of CD28 antibodies, 1-100 µg/mL of CD137 antibodies and 1-100 µg/mL of GITR antibodies, or the proportional concentration of these components.

Preferably, the culture composition comprises components in the following proportion: 200-500 IU/mL of IL-2: 5-50 ng/mL of IL-7: 5-50 ng/mL of IL-15: 3-50 µg/mL of PD-1 antibodies: 5-50 ng/mL of IL-21: 5-50 ng/mL of IL-12: 1-5 µg/mL of CD3 antibodies: 1-5 µg/mL of CD28 antibodies: 1-5 µg/mL of CD137 antibodies: 1-5 µg/mL of GITR antibodies. In one or more embodiments, the culture composition comprises components: 200-500 IU/mL of IL-2, 5-50 ng/mL of IL-7, 5-50 ng/mL of IL-15, 3-50 µg/mL of PD-1 antibodies, 5-50 ng/mL of IL-21, 5-50 ng/mL of IL-12, 1-5 µg/mL of CD3 antibodies, 1-5 µg/mL of CD28 antibodies, 1-5 µg/mL of CD137 antibodies and 1-5 µg/mL of GITR antibodies, or the proportional concentration of these components.

More preferably, the culture composition comprises components in the following proportion: 400 IU/mL of IL-2: 10 ng/mL of IL-7: 10 ng/mL of IL-15: 3 µg/mL of PD-1 antibodies: 10 ng/mL of IL-21: 20 ng/mL of IL-12: 3 µg/mL of CD3 antibodies: 3 µg/mL of CD28 antibodies: 3 µg/mL of CD137 antibodies: 3 µg/mL of GITR antibodies. In one or more embodiments, the culture composition comprises components: 400 IU/mL of IL-2, 10 ng/mL of IL-7, 10 ng/mL of IL-15, 3 µg/mL of PD-1 antibodies, 10 ng/mL of IL-21, 20 ng/mL of IL-12, 3 µg/mL of CD3 antibodies, 3 µg/mL of CD28 antibodies, 3 µg/mL of CD137 antibodies and 3 µg/mL of GITR antibodies, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibodies: 5-100 ng/mL of IL-21: 5-100 ng/mL of IL-12: 0.5-10 µg/mL of CD3 antibodies: 1-10 µg/mL of CD28 antibodies: 1-100 µg/mL of CD137 antibodies: 1-100 µg/mL of GITR antibodies. In one or more embodiments, the culture composition comprises components: 200-6000 IU/mL of IL-2, 5-100 ng/mL of IL-7, 5-100 ng/mL of IL-15, 1-100 µg/mL of PD-1 antibodies, 5-100 ng/mL of IL-21, 5-100 ng/mL of IL-12, 0.5-10 µg/mL of CD3 antibodies, 1-10 µg/mL of CD28 antibodies, 1-100 µg/mL of CD137 antibodies and 1-100 µg/mL of GITR antibodies, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of PD-1 antibodies: 5-50 ng/mL of IL-21: 5-50 ng/mL of IL-12: 0.5-5 µg/mL of CD3 antibodies: 1-5 µg/mL of CD28 antibodies: 1-5 µg/mL of CD137 antibodies: 1-5 µg/mL of GITR antibodies. In one or more embodiments, the culture composition comprises components: 200-500 IU/mL of IL-2, 5-50 ng/mL of IL-7, 5-50 ng/mL of IL-15, 1-50 µg/mL of PD-1 antibodies, 5-50 ng/mL of IL-21, 5-50 ng/mL of IL-12, 0.5-5 µg/mL of CD3 antibodies, 1-5 µg/mL of CD28 antibodies, 1-5 µg/mL of CD137 antibodies and 1-5 µg/mL of GITR antibodies, or the proportional concentration of these components.

More preferably, the culture composition comprises components in the following proportion: 400 IU/mL of IL-2: 30 ng/mL of IL-7: 30 ng/mL of IL-15: 1 µg/mL of PD-1 antibodies: 10 ng/mL of IL-21: 15 ng/mL of IL-12: 0.5 µg/mL of CD3 antibodies: 1 µg/mL of CD28 antibodies: 2.5 µg/mL of CD137 antibodies: 3 µg/mL of GITR antibodies. In one or more embodiments, the culture composition comprises components: 400 IU/mL of IL-2, 30 ng/mL of IL-7, 30 ng/mL of IL-15, 1 µg/mL of PD-1 antibodies, 10ng/mL of IL-21, 15 ng/mL of IL-12, 0.5 µg/mL of CD3 antibodies, 1 µg/mL of CD28 antibodies, 2.5 µg/mL of CD137 antibodies and 3 µg/mL of GITR antibodies, or the proportional concentration of these components.

In another or more embodiments, the culture composition comprises components in the following proportion: 200-6000 IU/mL of IL-2: 5-100 ng/mL of IL-7: 5-100 ng/mL of IL-15: 3-100 µg/mL of PD-1 antibodies: 1-10 µg/mL of CD3 antibodies: 1-10 µg/mL of CD28 antibodies: 1-100 µg/mL of CD137 antibodies: 1-100 µg/mL of GITR antibodies: 200-5000 U/mL of GM-CSF: 1-500 µM of TWS119. In one or more embodiments, the culture composition comprises components: 200-6000 IU/mL of IL-2, 5-100 ng/mL of IL-7, 5-100 ng/mL of IL-15, 3-100 µg/mL of PD-1 antibodies, 1-10 µg/mL of CD3 antibodies, 1-10 µg/mL of CD28 antibodies, 1-100 µg/mL of CD137 antibodies, 1-100 µg/mL of GITR antibodies, 200-5000 U/mL of GM-CSF and 1-500 µM of TWS119, or the proportional concentration of these components.

Preferably, the culture composition comprises components in the following proportion: 200-500 IU/mL of IL-2: 5-50 ng/mL of IL-7: 5-50 ng/mL of IL-15: 3-50 µg/mL of PD-1 antibodies: 1-5 µg/mL of CD3 antibodies: 1-5 µg/mL of CD28 antibodies: 1-5 µg/mL of CD137 antibodies: 1-5 µg/mL of GITR antibodies: 200-500 U/mL of GM-CSF: 1-100 µM of TWS119. In one or more embodiments, the culture composition comprises components: 200-500 IU/mL of IL-2, 5-50 ng/mL of IL-7, 5-50 ng/mL of IL-15, 3-50 µg/mL of PD-1 antibodies, 1-5 µg/mL of CD3 antibodies, 1-5 µg/mL of CD28 antibodies, 1-5 µg/mL of CD137 antibodies, 1-5 µg/mL of GITR antibodies, 200-500 U/mL of GM-CSF and 1-100 µM of TWS119, or the proportional concentration of these components.

More preferably, the culture composition comprises components in the following proportion: 300 IU/mL of IL-2: 10 ng/mL of IL-7: 10ng/mL of IL-15: 3 µg/mL of PD-1 antibodies: 3 µg/mL of CD3 antibodies: 3 µg/mL of CD28 antibodies: 3 µg/mL of CD137 antibodies: 3 µg/mL of GITR antibodies: 200 U/mL of GM-CSF: 10 µM of TWS119. In one or more embodiments, the culture composition comprises components: 300 IU/mL of IL-2, 10 ng/mL of IL-7, 10 ng/mL of IL-15, 3 µg/mL of PD-1 antibodies, 3 µg/mL of CD3 antibodies, 3 µg/mL of CD28 antibodies, 3 µg/mL of CD137 antibodies, 3 µg/mL of GITR antibodies, 200 U/mL of GM-CSF and 10 µM of TWS119, or the proportional concentration of these components.

More preferably, the culture composition comprises components in the following proportion: 300 IU/mL of IL-2: 40 ng/mL of IL-7: 40 ng/mL of IL-15: 3 µg/mL of PD-1 antibodies: 1 µg/mL of CD3 antibodies: 1 µg/mL of CD28 antibodies: 5 µg/mL of CD137 antibodies: 2 µg/mL of GITR antibodies: 200 U/mL of GM-CSF: 10 µM of TWS119. In one or more embodiments, the culture composition comprises components: 300 IU/mL of IL-2, 40 ng/mL of IL-7, 40 ng/mL of IL-15, 3 µg/mL of PD-1 antibodies, 1 µg/mL of CD3 antibodies, 1 µg/mL of CD28 antibodies, 5 µg/mL of CD137 antibodies, 2 µg/mL of GITR antibodies, 200 U/mL of GM-CSF and 10 µM of TWS119, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibodies: CD3 antibodies coupled to a matrix: CD28 antibodies coupled to a matrix: 200-5000 U/mL of GM-CSF. In one or more embodiments, the culture composition comprises components: 200-6000 IU/mL of IL-2, 5-100 ng/mL of IL-7, 5-100 ng/mL of IL-15, 1-100 µg/mL of PD-1 antibodies, CD3 antibodies coupled to a matrix, CD28 antibodies coupled to a matrix, 1-100 µg/mL ofCD137 antibodies and 1-100 µg/mL of GITR antibodies, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of PD-1 antibodies: CD3 antibodies coupled to a matrix: CD28 antibodies coupled to a matrix: 200-1000 U/mL of GM-CSF. In one or more embodiments, the culture composition comprises components: 200-500 IU/mL of IL-2, 5-50 ng/mL of IL-7, 5-50 ng/mL of IL-15, 1-50 µg/mL of PD-1 antibodies, CD3 antibodies coupled to a matrix, CD28 antibodies coupled to a matrix and 200-1000 U/mL of GM-CSF, or the proportional concentration of these components.

More preferably, the culture composition comprises components in the following proportion: 500 IU/mL of IL-2: 5 ng/mL of IL-7: 10 ng/mL of IL-15: 3 µg/mL of PD-1 antibodies: CD3 antibodies coupled to a cell culture dish or a cell culture bag: CD28 antibodies coupled to a cell culture dish or a cell culture bag: 500 U/mL of GM-CSF. In one or more embodiments, the culture composition comprises components: 500 IU/mL of IL-2, 5 ng/mL of IL-7, 10 ng/mL of IL-15, 3 µg/mL of PD-1 antibodies, 3 µg/mL of CD3 antibodies, 0.5 µg/mL of CD28 antibodies and 500 U/mL of GM-CSF, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibodies: 5-100 ng/mL of IL-21: 5-100 ng/mL of IL-12: 0.5-10 µg/mL of CD3 antibodies: 0.5-10 µg/mL of CD28 antibodies: 200-5000 U/mL of GM-CSF. In one or more embodiments, the culture composition comprises components: 200-6000 IU/mL of IL-2, 5-100 ng/mL of IL-7, 5-100 ng/mL of IL-15, 1-100 µg/mL of PD-1 antibodies, 5-100 ng/mL of IL-21, 5-100 ng/mL of IL-12, CD3 antibodies coupled to a matrix, CD28 antibodies coupled to a matrix, 1-100 µg/mL of CD137 antibodies and 1-100 µg/mL of GITR antibodies, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of PD-1 antibodies: 5-50 ng/mL of IL-21: 5-50 ng/mL of IL-12: 0.5-5 µg/mL of CD3 antibodies: 0.5-5 µg/mL of CD28 antibodies: 200-1000 U/mL of GM-CSF. In one or more embodiments, the culture composition comprises components: 200-500 IU/mL of IL-2, 5-50 ng/mL of IL-7, 5-50 ng/mL of IL-15, 1-50 µg/mL of PD-1 antibodies, 5-50 ng/mL of IL-21, 5-50 ng/mL of IL-12, CD3 antibodies coupled to a matrix, CD28 antibodies coupled to a matrix and 200-1000 U /mL of GM-CSF, or the proportional concentration of these components.

More preferably, the culture composition comprises components in the following proportion: 500 IU/mL of IL-2: 5 ng/mL of IL-7: 10 ng/mL of IL-15: 3 µg/mL of PD-1 antibodies: 10 ng/mL of IL-21: 10 ng/mL of IL-12: 3 µg/mL of CD3 antibodies: 0.5 µg/mL of CD28 antibodies: 500 U/mL of GM-CSF. In one or more embodiments, the culture composition comprises components: 500 IU/mL of IL-2, 5 ng/mL of IL-7, 10 ng/mL of IL-15, 3 µg/mL of PD-1 antibodies, 10 ng/mL of IL-21, 10 ng/mL of IL-12, 3 µg/mL of CD3 antibodies, 0.5 µg/mL of CD28 antibodies and 500 U/mL of GM-CSF, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibodies: CD3 antibodies coupled to a matrix: CD28 antibodies coupled to a matrix: CD137 antibodies coupled to a matrix. In one or more embodiments, the culture composition comprises components: 200-6000 IU/mL of IL-2, 5-100 ng/mL of IL-7, 5-100 ng/mL of IL-15, 1-100 µg/mL of PD-1 antibodies, CD3 antibodies coupled to a matrix, CD28 antibodies coupled to a matrix and CD137 antibodies coupled to a matrix, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of PD-1 antibodies: CD3 antibodies coupled to a matrix: CD28 antibodies coupled to a matrix: CD137 antibodies coupled to a matrix. In one or more embodiments, the culture composition comprises components: 200-500 IU/mL of IL-2, 5-50 ng/mL of IL-7, 5-50 ng/mL of IL-15, 1-50 µg/mL of PD-1 antibodies, CD3 antibodies coupled to a matrix, CD28 antibodies coupled to a matrix and CD137 antibodies coupled to a matrix, or the proportional concentration of these components.

More preferably, the culture composition comprises components in the following proportion: 500 IU/mL of IL-2: 50 ng/mL of IL-7: 30 ng/mL of IL-15: 1 µg/mL of PD-1 antibodies: CD3 antibodies coupled to a cell culture dish or a cell culture bag: CD28 antibodies coupled to a cell culture dish or a cell culture bag: CD137 antibodies coupled to a cell culture dish or a cell culture bag. In one or more embodiments, the culture composition comprises components: 500 IU/mL of IL-2, 50 ng/mL of IL-7, 30 ng/mL of IL-15, 1 µg/mL of PD-1 antibodies, CD3 antibodies coupled to a cell culture dish or a cell culture bag, CD28 antibodies coupled to a cell culture dish or a cell culture bag and CD137 antibodies coupled to a cell culture dish or a cell culture bag, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibodies: CD3 antibodies coupled to a matrix: CD28 antibodies coupled to a matrix: CD137 antibodies coupled to a matrix: 1-100 µg/mL of GITR antibodies: 1-500 µM of TWS119. In one or more embodiments, the culture composition comprises components: 200-6000 IU/mL of IL-2, 5-100 ng/mL of IL-7, 5-100 ng/mL of IL-15, 1-100 µg/mL of PD-1 antibodies, CD3 antibodies coupled to a matrix, CD28 antibodies coupled to a matrix, CD137 antibodies coupled to a matrix, 1-100 µg/mL of GITR antibodies and 1-500 µM of TWS119, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of PD-1 antibodies: CD3 antibodies coupled to a matrix: CD28 antibodies coupled to a matrix: CD137 antibodies coupled to a matrix: 1-5 µg/mL of GITR antibodies: 1-100 µM of TWS119. In one or more embodiments, the culture composition comprises components: 200-500 IU/mL of IL-2, 5-50 ng/mL of IL-7, 5-50 ng/mL of IL-15, 1-50 µg/mL of PD-1 antibodies, CD3 antibodies coupled to a matrix, CD28 antibodies coupled to a matrix, CD137 antibodies coupled to a matrix, 1-5 µg/mL of GITR antibodies and 1-100 µM of TWS119, or the proportional concentration of these components.

More preferably, the culture composition comprises components in the following proportion: 300 IU/mL of IL-2: 10 ng/mL of IL-7: 25 ng/mL of IL-15: 2 µg/mL of PD-1 antibodies: CD3 antibodies coupled to a matrix: CD28 antibodies coupled to a matrix: CD137 antibodies coupled to a matrix: 3 µg/mL of GITR antibodies: 10 µM of TWS119. In one or more embodiments, the culture composition comprises components: 300 IU/mL of IL-2, 10 ng/mL of IL-7, 25 ng/mL of IL-15, 2 µg/mL of PD-1 antibodies, CD3 antibodies coupled to a matrix, CD28 antibodies coupled to a matrix, CD137 antibodies coupled to a matrix, 3 µg/mL of GITR antibodies and 10 µM of TWS119, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibodies: CD3 antibodies coupled to a matrix: CD28 antibodies coupled to a matrix: CD137 antibodies coupled to a matrix: 1-100 µg/mL of TIGIT antibodies: 200-5000 U/mL of GM-CSF. In one or more embodiments, the culture composition comprises components: 200-6000 IU/mL of IL-2, 5-100 ng/mL of IL-7, 5-100 ng/mL of IL-15, 1-100 µg/mL of PD-1 antibodies, CD3 antibodies coupled to a matrix, CD28 antibodies coupled to a matrix, CD137 antibodies coupled to a matrix, 1-100 µg/mL of TIGIT antibodies and 200-5000 U/mL of GM-CSF, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of PD-1 antibodies: CD3 antibodies coupled to a matrix: CD28 antibodies coupled to a matrix: CD137 antibodies coupled to a matrix: 1-5 µg/mL of TIGIT antibodies: 200-500 U/mL of GM-CSF. In one or more embodiments, the culture composition comprises components: 200-500 IU/mL of IL-2, 5-50 ng/mL of IL-7, 5-50 ng/mL of IL-15, 1-50 µg/mL of PD-1 antibodies, CD3 antibodies coupled to a matrix, CD28 antibodies coupled to a matrix, CD137 antibodies coupled to a matrix, 1-5 µg/mL of TIGIT antibodies and 200-500 U/mL of GM-CSF, or the proportional concentration of these components.

More preferably, the culture composition comprises components in the following proportion: 500 IU/mL of IL-2: 7 ng/mL of IL-7: 50 ng/mL of IL-15: 2 µg/mL of PD-1 antibodies: CD3 antibodies coupled to a matrix: CD28 antibodies coupled to a matrix: CD137 antibodies coupled to a matrix: 2.5 µg/mL of TIGIT antibodies: 500 U/mL of GM-CSF. In one or more embodiments, the culture composition comprises components: 500 IU/mL of IL-2, 7 ng/mL of IL-7, 50 ng/mL of IL-15, 2 µg/mL of PD-1 antibodies, CD3 antibodies coupled to a matrix, CD28 antibodies coupled to a matrix, CD137 antibodies coupled to a matrix, 2.5 µg/mL of TIGIT antibodies and 500 U/mL of GM-CSF, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of LAG-3 antibodies: 5-100ng/mL of IL-21: 5-100 ng/mL of IL-12: 0.5-10 µg/mL of CD3 antibodies: 0.5-10 µg/mL of CD28 antibodies: 1-100 µg/mL of CD137 antibodies: 1-100 µg/mL of GITR antibodies. In one or more embodiments, the culture composition comprises components: 200-6000 IU/mL of IL-2, 5-100 ng/mL of IL-7, 5-100 ng/mL of IL-15, 1-100 µg/mL of LAG-3 antibodies, 5-100 ng/mL of IL-21, 5-100 ng/mL of IL-12, 0.5-10 µg/mL of CD3 antibodies, 0.5-10 µg/mL of CD28 antibodies, 1-100 µg/mL of CD137 antibodies and 1-100 µg/mL of GITR antibodies, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of LAG-3 antibodies: 5-50 ng/mL of IL-21: 5-50 ng/mL of IL-12: 0.5-5 µg/mL of CD3 antibodies: 0.5-5 µg/mL of CD28 antibodies: 1-5 µg/mL of CD137 antibodies: 1-5 µg/mL of GITR antibodies. In one or more embodiments, the culture composition comprises components: 200-500 IU/mL of IL-2, 5-50 ng/mL of IL-7, 5-50 ng/mL of IL-15, 1-50 µg/mL of LAG-3 antibodies, 5-50 ng/mL of IL-21, 5-50 ng/mL of IL-12, 0.5-5 µg/mL of CD3 antibodies, 0.5-5 µg/mL of CD28 antibodies, 1-5 µg/mL of CD137 antibodies and 1-5 µg/mL of GITR antibodies, or the proportional concentration of these components.

More preferably, the culture composition comprises components in the following proportion: 400 IU/mL of IL-2: 30 ng/mL of IL-7: 30 ng/mL of IL-15: 3 µg/mL of LAG-3 antibodies: 10 ng/mL of IL-21: 15 ng/mL of IL-12: 0.5 µg/mL of CD3 antibodies: 1 µg/mL of CD28 antibodies: 2.5 µg/mL of CD137 antibodies: 3 µg/mL of GITR antibodies. In one or more embodiments, the culture composition comprise components: 400 IU/mL of IL-2, 30 ng/mL of IL-7, 30 ng/mL of IL-15, 3 µg/mL of LAG-3 antibodies, 10 ng/mL of IL-21, 15 ng/mL of IL-12, 0.5 µg/mL of CD3 antibodies, 1 µg/mL of CD28 antibodies, 2.5 µg/mL of CD137 antibodies and 3 µg/mL of GITR antibodies, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibodies: CD3 antibodies coupled to a matrix: CD28 antibodies coupled to a matrix: CD137 antibodies coupled to a matrix: 1-100 µg/mL of TIGIT antibodies: 1-100 µg/mL of CD40 antibodies: 1-100 µg/mL of OX-40 antibodies: 1×10⁸-5×10⁸/mL of autologous platelets. In one or more embodiments, the culture composition comprises components: 200-6000 IU/mL of IL-2, 5-100 ng/mL of IL-7, 5-100 ng/mL of IL-15, 1-100 µg/mL of PD-1 antibodies, CD3 antibodies coupled to a matrix, CD28 antibodies coupled to a matrix, CD137 antibodies coupled to a matrix, 1-100 µg/mL of TIGIT antibodies, 1-100 µg/mL of CD40 antibodies, 1-100 µg/mL of OX-40 antibodies, 1×10⁸-5×10⁸/mL of autologous platelets, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of PD-1 antibodies: CD3 antibodies coupled to a matrix: CD28 antibodies coupled to a matrix: CD137 antibodies coupled to a matrix: 1-50 µg/mL of TIGIT antibodies: 1-50 µg/mL of CD40 antibodies: 1-50 µg/mL of OX-40 antibodies: 1×10⁸-3×10⁸/mL of autologous platelets. In one or more embodiments, the culture composition comprises components: 200-500 IU/mL of IL-2, 5-50 ng/mL of IL-7, 5-50 ng/mL of IL-15, 1-50 µg/mL of PD-1 antibodies, CD3 antibodies coupled to a matrix, CD28 antibodies coupled to a matrix, CD137 antibodies coupled to a matrix, 1-50 µg/mL of TIGIT antibodies, 1-50 µg/mL of CD40 antibodies, 1-50 µg/mL of OX-40 antibodies and 1×10⁸-3×10⁸/mL of autologous platelets, or the proportional concentration of these components.

More preferably, the culture composition comprises components in the following proportion: 500 IU/mL of IL-2: 5ng/mL of IL-7: 25 ng/mL of IL-15: 3 µg/mL of PD-1 antibodies: CD3 antibodies coupled to a cell culture dish or a cell culture bag: CD28 antibodies coupled to a cell culture dish or a cell culture bag: CD137 antibodies coupled to a cell culture dish or a cell culture bag: 2.5 µg/mL of TIGIT antibodies: 2 µg/mL of CD40 antibodies: 3 µg/mL of OX-40 antibodies: 3×10⁸/mL of autologous platelets. In one or more embodiments, the culture composition comprises components: 500 IU/mL of IL-2, 5 ng/mL of IL-7, 25 ng/mL of IL-15, 3 µg/mL of PD-1 antibodies, CD3 antibodies coupled to a matrix, CD28 antibodies coupled to a matrix, CD137 antibodies coupled to a matrix, 2.5 µg/mL of TIGIT antibodies, 2 µg/mL of CD40 antibodies, 3 µg/mL of OX-40 antibodies and 3 ×10⁸/mL of autologous platelets, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibodies: 1-100 µg/mL of CTLA-4 antibodies: CD3 antibodies coupled to a matrix: CD28 antibodies coupled to a matrix: 1-100 µg/mL of CD137 antibodies: 1-100 µg/mL of GITR antibodies: 200-5000 U/mL of GM-CSF: 1×10⁸-5×10⁸/mL of autologous platelets. In another or more embodiments, the culture composition comprises components: 200-6000 IU/mL of IL-2, 5-100 ng/mL of IL-7, 5-100 ng/mL of IL-15, 1-100 µg/mL of PD-1 antibodies, 1-100 µg/mL of CTLA-4 antibodies, CD3 antibodies coupled to a matrix, CD28 antibodies coupled to a matrix, 1-100 µg/mL of CD137 antibodies, 1-100 µg/mL of GITR antibodies, 200-5000 U/mL of GM-CSF, 1×10⁸-5×10⁸/mL of allogeneic platelets, or the proportional concentration of these components.

Preferably, the culture composition comprises components in the following proportion: 200-500 IU/mL of IL-2: 5-50 ng/mL of IL-7: 5-50 ng/mL of IL-15: 1-50 µg/mL of PD-1 antibodies: 1-50 µg/mL of CTLA-4 antibodies: CD3 antibodies coupled to a matrix: CD28 antibodies coupled to a matrix: 1-50 µg/mL of CD137 antibodies: 1-50 µg/mL of GITR antibodies: 200-500 U/mL of GM-CSF: 1×10⁸-3×10⁸/mL of allogeneic platelets. In one or more embodiments, the culture composition comprises components: 200-500 IU/mL of IL-2, 5-50 ng/mL of IL-7, 5-50 ng/mL of IL-15, 1-50 µg/mL of PD-1 antibodies, 1-50 µg/mL of CTLA-4 antibodies, CD3 antibodies coupled to a matrix, CD28 antibodies coupled to a matrix, 1-50 µg/mL of CD137 antibodies, 1-50 µg/mL of GITR antibodies, 200-500 U/mL of GM-CSF, 1×10⁸-3×10⁸/mL of allogeneic platelets, or the proportional concentration of these components.

More preferably, the culture composition comprises components in the following proportion: 300 IU/mL of IL-2: 10 ng/mL of IL-7: 30 ng/mL of IL-15: 1 µg/mL of PD-1 antibodies: 2 µg/mL of CTLA-4 antibodies: CD3 antibodies coupled to a matrix: CD28 antibodies coupled to a matrix: 5 µg/mL of CD137 antibodies: 2 µg/mL of GITR antibodies: 200 U/mL of GM-CSF: 1×10⁸/mL of allogeneic platelets. In one or more embodiments, the culture composition comprises components: 300 IU/mL of IL-2, 10 ng/mL of IL-7, 30 ng/mL of IL-15, 1 µg/mL of PD-1 antibodies, 2 µg/mL of CTLA-4 antibodies, CD3 antibodies coupled to a matrix, CD28 antibodies coupled to a matrix, 5 µg/mL of CD137 antibodies, 2 µg/mL of GITR antibodies, 200 U/mL of GM-CSF, 1 ×10⁸/mL of allogeneic platelets, or the proportional concentration of these components.

The present invention also provides a TIL expansion medium, comprising the culture composition for expanding TILs described herein, serum and/or platelets, and a basal medium.

In one or more embodiments, the basal medium is selected from the group consisting of AIM-V, X-VIVO, DMEM, RPMI1640, OpTmizer^{™}, and FUJIFILM Irvin MHM-C.

In one or more embodiments, the serum is selected from human AB serum, autologous serum, or animal-derived serum.

In one or more embodiments, the concentration of serum is 1-10%, such as 1%, 2%, 3%, 4%, 5%, 6%, 7%, 8%, 9%, 10%, or in a range between any two of these values. Preferably, the concentration of serum is 1.5-9.5%, 2.5-8.5%, 3.5-7.5%, 4.5-6.5%.

In one or more embodiments, the density of the platelets is 0.5×10⁸/mL-3×10⁸/mL, such as 0.5×10⁸/mL, 1×10⁸/mL, 1.5×10⁸/mL, 2×10⁸/mL, 2.5×10⁸/mL, or 3×10⁸/mL.

In one or more embodiments, the platelets are platelets derived from the subject's own blood or allogeneic blood.

In one or more embodiments, the medium further comprises antibiotics. Preferably, the antibiotic is selected from one or more of penicillin and streptomycin.

In one or more embodiments, the concentration of penicillin in the medium is 10-1000 IU/mL, 20-800 IU/mL, or 50-100 IU/mL.

In one or more embodiments, the concentration of penicillin in the medium is 10-1000 IU/mL, 20-800 IU/mL, or 50-100 IU/mL.

The present invention also provides a seed cell culture medium for tumor-infiltrating lymphocytes (TIL), which comprises the following components: a cell culture component, a cytokine and an immune checkpoint antibody or an antigen-binding fragment thereof, wherein the cytokine includes IL-2; the immune checkpoint includes: PD-1, LAG-3, TIGIT and/or CTLA-4, and the cell culture component is serum medium or serum-free medium.

In one or more embodiments, the concentration of IL-2 is about 3000 IU/mL and below.

In one or more embodiments, the concentration of IL-2 is about 2000 IU/mL to about 3000 IU/mL.

In one or more embodiments, the cytokine comprises IL-7.

In one or more embodiments, the concentration of IL-7 is about 200 to about 1000 U/mL.

In one or more embodiments, the cytokine comprises IL-15.

In one or more embodiments, the concentration of IL-15 is about 200 to about 500 U/mL.

In one or more embodiments, the cytokine comprises tumor necrosis factor TNF.

In one or more embodiments, the cytokine comprises TNF alpha.

In one or more embodiments, the concentration of TNF alpha is about 10 to about 100 pg/mL.

In one or more embodiments, the cytokine comprises a colony stimulating factor.

In one or more embodiments, the cytokine comprises GM-CSF, G-CSF and/or M-CSF.

In one or more embodiments, the concentration of GM-CSF is about 200 to about 5000 U/mL.

In one or more embodiments, the concentration of G-CSF is about 300 U/mL to about 1000 U/mL.

In one or more embodiments, the concentration of M-CSF is about 300 U/mL to about 800 U/mL.

In one or more embodiments, the cytokine comprises interferon.

In one or more embodiments, the cytokines include IFN-gamma, IFN-alpha and/or IFN-beta.In one or more embodiments, the concentration of IFN-gamma is about 10 to about 1000 ng/mL.

In one or more embodiments, the concentration of IFN-gamma is about 300 to about 1000 U/mL.

In one or more embodiments, the concentration of IFN-alpha is about 500 U/mL to about 1000 U/mL.

In one or more embodiments, the concentration of IFN-beta is about 200 U/mL to about 500 U/mL.

In one or more embodiments, the cytokines also include: IL-4, IL-1alpha, IL-1beta, IL-6, IL-9, IL-18, IL-12, IL-21 and/or IL-10.

In one or more embodiments, the concentration of IL-4 is about 200 to about 500 U/mL.

In one or more embodiments, the concentration of IL-1alpha is about 200 to about 500 U/mL.

In one or more embodiments, the concentration of IL-1beta is about 200 to about 500 U/mL.

In one or more embodiments, the concentration of IL-6 is about 200 to about 500 U/mL.

In one or more embodiments, the concentration of IL-9 is about 200 to about 500 U/mL.

In one or more embodiments, the concentration of IL-18 is about 200 to about 500 U/mL.

In one or more embodiments, the concentration of IL-12 is about 200 to about 1000 U/mL.

In one or more embodiments, the concentration of IL-21 is about 200 to about 500 U/mL.

In one or more embodiments, the concentration of IL-10 is about 200 to about 500 U/mL.

In one or more embodiments, the immune checkpoint antibody or antigen-binding fragment thereof is a human PD-1 antibody or antigen-binding fragment thereof.

In one or more embodiments, the immune checkpoint antibody or antigen-binding fragment thereof is the PD-1 antibody or antigen-binding fragment thereof, and the concentration of the PD-1 antibody or antigen-binding fragment thereof is about 1 to about 100 µg/mL.

In one or more embodiments, the immune checkpoint antibody or antigen-binding fragment thereof is a human LAG-3 antibody or antigen-binding fragment thereof.

In one or more embodiments, the immune checkpoint antibody or antigen-binding fragment thereof is the LAG-3 antibody or antigen-binding fragment thereof, and the concentration of the LAG-3 antibody or antigen-binding fragment thereof is about 3 to about 10 µg/mL.

In one or more embodiments, the TIGIT antibody or antigen-binding fragment thereof is a human TIGIT antibody or antigen-binding fragment thereof.

In one or more embodiments, the immune checkpoint antibody or antigen-binding fragment thereof is the TIGIT antibody or antigen-binding fragment thereof, and the concentration of the TIGIT antibody or antigen-binding fragment thereof is about 1 to about 25 µg/mL.

In one or more embodiments, the immune checkpoint antibody or antigen-binding fragment thereof is a human CTLA-4 antibody or antigen-binding fragment thereof.

In one or more embodiments, the immune checkpoint antibody or antigen-binding fragment thereof is the CTLA-4 antibody or antigen-binding fragment thereof, and the concentration of the CTLA-4 antibody or antigen-binding fragment thereof is about 1 to about 100 µg/mL.

In one or more embodiments, the seed cell culture medium further includes a co-stimulatory receptor antibody or an antigen-binding fragment thereof, wherein the co-stimulatory receptor or antigen-binding fragment thereof includes: a CD40 antibody or an antigen-binding fragment thereof , OX-40 antibody or antigen-binding fragment thereof, CD137 antibody or antigen-binding fragment thereof and/or CD28 antibody or antigen-binding fragment thereof.

In one or more embodiments, the immune checkpoint antibody or antigen-binding fragment thereof comprises a CD137 antibody or antigen-binding fragment thereof.

In one or more embodiments, the CD137 antibody or antigen-binding fragment thereof is a human CD137 antibody or antigen-binding fragment thereof.

In one or more embodiments, the concentration of CD137 antibody or antigen-binding fragment thereof is about 1 to about 100 µg/mL.

In one or more embodiments, the immune checkpoint antibody or antigen-binding fragment thereof comprises a CD28 antibody or antigen-binding fragment thereof.

In one or more embodiments, the CD28 antibody or antigen-binding fragment thereof is a human CD28 antibody or antigen-binding fragment thereof.

In one or more embodiments, the concentration of CD28 antibody or antigen-binding fragment thereof is about 1 to about 10 µg/mL.

In one or more embodiments, the CD40 antibody or antigen-binding fragment thereof is a human CD40 antibody or antigen-binding fragment thereof.

In one or more embodiments, the concentration of CD40 antibody or antigen-binding fragment thereof is about 5 to about 10 µg/mL.

In one or more embodiments, the OX-40 antibody or antigen-binding fragment thereof is a human OX-40 antibody or antigen-binding fragment thereof.

In one or more embodiments, the concentration of OX-40 antibody or antigen-binding fragment thereof is about 3 to about 10 µg/mL.

In one or more embodiments, the serum medium includes serum.

In one or more embodiments, the serum includes human AB serum.

In one or more embodiments, the concentration of serum is about 1-about 10% (v/v).

In one or more embodiments, the serum medium includes AIM-V medium.

In one or more embodiments, the serum-free medium includes X-VIVO medium.

In one or more embodiments, the cell culture components include antibiotics.

In one or more embodiments, the cell culture components include penicillin-streptomycin mixture PS.

In one or more embodiments, the concentration of the penicillin-streptomycin mixture PS is about 1-about 200 U/mL.

In one or more embodiments, the seed cell culture medium further includes an M2 macrophage inhibitor, and the M2 macrophage inhibitor includes RRx001 and/or CNI-1493.

In one or more embodiments, the concentration of M2 macrophage inhibitor is about 0.1 to about 100 µM.

In one or more embodiments, the seed cell culture medium also includes a regulatory T cell (Treg) inhibitor, and the regulatory T cell inhibitor includes CAL-101, dasatinib, imatinib and/or panobinostat .

In one or more embodiments, the concentration of regulatory T cell inhibitor is about 0.1 to about 100 µM.

In one or more embodiments, the seed cell culture medium also includes a myeloid-derived suppressor cell (MDSC) inhibitor, and the myeloid-derived suppressor cell inhibitor includes AG490, Decitabine, Sunitinib and/or BBI608.

In one or more embodiments, the concentration of myeloid-derived suppressor cell inhibitor is about 0.1 to about 100 µg/mL.

In one or more embodiments, the seed cell culture medium further includes a T cell activator, and the T cell activator includes LYC-55716, GNE-1858 and/or methylene blue.

In one or more embodiments, the concentration of T cell activator is about 1 to about 10 µM.

In one or more embodiments, the seed cell culture medium further includes a T cell differentiation inhibitor, and the T cell differentiation inhibitor includes TWS119.

In one or more embodiments, the concentration of T cell differentiation inhibitor is about 1 to about 10 µM.

In another aspect, the present application provides the seed cells of tumor infiltrating lymphocytes or the cell population thereof obtained by culturing the seed cell medium described in the present application.

In another aspect, the present application provides a pharmaceutical composition, which comprises the seed cells of tumor infiltrating lymphocytes or the cell population thereof described in the present application and a pharmaceutically acceptable carrier.

In another aspect, the present application provides use of the seed cells of tumor infiltrating lymphocytes or the cell population thereof and the pharmaceutical composition described in the present application in the manufacture of a drug for treating cancer.

In one or more embodiments, the cancer is selected from the group consisting of melanoma, glioma, gastric cancer, lung cancer, gastrointestinal stromal tumor, intestinal cancer, liver cancer, cervical cancer, ovarian cancer, breast cancer, endometrial stromal sarcomas, pelvic poorly differentiated adenocarcinomas and cholangiocarcinoma.

In another aspect, the present application provides a use of the seed cell culture medium in expanding tumor infiltrating lymphocytes.

In another aspect, the present application provides a method for culturing seed cells of tumor-infiltrating lymphocytes, which includes the following steps: culturing isolated tumor-infiltrating lymphocytes in the seed cell culture medium described in the present application.

In another aspect, the present application provides a method for expanding tumor-infiltrating lymphocytes, which includes the following steps, culturing isolated tumor-infiltrating lymphocytes in the seed cell culture medium described in the present application, obtaining the seed cells of the tumor-infiltrating lymphocytes.

In one or more embodiments, the isolated tumor infiltrating lymphocytes are derived from a sample selected from the group consisting of ascites from a subject in need thereof, surgically resected primary tumor samples, synchronous and metachronous surgical resected metastatic samples, biopsy samples and body fluids.

In one or more embodiments, the body fluids include blood, interstitial fluid, lymph and/or body cavity fluid.

In one or more embodiments, the isolated tumor infiltrating lymphocytes are derived from a tumor selected from the group consisting of a melanoma, a glioma, a gastric cancer, a lung cancer, a gastrointestinal stromal tumor, a intestinal cancer, a liver cancer, a cervical cancer, an ovarian cancer, a breast cancer, an endometrial stromal sarcomas, a pelvic poorly differentiated adenocarcinomas and a cholangiocarcinoma.

In one or more embodiments, the isolated tumor infiltrating lymphocytes are derived from dissected tumor tissue, the dissected tumor tissue having a diameter of about 1 mm to about 10 mm.

In one or more embodiments, the culture time is about 3-20 days.

In one or more embodiments, the culture temperature is about 30-42 °C.

The present invention also provides the use of the culture composition for expanding TILs described herein in the manufacture of a culture medium for expanding TILs or a drug that contains TILs. In one or more embodiments, the drugs are used to treat cancer.

In one or more embodiments, the cancer is selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma.

The present invention also provides the use of the culture medium for expanding TILs described herein in the manufacture of a drug that contains TILs. In one or more embodiments, the drugs are used to treat cancer.

In one or more embodiments, the cancer is selected from the group consisting of: a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma.

The invention also provides a method of expanding a population of TILs comprising incubating the population of TILs with a medium as described herein.

In one or more embodiments, the incubation lasts for at least 5 days, at least 6 days, at least 7 days, at least 8 days, at least 9 days, at least 10 days, at least 11 days, at least 12 days, at least 13 days, at least 14 days, at least 15 days.

In one or more embodiments, the incubation lasts for at most 20 days, at most 15 days, at most 14 days, at most 13 days, at most 12 days, at most 11 days, at most 10 days, at most 9 days, at most 8 days, at most 7 days, at most 6 days, at most 5 days.

The present invention also provides a method for preparing the population of TILs, comprising:
(1) incubating a tumor cell-containing tissue with TIL seed cell culture medium to obtain the first population of TILs,
(2) incubating the first population of TILs with the medium described herein to obtain the second population of TILs.

In one or more embodiments, the tumor tissue is obtained from a cancer subject.

In one or more embodiments, the tumor tissue is pretreated. In one or more embodiments, the pretreatment includes fragmenting and/or dissociating tumor tissue.

In one or more embodiments, the tumor tissue is cut into fragmented tissue pieces by mechanical force.

In one or more embodiments, the size of the fragmented tissue pieces is 2x2x2 mm³, 3x3x3 mm³, 4x4x4 mm³, 5x5x5 mm³ or 6x6x6 mm³.

In one or more embodiments, the incubation of step (1) lasts for at least 5 days, at least 6 days, at least 7 days, at least 8 days, at least 9 days, at least 10 days, at least 11 days, at least 12 days, at least 13 days, at least 14 days, at least 15 days.

In one or more embodiments, the incubation of step (1) lasts for at most 20 days, at most 15 days, at most 14 days, at most 13 days, at most 12 days, at most 11 days, at most 10 days, at most 9 days, at most 8 days, at most 7 days, at most 6 days, at most 5 days.

In one or more embodiments, the incubation of step (2) lasts for at least 5 days, at least 6 days, at least 7 days, at least 8 days, at least 9 days, at least 10 days, at least 11 days, at least 12 days, at least 13 days, at least 14 days, at least 15 days.

In one or more embodiments, the incubation of step (2) lasts for at most 20 days, at most 15 days, at most 14 days, at most 13 days, at most 12 days, at most 11 days, at most 10 days, at most 9 days, at most 8 days, at most 7 days, at most 6 days, at most 5 days.

In one or more embodiments, the initial seeding density of the first population of TILs in step (2) is 2.0×10⁵/cm²~5.0×10⁵/cm², preferably, 2.5×10⁵/cm²~4.5 × 10⁵/cm², more preferably, 2.5×10⁵/cm²~3.0×10⁵/cm².

In one or more embodiments, the tissue that contains tumor cell is a tumor tissue or body fluid of a patient having a cancer selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma, etc.

In one or more embodiments, the body fluids include blood, interstitial fluid, lymph and/or ascites.

In one or more embodiments, the TIL seed cell culture medium is as described above, referring to those described in PCT/CN2021/096585, which is incorporated herein by reference in its entirety for all purposes.

In one or more embodiments, the incubation temperature in step (1) is 30-42°C, preferably 37°C.

In one or more embodiments, the incubation temperature in step (2) is 30-42°C, preferably 37°C.

In one or more embodiments, the concentration of CO₂ in the incubation of step (1) and step (2) is 5%.

The present invention also provides a method for preparing the population of TILs, comprising:
(1) incubating a tumor cell-containing tissue with TIL seed cell culture medium to obtain the first population of TILs,
(2) contacting the first population of TILs described in (1) with any one or more of the antibody coupled to a matrix described in the above culture composition herein to obtain a second population of TILs,
(3) culturing the second population of TILs described in (2) in a medium containing any one or more of the components in the above culture composition herein except for the antibodies coupled to the matrix, to obtain a third population of TILs.

In one or more embodiments, the tumor tissue is obtained from a cancer subject.

In one or more embodiments, the tumor tissue is a tumor tissue obtained by surgery or biopsy.

In one or more embodiments, the tumor tissue is pretreated. In one or more embodiments, the pretreatment includes fragmenting and/or dissociating tumor tissue.

In one or more embodiments, the tumor tissue is cut into fragmented tissue pieces by mechanical force.

In one or more embodiments, the size of the fragmented tissue pieces is 2x2x2 mm³, 3x3x3 mm³, 4x4x4 mm³, 5x5x5 mm³ or 6x6x6 mm³.

In one or more embodiments, the TIL seed cell culture medium in step (1) is the above TIL seed cell culture medium described herein.

In one or more embodiments, the incubation temperature in step (1) is 30-42°C, preferably 37°C.

In one or more embodiments, the concentration of CO₂ in incubation of step (1) is 5%.

In one or more embodiments, the incubation of step (1) lasts for at least 5 days, at least 6 days, at least 7 days, at least 8 days, at least 9 days, at least 10 days, at least 11 days, at least 12 days, at least 13 days, at least 14 days, at least 15 days.

In one or more embodiments, the incubation of step (1) lasts for at most 20 days, at most 15 days, at most 14 days, at most 13 days, at most 12 days, at most 11 days, at most 10 days, at most 9 days, at most 8 days, at most 7 days, at most 6 days, at most 5 days.

In one or more embodiments, the antibody coupled to the matrix in step (2) includes any one or more of a CD3 antibody, a CD28 antibody and a CD137 antibody; preferably, includes a CD3 antibody and a CD28 antibody; more preferably, includes a CD3 antibody, a CD28 antibody and a CD137 antibody.

In one or more embodiments, the matrix in step (2) is a multi-well plate, a cell culture dish or a cell culture bag.

In one or more embodiments, the coupling in step (2) is covalent coupling or non-covalent coupling.

In one or more embodiments, the contacting in step (2) is to incubate the first population of TILs in step (1) with the antibody coupled to the matrix; preferably, the incubation temperature is 30 -42°C, preferably 37°C. Preferably, the concentration of the incubation of CO₂ is 5%.

In one or more embodiments, in step (2), the incubation is carried out in a culture medium containing any one or more of the components in the above culture composition described herein except for the antibodies coupled to the matrix.

In one or more embodiments, the contacting in step (2) lasts for 1-3 days. Preferably, the contacting in step (2) lasts for 2-3 days.

In one or more embodiments, the density of the first population of TILs in step (2) is 1.0×10⁵/mL~1.0×10⁶/mL; preferably, 2.0×10⁵/mL~1.0 ×10⁶/mL; more preferably, 2.5×10⁵/mL~3.5×10⁶/mL.

In one or more embodiments, the culturing of step (3) lasts for at least 5 days, at least 6 days, at least 7 days, at least 8 days, at least 9 days, at least 10 days, at least 11 days, at least 12 days, at least 13 days, at least 14 days, at least 15 days.

In one or more embodiments, the culturing of step (3) lasts for at most 20 days, at most 15 days, at most 14 days, at most 13 days, at most 12 days, at most 11 days, at most 10 days, at most 9 days, at most 8 days, at most 7 days, at most 6 days, at most 5 days.

In one or more embodiments, the culture temperature in step (3) is 30-42°C, preferably 37°C.

In one or more embodiments, the concentration of CO₂ in the culturing of step (3) is 5%.

The present invention also provides a population of TILs obtained by the method described in any embodiment herein.

The present invention also provides the use of the population of TILs obtained by the method described in any embodiment herein in the manufacture of a drug for treating cancer.

In one or more embodiments, the cancer is selected from the group consisting of a gastric cancer, a thyroid tumor, a gallbladder cancer, a cholangiocarcinoma, a lung cancer, a melanoma, a head and neck cancer, a breast cancer, an ovarian cancer, cervical cancer, a liver cancer, a colorectal cancer, a cerebral glioma, a pancreatic cancer, a bladder cancer, a prostate cancer, a renal cancer, an osteosarcoma.

The present invention also provides a pharmaceutical composition, comprising the population of TILs obtained by the method described in any embodiment herein, and pharmaceutically acceptable excipients.

In one or more embodiments, the pharmaceutical composition comprises the population of TILs obtained by the method described in any embodiment herein and hydroxychloroquine. Preferably, the pharmaceutical composition also includes chemotherapeutic drugs. More preferably, the chemotherapeutic drug is cyclophosphamide.

The present invention also provides a cell cryopreservant preparation, including the population of TILs obtained by the method described in any embodiment herein and a cryopreservant.

In one or more embodiments, the cryopreservant is a cell cryopreservant or a tissue cryopreservant.

In one or more embodiments, the cryopreservant is a serum-free cryopreservant, such as CryoStor CS10 Freezing Medium from BioLifeSolutions.

The present invention also provides a method for treating a solid tumor, comprising:
(1) obtaining the tumor tissue from a solid tumor patient, and preparing TILs by the method for preparing the TIL population described above;
(2) pretreating the solid tumor patient;
(3) infusing the TILs in (1) into the pretreated solid tumor patient.
   In one or more embodiments, the method for treating a solid tumor may also include:
(4) assessment of efficacy.

In one or more embodiments, the solid tumor includes a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma.

In one or more embodiments, the pretreatment in step (2) includes administering a chemotherapeutic drug and hydroxychloroquine to the solid tumor patient. In one or more embodiments, the chemotherapeutic drug is cyclophosphamide.

In one or more embodiments, the cyclophosphamide is administered at a dose of 5-50 mg/kg/day, preferably, at a dose of 20-50 mg/kg/day, more preferably, at a dose of 20-25 mg/kg/day.

In one or more embodiments, the hydroxychloroquine is administered at a dose of 400 mg/day to 800 mg/day, preferably, at a dose of 500 mg/day to 800 mg/day, more preferably, at a dose of 600 mg/day to 800 mg/day.

In one or more embodiments, the pretreatment in step (2) is performed on any one day, two days or three days of the 6th day to the 3rd day before TIL infusion. Preferably, the pretreatment is carried out on Day 5, Day 4 and Day 3 before TII,infusion.

In one or more embodiments, the pretreatment in step (2) includes: administering 400-800 mg hydroxychloroquine orally and administering 5-50 mg/kg cyclophosphamide by intravenous injection to the solid tumor patient on Day 5 before TIL infusion, and administering 5-50 mg/kg cyclophosphamide by intravenous injection to the solid tumor patient on Day 4 and Day 3 before infusion.

In one or more embodiments, the cyclophosphamide is administered intravenously or orally.

In one or more embodiments, the hydroxychloroquine is administered intravenously or orally.

In one or more embodiments, the TILs described in the (1) of step (3) are present in the above cell cryopreservant preparation herein.

In one or more embodiments, the number of autologous TILs infused in step (3) is 1.0×10¹⁰-1.2×10¹¹ TILs, preferably, 2.0×10¹⁰-8.0×10¹⁰ TILs, more preferably, 2.0×10¹⁰-5.0×10¹⁰ TILs, still more preferably, 2.0×10¹⁰-4.0×10¹⁰ TILs.

In one or more embodiments, the number of autologous TILs infused in step (3) is 1.0×10¹⁰ TILs, 1.5×10¹⁰ TILs, 2.0×10¹⁰ TILs, 2.5×10¹⁰ TILs, 3.0×10¹⁰ TILs, 3.5×10¹⁰ TILs, 4.0×10¹⁰ TILs, 4.5×10¹⁰ TILs, 5.0×10¹⁰ TILs, 5.5×10¹⁰ TILs, 6.0×10¹⁰ TILs, 6.5×10¹⁰ TILs, 7.0×10¹⁰ TILs, 7.5×10¹⁰ TILs, 8.0×10¹⁰ TILs, 8.5×10¹⁰ TILs, 9.0×10¹⁰ TILs, 9.5×10¹⁰ TILs , 1.0×10¹¹ TILs, or 1.2×10¹⁰ TILs.

In one or more embodiments, the cell density of the cell preparation of TILs infused in step (3) is 1.0×10⁸/mL~2.0×10⁸/mL.

In one or more embodiments, the volume of the cell preparation of TILs infused in step (3) is 200 mL to 600 mL.

In one or more embodiments, the autologous infusion in step (3) is performed by intravenous injection.

In one or more embodiments, the efficacy assessment in step (4) includes tumor imaging assessment and tumor marker expression level assessment.

In one or more embodiments, said tumor imaging assessment is performed by CT and/or MRI. Preferably, the criterion of the tumor imaging assessment is RECIST v1.1.

In one or more embodiments, the expression levels of tumor markers are detected by immunohistochemistry, blood index measurement and/or high-throughput sequencing.

Advantages of the invention:
1) The present invention can effectively reactivate TILs that are inhibited in the tumor microenvironment during *in vitro* culture by a component-optimized expansion medium containing IL-2 and other cytokines and activating antibodies;
2) While significantly reducing the amount of IL-2, cell proliferation and viability are guaranteed, and the risk of potential cell exhaustion is reduced, and at the same time, the dependence of cultured TILs on IL-2 is greatly reduced through the optimized *in vitro* culture process;
3) TILs obtained using the TIL culture medium and the TIL culture method of the present invention present greatly reduced dependence on IL-2, therefore the indispensable chemotherapeutic drug fludarabine in the conventional treatment plan in the pretreatment before the infusion for clinical patients can be removed, and the dosage of the chemotherapeutic drug cyclophosphamide used is also greatly reduced compared with the current conventional dosage. Meanwhile, compared with the conventional high-dose IL-2 injection after TIL infusion, no IL-2 injection is needed after infusion, and efficient and robust proliferation in the human body after TIL infusion can be achieved without high-grade adverse events, which greatly reduces clinical side effects while producing significant and positive therapeutic effects. At the same time, for some patients with advanced solid tumors who have poor performance and cannot tolerate high-dose IL-2 treatment, the TILs prepared by the culture method of the present invention for autologous infusion therapy can be used. That is, the TILs prepared by the TIL culture medium and the TIL culture method of the present invention can potentially cover a larger population of solid tumor patients;
4) No feeder cells (such as exogenous allogeneic PBMC or other cells) are needed for the expansion of the TILs of the present invention, which greatly simplifies the manufacturing process while reducing exogenous pollution, lowers production costs, and shortens the manufacturing period. In preparing TILs by the medium of the present invention, 10¹⁰-10¹¹ cells can be quickly obtained without using feeder cells.

### Brief Description of Figures

Figure 1: Flow cytometric phenotyping results of product TILs derived from tissue sample T001.
Figure 2: Flow cytometric phenotyping results of product TILs derived from tissue sample T002.
Figure 3: Flow cytometric phenotyping results of product TILs derived from tissue sample T003.
Figure 4: Flow cytometric phenotyping results of product TILs derived from tissue sample T004.
Figure 5: Flow cytometric phenotyping results of product TILs derived from tissue sample T005.
Figure 6: Flow cytometric phenotyping results of product TILs derived from tissue sample T006.
Figure 7: Flow cytometric phenotyping results of product TILs derived from tissue sample T007.
Figure 8: Flow cytometric phenotyping results of product TILs derived from tissue sample T008.
Figure 9: Flow cytometric phenotyping results of product TILs derived from tissue sample T009.
Figure 10: Flow cytometric phenotyping results of product TILs derived from tissue sample T010.
Figure 11: Flow cytometric phenotyping results of product TILs derived from tissue sample T011.
Figure 12: Flow cytometric phenotyping results of product TILs derived from tissue sample T012.
Figure 13: Flow cytometric phenotyping results of product TILs derived from tissue sample T013.
Figure 14: Flow cytometric phenotyping results of product TILs derived from tissue sample T014.
Figure 15: Flow cytometric phenotyping results of product TILs derived from tissue sample T015.
Figure 16: Flow cytometric phenotyping results of product TILs derived from tissue sample T016.
Figure 17: Flow cytometric phenotyping results of product TILs derived from tissue sample T017.
Figure 18: RTCA killing effect of TILs derived from T001 on target cell HGC27.
Figure 19: RTCA killing effect of TILs derived from T002 on target cell A375.
Figure 20: RTCA killing effect of TILs derived from T003 on target cell HeLa.
Figure 21: RTCA killing effect of TILs derived from T005 on target cell HGC27.
Figure 22: RTCA killing effect of TILs derived from T004 on target cell HGC27.
Figure 23: The killing effect of TILs derived from T006 on syngeneic tumor organoids.
Figure 24: The killing effect of TILs derived from T001 on the mouse model of HGC27-derived CDX tumor tissue.
Figure 25: The killing effect of TILs derived from T002 on the mouse model of A375-derived CDX tumor tissue.
Figure 26: The killing effect of TILs derived from T008 on the syngeneic PDX tumor tissue mouse model.
Figure 27: Effect of TILs derived from T007 on the *in vivo* tumorigenesis tendency of the syngeneic PDX tumor tissue mouse model.
Figure 28: The imaging evaluation results of T009 patients at different time points after TILs infusion therapy.
Figure 29: Tendency curve of the number of white blood cells, neutrophils and lymphocytes in the peripheral blood of patient T009 from before infusion to nearly 120 days after infusion.
Figure 30: Tendency curve of the number of white blood cells, neutrophils and lymphocytes in the peripheral blood of patient T009 from before infusion to 20 days after infusion.
Figure 31: The superimposed tendency of the TCR clonotype frequency of the TILs infused and the total number of lymphocytes in the peripheral blood of patient T009 over time.

### Detailed Description

In order to reduce exogenous contamination in TIL culture, simplify manufacturing process, reduce production cost and shorten manufacturing time, the inventors have developed the expansion (REP) medium that is used to prepare TILs from tumor tissue and a method for preparing or expanding TILs. In treating TILs, more than 10¹⁰-10¹¹ cells can be obtained through rapid expansion using these media even without the use of feeder cells.

### Definition of Terms

In this application, the term "interferon" generally refers to a family of highly species-specific proteins that inhibit viral replication and cell proliferation and modulate immune responses. Exemplary suitable interferons include, but are not limited to: recombinant interferon alpha-2b, recombinant interferon alpha-2a, recombinant interferon alpha-2c, interferon alpha-n1, consensus alpha interferon, or interferon alpha-n3.

In this application, the term "colony stimulating factor" generally refers to a class of secreted glycoproteins that bind to receptor proteins on the surface of hematopoietic stem cells, thereby activating intracellular signaling pathways that lead to cell proliferation and differentiation into a specific type of blood cell (usually white blood cells, for red blood cell formation see erythropoietin). They can be synthesized artificially and administered exogenously.

In this application, the term "antigen-binding fragment" generally refers to a portion of an antibody molecule comprising the amino acids responsible for the specific binding between the antibody and the antigen. The portion of an antigen that is specifically recognized and bound by an antibody is called an "epitope" as described above. As noted above, an antigen-binding domain may typically comprise an antibody light chain variable region (VL) and an antibody heavy chain variable region (VH); however, it need not necessarily comprise both. Fd fragments, for example, have two VH regions and typically retain some antigen-binding function of the full antigen-binding domain. Examples of antigen-binding fragments of antibodies include (1) Fab fragments, a monovalent fragments having VL, VH, constant light chain (CL) and CH1 domains; (2) F(ab')2 fragments, two Bivalent fragment having two Fab fragments connected by sulfur bridge; (3) Fd fragment with two VH and CH1 domains; (4) Fv fragment with VL and VH domains of antibody single arm, (5) dAb fragment (Ward et al., "Binding Activities of a Repertoire of Single Immunoglobulin Variable Domains Secreted From Escherichia coli," Nature 341:544-546 (1989), which is hereby incorporated by reference in its entirety), has a VH domain; (6) isolated complementarity determining regions (CDRs), and (7) single chain Fv (scFv), for example derived from scFv-libraries. Although the two domains VL and VH of the Fv fragment are encoded by separate genes, they can be joined using recombinant methods by a synthetic linker that allows it to be produced as a single protein chain in which the VL and VH regions pair to form a monovalent molecule (termed as single-chain Fv (scFv)) (see, e.g., Huston et al., "Protein Engineering of Antibody Binding Sites: Recovery of Specific Activity in an Anti-Digoxin Single-Chain Fv Analogue Produced in Escherichia coli," Proc.Natl.Acad.Sci.USA 85:5879-5883 (1988)). These antibody fragments are obtained using conventional techniques known to those skilled in the art, and the function of the fragments can be evaluated in the same manner as intact antibodies. In the present application, the term "antigen-binding fragment" also includes ligands that bind to antigens, especially cell surface receptor molecule antigens. A ligand is typically a polypeptide or compound that can bind to a receptor protein (eg, the antigen) with high affinity and specificity to elicit a functional response. For example, the ligands may include CD40L for CD40, CD137L for CD137, CD80 for CD28 and/or OX-40L for OX-40.

In this application, the term "immune checkpoint" generally refers to a group of molecules on the cell surface of CD4+ and CD8+ T cells. These molecules can be effectively used as "brakes" to down-regulate or suppress anti-tumor immune responses. These inhibitory molecules can be inhibited by inhibiting levels of DNA, RNA or protein.

In this application, the term "activating antibody" generally refers to antibodies against immune cells such as T cell surface receptors such as TCR and/or co-stimulatory receptors, these antibodies bind to TCR and/or co-stimulatory receptors, the activation signaling pathways downstream of these receptors can be turned on, so that the activation and proliferation levels of the immune cells are significantly improved.

In this application, the term "co-stimulatory receptor" generally refers to a class of receptors expressed on the surface of immune cells, such as T cells, upon which the activation of immune responses depends. On the premise that the antigen presenting cells exist and the first signal has been provided, the co-stimulatory receptor will turned on the co-stimulatory signal by binding to its corresponding activating antibody, ligand or antigen-binding fragment, and then the immune effector cells are fully activated. Activation of co-stimulatory receptor-mediated signaling pathways often plays a key role for the efficient development of immune responses. Common co-stimulatory receptors include, but are not limited to, CD28, CD40, CD137, and OX-40.

In this application, the term "tumor necrosis factor TNF" generally refers to the TNF protein, and not to the superfamily of TNF ligands. The Genbank accession number of human TNF alpha is NP_000585.2. The term "TNF alpha" encompasses precursor forms of TNF alpha, pro-TNF alpha, full-length TNF alpha, and any form of TNF alpha produced by intracellular processes. The term also encompasses naturally occurring and non-naturally occurring variants of TNF alpha, such as splice variants, allelic variants, and isoforms. TNF alpha can bind two receptors, TNFR1 (TNF receptor type 1; CD 120a; p55/60) and TNFR2 (TNF receptor type 2; CD120b; p75/80). TNF alpha functions as a pro-inflammatory cytokine, such as in neuroinflammation.

In the present application, the term "tumor infiltrating lymphocytes" generally refers to infiltrating lymphocytes isolated from tumor tissue. Immunohistochemical staining showed that tumor-infiltrating lymphocytes could include CD3+T cells, CD20+, CD79 alpha+B cells, plasma cells, and CD56+NK cells; they could also include T cells, a small amount of B cells, NK cells, macrophages, and dendritic cells. The tumor-infiltrating lymphocytes can produce highly efficient and specific anti-tumor killing effects through direct cytotoxic T lymphocytes and secretion of cytokines. In the present application, the tumor infiltrating lymphocytes may include mononuclear leukocytes that leave the bloodstream and migrate to a tumor. The tumor infiltrating lymphocytes may be selected from the group consisting of T cells, B cells, natural killer cells, and natural killer T cells.

In the present application, the term "seed cells" generally refers to tumor infiltrating lymphocytes cultured from tumor tissue using any suitable medium including the medium of the present application. These cells can be used not only as the initial population of cell for subsequent further expansion, but also as the end-use population of cell, such as the population of cell for infusion therapy to individual subjects. The seed cells can be obtained from the tumor tissue through any different culture period, and the culture period of the seed cells can be determined according to the subsequent use of the seed cells, and are not limited to a specific culture period.

In this application, the term "IL-15" generally refers to a pro-inflammatory cytokine that acts as a potent growth, survival and activation factor for T cells, especially intestinal intraepithelial lymphocytes (IEL) and natural killer (NK) cells, also known as "IL-15 antigen" and "Interleukin 15". Increased expression of IL-15 has been demonstrated in various inflammatory disorders including CD, rheumatoid arthritis (RA) and psoriasis (Malamut et al., 2010). IL-15 is considered a central regulator of immunopathology in CD and a non-redundant driver of lymphomagenesis in RCD.

In the present application, the term "IL-2" generally refers to a protein derived from a lymphokine produced by normal peripheral blood lymphocytes and present in low concentrations in the body. Initially, Morgan et al. (1976) Science 193: 1007-1008 described IL-2, which is originally called a T cell growth factor, because of its ability to induce proliferation of stimulated T lymphocytes. The term also includes post-expression modified proteins of IL-2, such as glycosylation, acetylation, phosphorylation, and the like.

In this application, the term "IL-7" generally refers to a protein encoded by the IL-7 gene. IL-7 is a hematopoietic growth factor secreted by stromal cells in the bone marrow and thymus. It can be produced by keratinocytes, dendritic cells, hepatocytes, neurons and epithelial cells. IL-7 can be involved in effects on T cells and B cells.

In this application, the term "PD-1" generally refers to an immunosuppressive receptor belonging to the CD28 family. PD-1 is predominantly expressed in vivo on previously activated T cells and binds two ligands, PD-1 and PD-2. The complete hPD-1 sequence can be found at GenBank accession No.U64863.

In this application, the term "LYC-55716" generally refers to a ROR gamma activator. Its CAS number is 2055536-64-4.

In this application, the term "GNE-1858" generally refers to an HPK1 inhibitor whose CAS number is T11438.

In this application, the term "TWS119" generally refers to a GSK-3 inhibitor whose CAS number is 601514-19-6.

In this application, the term "RRX-001" generally refers to an epigenetic modulator with radiosensitizing activity. The RRX-001 downregulates the CD47/SIRP alpha axis and repolarizes TAMs and other immunosuppressive cells in the tumor microenvironment to an immunostimulatory phenotype. Its CAS number is 925206-65-1.

In this application, the term "CNI-1493" generally refers to a compound that can prevent the production of inflammatory cytokines (including TNF), and its CAS number is 164301-51-3.

Herein, the term "inducing TIL" refers to the process of isolating TILs from animal tumors. After the TILs are expanded to a certain number in vitro, they can be infused back into the animal body to achieve the effect of controlling tumor growth and metastasis.

The present invention is suitable for isolating TIL from any tumor tissue, including tumor tissue obtained by open surgery or minimally invasive surgery. Exemplary tumor tissues are tumor tissues of cancers, such as gastric cancers, thyroid tumors, gallbladder cancers, cholangiocarcinomas, lung cancers, melanomas, head and neck cancers, breast cancers, ovarian cancers, cervical cancers, liver cancers, colorectal cancers, cerebral gliomas, pancreatic cancers, bladder cancers, prostate cancers, renal cancers, osteosarcomas, etc.

### Summary of the Invention

The present invention provides culture compositions and media for expanding TILs. The culture composition comprises IL-2, IL-7, IL-15 and an immune checkpoint antibody or antigen-binding fragment thereof. In some embodiments, the culture composition further comprises IL-21, IL-12, GM-CSF, and any one, two, three or four of small molecules that increase the proportion of memory T cells. The small molecules that increase the proportion of memory T cells include but are not limited to TWS119.

The immune checkpoint antibody is selected from any one or more of a PD-1 antibody, a CTLA-4 antibody, a LAG3 antibody, a TIM3 antibody, a TIGIT antibody and a BTLA antibody.

The culture composition may also include any one or more of a CD3 antibody, a CD28 antibody, a CD137 antibody, a OX40 antibody, a GITR antibody, ICOS, a CD206 antibody and a CD40 antibody. Preferably, the culture composition further includes any one, two, three or four of a CD3 antibody, a CD28 antibody, a CD137 antibody and a GITR antibody. Antibodies in the culture composition or medium herein may exist in dispersed or immobilized form. For example, one or more of the antibodies described above can be coupled to a matrix (e.g., a solid matrix). In a certain exemplary embodiment, the solid matrix is a microsphere, such as magnetic beads. In an exemplary embodiment, the solid matrix is a plate for cell culture, such as a well-plate, a dish, a cell culture flask or a cell culture bag.

Herein, immune checkpoint antibodies are antibodies against protein molecules in immune cells that regulate autoimmune functions.

Herein, natural intact antibodies are tetramers comprising two identical heavy chain polypeptides and two identical light chain polypeptides, as known in the art. Each heavy chain comprises at least four domains: an amino-terminal variable domain VH, a constant domain CH1, a constant domain CH2 and a carboxy-terminal constant domain CH3. Each light chain comprises two domains: an amino-terminal variable domain VL and a carboxy-terminal constant domain CL. Each variable domain comprises three "complementarity determining regions": CDR1, CDR2 and CDR3, and four "framework" regions: FR1, FR2, FR3 and FR4. "Antigen-binding fragment" means a fragment of an antibody that specifically binds to a target antigen. Antibodies or antigen-binding fragments thereof include Fab fragments, Fab' fragments, F(ab')2 fragments, Fd' fragments, Fd fragments, Fv fragments, disulfide bonded Fv fragments, single chain antibodies (scFv), isolated CDRs or CDR groups, polypeptide-Fc fusion, single domain antibody, camel antibody, masking antibody, small module immune medicament, bifunctional antibody, nanobody, Humabody antibody.

In specific embodiments, the culture composition comprises IL-2, IL-7, IL-15, PD-1 antibody, IL-21, IL-12, CD3 antibody, CD28 antibody and GM-CSF. Alternatively, the culture composition comprises IL-2, IL-7, IL-15, PD-1 antibody, IL-21, IL-12, CD3 antibody, CD28 antibody, CD137 antibody and GITR antibody. Alternatively, the culture composition comprises IL-2, IL-7, IL-15, PD-1 antibody, CD3 antibody, CD28 antibody, CD137 antibody, GITR antibody, GM-CSF and TWS119.

The TIL expansion medium of the present invention comprises the culture composition for expanding TILs described herein, serum and/or platelets, and a basal medium. The culture compositions or media described herein may also include antibiotics. Preferably, the antibiotic is selected from one or both of penicillin and streptomycin.

In the culture composition or medium herein, the concentration of IL-2 is 200-6000IU/mL, such as 200, 300, 400, 500, 600, 700, 800, 900, 1000, 1500, 2000, 2500, 3000, 3500 , 4000, 4500, 5000, 5500, 6000IU/mL, or in a range between any two of these values; the concentration of IL-7 is 5-100ng/mL, such as 5, 10, 20, 30, 40, 50, 60, 70, 80, 90, 100ng/mL, or in a range between any two of these values; the concentration of IL-15 is 5-100ng/mL, such as 5, 10, 20, 30, 40, 50, 60, 70, 80, 90, 100ng/mL, or in a range between any two of these values; the concentration of PD-1 antibody is 1-100µg/mL, such as 1, 2, 3, 5, 7, 9, 10 , 20, 30, 40, 50, 60, 70, 80, 90, 100µg/mL, or in a range between any two of these values; the concentration of IL-21 is 5-100ng/mL, such as 5, 10, 20, 30, 40, 50, 60, 70, 80, 90, 100ng/mL, or in a range between any two of these values; the concentration of IL-12 is 5-100ng/mL, such as 5, 10, 20, 30, 40, 50, 60, 70, 80, 90, 100ng/mL, or in a range between any two of these values; the concentration of CD3 antibody is 0.5-10µg/mL, such as 0.5, 1, 2, 3 , 4, 5, 6, 7, 8, 9, 10 µg/mL, or in a range between any two of these values; the concentration of CD28 antibody is 0.5-10 µg/mL, such as 0.5, 1, 2, 3, 4 , 5, 6, 7, 8, 9, 10 µg/mL, or in a range between any two of these values; the concentration of CD137 antibody is 1-100 µg/mL, such as 1, 5, 10, 20, 30, 40 , 50, 60, 70, 80, 90, 100 µg/mL, or a range between any two of the above values; the concentration of GITR antibody in the culture composition is 1-100 µg/mL, such as 1, 5, 10, 20, 30, 40 , 50, 60, 70, 80, 90, 100 µg/mL, or a range between any two of the above values; the concentration of GM-CSF of culture compositions is 200-5000U/mL, such as 200, 300, 400, 500, 600, 700, 800, 900, 1000, 1500, 2000, 2500, 3000, 3500, 4000, 4500, 5000U/mL, or in a range between any two of these values; the concentration of TWS119 is 1-500µM, such as 1, 5, 10, 15, 20, 30, 40, 50, 60, 70, 80, 90, 100, 200, 300, 400, 500µM, or in a range between any two of these values.

In specific embodiments, the culture composition or medium described herein comprises: 200-6000 IU/mL of IL-2, 5-100 ng/mL of IL-7, 5-100 ng/mL of IL-15, and 3-100 µg/mL of a PD-1 antibody. Preferably, the culture composition or medium comprises 200-500 IU/mL of IL-2, 5-50 ng/mL of IL-7, 5-50 ng/mL of IL-15 and 3-50 µg/mL of a PD -1 antibody. More preferably, the culture composition or medium comprises 500 IU/mL of IL-2, 10 ng/mL of IL-7, 10 ng/mL of IL-15 and 3µg/mL of a PD -1 antibody. The culture medium also contains human AB serum, PS dual antibiotics and OpTmizer^{™}.

In another specific embodiment, the culture composition or medium described herein comprises: 200-6000 IU of IL-2: 5-100 ng/mL of IL-7: 5-100 ng/mL of IL-15: 1-100 µg /mL of PD-1 antibody. Preferably, the culture composition or medium comprises: 200-500 IU/mL of IL-2: 5-50 ng/mL of IL-7: 5-50 ng/mL of IL-15: 3-50 µg/mL of PD -1 antibody. More preferably, the culture composition or medium comprises: 500 IU/mL of IL-2: 5 ng/mL of IL-7: 10 ng/mL of IL-15: 1 µg/mL of PD-1 antibody. The culture medium also contains human AB serum, PS dual antibiotics and OpTmizer^{™}.

In another specific embodiment, the culture composition or medium comprises: 200-6000 IU/mL of IL-2, 5-100 ng/mL of IL-7, 5-100 ng/mL of IL-15, 3-100µg/mL of PD-1 antibody, 5-100ng/mL of IL-21, 5-100ng/mL of IL-12, 1-10µg/mL of CD3 antibody, 1-10µg/mL of CD28 antibody and 200-5000U /mL of GM-CSF. Preferably, the culture composition or medium comprises 200-500 IU/mL of IL-2, 5-50 ng/mL of IL-7, 5-50 ng/mL of IL-15, 3-50 µg/mL of PD- 1 antibody, 5-50ng/mL IL-21, 5-50ng/mL IL-12, 1-5µg/mL CD3 antibody, 1-5µg/mL CD28 antibody and 200-1000U/mL GM-CSF. More preferably, the culture composition or medium comprises 200 IU/mL of IL-2, 10 ng/mL of IL-7, 10 ng/mL of IL-15, 3 µg/mL of PD-1 antibody, 10 ng/mL of IL-21, 20 ng/mL of IL-12, 3 µg/mL of CD3 antibody, 3 µg/mL of CD28 antibody and 500 U/mL of GM-CSF. The culture medium also contains human AB serum, PS dual antibiotics and AIM-V.

In another or more embodiments, the culture composition or medium comprises: 200-6000 IU/mL of IL-2: 5-100 ng/mL of IL-7: 5-100 ng/mL of IL-15: 3-100 µg/mL of PD-1 antibody: 5-100ng/mL of IL-21: 5-100ng/mL of IL-12: 1-10 µg/mL of CD3 antibody: 0.5-10 µg/mL of CD28 antibody: 200-5000 U/mL of GM-CSF. Preferably, the culture composition or medium comprises: 200-500 IU/mL of IL-2: 5-50 ng/mL of IL-7: 5-50 ng/mL of IL-15: 3-50 µg/mL of PD-1 antibody: 5-50 ng/mL of IL-21: 5-50 ng/mL of IL-12: 1-5 µg/mL of CD3 antibody: 0.5-5 µg/mL CD28 antibody: 200-1000 U/mL GM-CSF. More preferably, the culture composition or medium comprises: 200 IU/mL of IL-2: 50 ng/mL of IL-7: 50 ng/mL of IL-15: 3 µg/mL of PD-1 antibody: 10 ng/mL of IL-21: 10 ng/mL of IL-12: 3 µg/mL of CD3 antibody: 0.5 µg/mL of CD28 antibody: 500 U/mL of GM-CSF. The culture medium also contains human AB serum, PS dual antibiotics and AIM-V.

In another specific embodiment, the culture composition or medium comprises: 200-6000 IU/mL of IL-2, 5-100 ng/mL of IL-7, 5-100 ng/mL of IL-15, 3-100 µg/mL of PD-1 antibody, 5-100 ng/mL of IL-21, 5-100 ng/mL of IL-12, 1-10 µg/mL of CD3 antibody, 1-10 µg/mL of CD28 antibody, 1-100 µg /mL of CD137 antibody and 1-100 µg/mL of GITR antibody. Preferably, the culture composition or medium comprises: 200-500 IU/mL of IL-2, 5-50 ng/mL of IL-7, 5-50 ng/mL of IL-15, 3-50 µg/mL of PD-1 antibody, 5-50 ng/mL of IL-21, 5-50 ng/mL of IL-12, 1-5 µg/mL of CD3 antibody, 1-5 µg/mL of CD28 antibody, 1-5 µg/mL of CD137 antibody and 1-5 µg/mL of GITR antibody. Preferably, the culture composition or medium comprises 400 IU/mL of IL-2, 10 ng/mL of IL-7, 10 ng/mL of IL-15, 3 µg/mL of PD-1 antibody, 10 ng/mL of IL-21, 20 ng/mL of IL-12, 3 µg/mL of CD3 antibody, 3 µg/mL of CD28 antibody, 3 µg/mL of CD137 antibody and 3 µg/mL of GITR antibody. The culture medium also contains autologous platelets, PS dual antibiotics and X-VIVO.

In another specific embodiment, the culture composition or medium comprises: 200-6000 IU/mL of IL-2: 5-100 ng/mL of IL-7: 5-100 ng/mL of IL-15: 1-100 µg/mL of PD-1 antibody: 5-100 ng/mL of IL-21: 5-100 ng/mL of IL-12: 0.5-10 µg/mL of CD3 antibody: 1-10 µg/mL of CD28 antibody: 1-100 µg /mL of CD137 antibody: 1-100 µg/mL of GITR antibody. Preferably, the culture composition or medium comprises 200-500 IU/mL of IL-2: 5-50 ng/mL of IL-7: 5-50 ng/mL of IL-15: 1-50 µg/mL of PD-1 antibody: 5-50 ng/mL of IL-21: 5-50 ng/mL of IL-12: 0.5-5 µg/mL of CD3 antibody: 1-5 µg/mL of CD28 antibody: 1-5 µg/mL of CD137 antibody: 1-5 µg/mL of GITR antibody. More preferably, the culture composition or medium comprises: 400 IU/mL of IL-2: 30 ng/mL of IL-7: 30 ng/mL of IL-15: 1 µg/mL of PD-1 antibody: 10ng/mL of IL-21: 15 ng/mL of IL-12: 0.5 µg/mL of CD3 antibody: 1 µg/mL of CD28 antibody: 2.5 µg/mL of CD137 antibody: 3 µg/mL of GITR antibody. The culture medium also contains autologous platelets, PS dual antibiotics and X-VIVO.

In another specific embodiments, the culture composition or medium comprises: 200-6000 IU/mL of IL-2, 5-100 ng/mL of IL-7, 5-100 ng/mL of IL-15, 3-100 µg/mL of PD-1 antibody, 1-10 µg/mL of CD3 antibody, 1-10 µg/mL of CD28 antibody, 1-100 µg/mL of CD137 antibody, 1-100 µg/mL of GITR antibody, 200-5000 U/mL of GM-CSF and 1-500 µM of TWS119. Preferably, the culture composition or medium comprises 200-500 IU/mL of IL-2, 5-50 ng/mL of IL-7, 5-50 ng/mL of IL-15, 3-50 µg/mL of PD- 1 antibody, 1-5 µg/mL CD3 antibody, 1-5 µg/mL CD28 antibody, 1-5 µg/mL CD137 antibody, 1-5 µg/mL GITR antibody, 200-500 U/mL GM-CSF and 1-100 µM of TWS119. More preferably, the culture composition or medium comprises 300 IU/mL of IL-2, 10 ng/mL of IL-7, 10 ng/mL of IL-15, 3 µg/mL of PD-1 antibody, 3 µg/mL of CD3 antibody, 3 µg/mL of CD28 antibody, 3 µg/mL of CD137 antibody, 3 µg/mL of GITR antibody, 200 U/mL of GM-CSF and 10 µ M of TWS 119; or more preferably, the culture composition or medium: 300 IU/mL of IL-2: 40 ng/mL of IL-7: 40 ng/mL of IL-15: 3 µg/mL of PD-1 antibody: 1 µg/mL of CD3 antibody: 1 µg/mL of CD28 antibody: 5 µg/mL of CD137 antibody: 2 µg/mL of GITR antibody: 200 U/mL of GM-CSF: 10 µM of TWS119. The culture medium also contains allogeneic platelets, PS dual antibiotics and MHM-C.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibody: CD3 antibodies coupled to a matrix: CD28 antibodies coupled to a matrix: 200-5000 U/mL of GM-CSF. Preferably, the culture composition comprises components in the following proportion: 200-500 IU/mL of IL-2: 5-50 ng/mL of IL-7: 5-50 ng/mL of IL-15: 1-50 µg/mL of PD-1 antibody: CD3 antibodies coupled to a matrix: CD28 antibodies coupled to a matrix: 200-1000 U/mL of GM-CSF. More preferably, the culture composition comprises components in the following proportion: 500 IU/mL of IL-2: 5 ng/mL of IL-7: 10 ng/mL of IL-15: 3 µg/mL of PD-1 antibody: CD3 antibody coupled to a cell culture dish or a cell culture bag: CD28 antibody coupled to a cell culture dish or a cell culture bag: 500 U/mL of GM-CSF.

In another specific embodiment, the culture composition comprises components: 200-6000 IU/mL of IL-2, 5-100 ng/mL of IL-7, 5-100 ng/mL of IL-15, 1-100 µg/mL of PD-1 antibody, CD3 antibodies coupled to a matrix, CD28 antibodies coupled to a matrix, 1-100 µg/mL of CD137 antibody and 1-100 µg/mL of GITR antibody, or the proportional concentration of these components. Preferably, the culture composition comprises components: 200-500 IU/mL of IL-2, 5-50 ng/mL of IL-7, 5-50 ng/mL of IL-15, 1-50 µg/mL of PD-1 antibody, CD3 antibodies coupled to a matrix, CD28 antibodies coupled to a matrix and 200-1000 U/mL of GM-CSF, or the proportional concentration of these components. More preferably, the culture composition comprise component: 500 IU/mL of IL-2, 5 ng/mL of IL-7, 10 ng/mL of IL-15, 3 µg/mL of PD-1 antibody, 3 µg/mL of CD3 antibody, 0.5 µg/mL of CD28 antibody and 500 U/mL of GM-CSF, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibody: 5-100ng/mL of IL-21: 5-100 ng/mL of IL-12: 0.5-10 µg/mL of CD3 antibody: 0.5-10 µg/mL of CD28 antibody: 200-5000 U/mL of GM-CSF. Preferably, the culture composition comprises components in the following proportion: 200-500 IU/mL of IL-2: 5-50 ng/mL of IL-7: 5-50 ng/mL of IL-15: 1-50 µg/mL of PD-1 antibody: 5-50ng/mL of IL-21: 5-50ng/mL of IL-12: 0.5-5 µg/mL of CD3 antibody: 0.5-5 µg/mL of CD28 antibody: 200-1000 U/mL of GM-CSF. More preferably, the culture composition comprises components in the following proportion: 500IU/mL of IL-2: 5ng/mL of IL-7: 10ng/mL of IL-15: 3 µg/mL of PD-1 antibody: 10ng/mL of IL-21: 10 ng/mL of IL-12: 3 µg/mL of CD3 antibody: 0.5 µg/mL of CD28 antibody: 500 U/mL of GM-CSF.

In another specific embodiment, the culture composition comprises components: 200-6000 IU/mL of IL-2, 5-100 ng/mL of IL-7, 5-100 ng/mL of IL-15, 1-100 µg/mL of PD-1 antibody, 5-100ng/mL of IL-21, 5-100ng/mL of IL-12, CD3 antibodies coupled to a matrix, CD28 antibodies coupled to a matrix, 1-100 µg/mL of CD137 antibody and 1-100 µg/mL of GITR antibody, or the proportional concentration of these components. Preferably, the culture composition comprises components: 200-500 IU/mL of IL-2, 5-50 ng/mL of IL-7, 5-50 ng/mL of IL-15, 1-50 µg/mL of PD-1 antibody, 5-50ng/mL of IL-21, 5-50ng/mL of IL-12, CD3 antibodies coupled to a matrix, CD28 antibodies coupled to a matrix and 200-1000U /mL of GM-CSF, or the proportional concentration of these components. More preferably, the culture composition comprise component: 500 IU/mL of IL-2, 5 ng/mL of IL-7, 10 ng/mL of IL-15, 3 µg/mL of PD-1 antibody, 10 ng/mL of IL-21, 10 ng/mL of IL-12, 3 µg/mL of CD3 antibody, 0.5 µg/mL of CD28 antibody and 500 U/mL of GM-CSF, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibody: CD3 antibodies coupled to a matrix: CD28 antibodies coupled to a matrix: CD137 antibodies coupled to a matrix. Preferably, the culture composition comprises components in the following proportion: 200-500 IU/mL of IL-2: 5-50 ng/mL of IL-7: 5-50 ng/mL of IL-15: 1-50 µg/mL of PD-1 antibody: CD3 antibodies coupled to a matrix: CD28 antibodies coupled to a matrix: CD137 antibodies coupled to a matrix. More preferably, the culture composition comprises components in the following proportion: 500 IU/mL of IL-2: 50 ng/mL of IL-7: 30 ng/mL of IL-15: 1 µg/mL of PD-1 antibody: CD3 antibody coupled to a cell culture dish or a cell culture bag: CD28 antibody coupled to a cell culture dish or a cell culture bag: CD137 antibody coupled to a cell culture dish or a cell culture bag.

In another specific embodiment, the culture composition comprises components: 200-6000 IU/mL of IL-2, 5-100 ng/mL of IL-7, 5-100 ng/mL of IL-15, 1-100 µg/mL of PD-1 antibody, CD3 antibodies coupled to a matrix, CD28 antibodies coupled to a matrix and CD137 antibodies coupled to a matrix, or the proportional concentration of these components. Preferably, the culture composition comprises component: 200-500 IU/mL of IL-2, 5-50 ng/mL of IL-7, 5-50 ng/mL of IL-15, 1-50 µg/mL of PD-1 antibody, CD3 antibodies coupled to a matrix, CD28 antibodies coupled to a matrix and CD137 antibodies coupled to a matrix, or the proportional concentration of these components. More preferably, the culture composition comprises components: 500 IU/mL of IL-2, 50 ng/mL of IL-7, 30 ng/mL of IL-15, 1 µg/mL of PD-1 antibody, CD3 antibody coupled to a cell culture dish or a cell culture bag, CD28 antibody coupled to a cell culture dish or a cell culture bag and CD137 antibody coupled to a cell culture dish or a cell culture bag, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibody: CD3 antibodies coupled to a matrix: CD28 antibodies coupled to a matrix: CD137 antibodies coupled to a matrix: 1-100 µg/mL of GITR antibody: 1-500 µM of TWS119. Preferably, the culture composition comprises components in the following proportion: 200-500 IU/mL of IL-2: 5-50 ng/mL of IL-7: 5-50 ng/mL of IL-15: 1-50 µg/mL of PD-1 antibody: CD3 antibodies coupled to a matrix: CD28 antibodies coupled to a matrix: CD137 antibodies coupled to a matrix: 1-5 µg/mL of GITR antibody: 1-100 µM of TWS119. More preferably, the culture composition comprises components in the following proportion: 300 IU/mL of IL-2: 10 ng/mL of IL-7: 25 ng/mL of IL-15: 2 µg/mL of PD-1 antibody: CD3 antibodies coupled to a matrix: CD28 antibodies coupled to a matrix: CD137 antibodies coupled to a matrix: 3 µg/mL of GITR antibody: 10 µM of TWS119.

In another specific embodiment, the culture composition comprises components: 200-6000 IU/mL of IL-2, 5-100 ng/mL of IL-7, 5-100 ng/mL of IL-15, 1-100 µg/mL of PD-1 antibody, CD3 antibodies coupled to a matrix, CD28 antibodies coupled to a matrix, CD137 antibodies coupled to a matrix, 1-100 µg/mL of GITR antibody and 1-500 µM of TWS119, or the proportional concentration of these components. Preferably, the culture composition comprises components: 200-500 IU/mL of IL-2, 5-50 ng/mL of IL-7, 5-50 ng/mL of IL-15, 1-50 µg/mL of PD-1 antibody, CD3 antibodies coupled to a matrix, CD28 antibodies coupled to a matrix, CD137 antibodies coupled to a matrix, 1-5 µg/mL of GITR antibody and 1-100 µM of TWS119, or the proportional concentration of these components. More preferably, the culture composition comprises component: 300 IU/mL of IL-2, 10 ng/mL of IL-7, 25 ng/mL of IL-15, 2 µg/mL of PD-1 antibody, CD3 antibodies coupled to a matrix, CD28 antibodies coupled to a matrix, CD137 antibodies coupled to a matrix, 3 µg/mL of GITR antibody and 10 µM of TWS119, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibody: CD3 antibodies coupled to a matrix: CD28 antibodies coupled to a matrix: CD137 antibodies coupled to a matrix: 1-100 µg/mL of TIGIT antibody: 200-5000 U/mL of GM-CSF. Preferably, the culture composition comprises components in the following proportion: 200-500 IU/mL of IL-2: 5-50 ng/mL of IL-7: 5-50 ng/mL of IL-15: 1-50 µg/mL of PD-1 antibody: CD3 antibodies coupled to a matrix: CD28 antibodies coupled to a matrix: CD137 antibodies coupled to a matrix: 1-5 µg/mL of TIGIT antibody: 200-500 U/mL of GM-CSF. More preferably, the culture composition comprises components in the following proportion: 500 IU/mL of IL-2: 7 ng/mL of IL-7: 50 ng/mL of IL-15: 2 µg/mL of PD-1 antibody: CD3 antibodies coupled to a matrix: CD28 antibodies coupled to a matrix: CD137 antibodies coupled to a matrix: 2.5 µg/mL of TIGIT antibody: 500 U/mL of GM-CSF.

In another specific embodiment, the culture composition comprises components: 200-6000 IU/mL of IL-2, 5-100 ng/mL of IL-7, 5-100 ng/mL of IL-15, 1-100 µg/mL of PD-1 antibody, CD3 antibodies coupled to a matrix, CD28 antibodies coupled to a matrix, CD137 antibodies coupled to a matrix, 1-100 µg/mL of TIGIT antibody and 200-5000 U/mL of GM-CSF, or the proportional concentration of these components. Preferably, the culture composition comprises component: 200-500 IU/mL of IL-2, 5-50 ng/mL of IL-7, 5-50 ng/mL of IL-15, 1-50 µg/mL of PD-1 antibody, CD3 antibodies coupled to a matrix, CD28 antibodies coupled to a matrix, CD137 antibodies coupled to a matrix, 1-5 µg/mL of TIGIT antibody and 200-500 U/mL of GM-CSF, or the proportional concentration of these components. More preferably, the culture composition comprises component: 500 IU/mL of IL-2, 7 ng/mL of IL-7, 50 ng/mL of IL-15, 2 µg/mL of PD-1 antibody, CD3 antibodies coupled to a matrix, CD28 antibodies coupled to a matrix, CD137 antibodies coupled to a matrix, 2.5 µg/mL of TIGIT antibody and 500 U/mL of GM-CSF, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of LAG-3 antibody: 5-100ng/mL of IL-21: 5-100ng/mL of IL-12: 0.5-10 µg/mL of CD3 antibody: 0.5-10 µg/mL of CD28 antibody: 1-100 µg/mL of CD137 antibody: 1-100 µg/mL of GITR antibody. Preferably, the culture composition comprises components in the following proportion: 200-500 IU/mL of IL-2: 5-50 ng/mL of IL-7: 5-50 ng/mL of IL-15: 1-50 µg/mL of LAG-3 antibody: 5-50ng/mL of IL-21: 5-50ng/mL of IL-12: 0.5-5 µg/mL of CD3 antibody: 0.5-5 µg/mL of CD28 antibody: 1-5 µg/mL of CD137 antibody: 1-5 µg/mL of GITR antibody. More preferably, the culture composition comprises components in the following proportion: 400 IU/mL of IL-2: 30 ng/mL of IL-7: 30 ng/mL of IL-15: 3 µg/mL of LAG-3 antibody: 10 ng/mL of IL-21: 15 ng/mL of IL-12: 0.5 µg/mL of CD3 antibody: 1 µg/mL of CD28 antibody: 2.5 µg/mL of CD137 antibody: 3 µg/mL of GITR antibody.

In another specific embodiment, the culture composition comprises components: 200-6000 IU/mL of IL-2, 5-100 ng/mL of IL-7, 5-100 ng/mL of IL-15, 1-100 µg/mL of LAG-3 antibody, 5-100ng/mL of IL-21, 5-100ng/mL of IL-12, 0.5-10 µg/mL of CD3 antibody, 0.5-10 µg/mL of CD28 antibody, 1-100 µg/mL of CD137 antibody and 1-100 µg/mL of GITR antibody, or the proportional concentration of these components. Preferably, the culture composition comprises components: 200-500 IU/mL of IL-2, 5-50 ng/mL of IL-7, 5-50 ng/mL of IL-15, 1-50 µg/mL of LAG-3 antibody, 5-50ng/mL of IL-21, 5-50ng/mL of IL-12, 0.5-5 µg/mL of CD3 antibody, 0.5-5 µg/mL of CD28 antibody, 1-5 µg/mL of CD137 antibody and 1-5 µg/mL of GITR antibody, or the proportional concentration of these components. More preferably, the culture composition comprise component: 400 IU/mL of IL-2, 30 ng/mL of IL-7, 30 ng/mL of IL-15, 3 µg/mL of LAG-3 antibody, 10 ng/mL of IL-21, 15 ng/mL of IL-12, 0.5 µg/mL of CD3 antibody, 1 µg/mL of CD28 antibody, 2.5 µg/mL of CD137 antibody and 3 µg/mL of GITR antibody, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibody: CD3 antibodies coupled to a matrix: CD28 antibodies coupled to a matrix: CD137 antibodies coupled to a matrix: 1-100 µg/mL of TIGIT antibody: 1-100 µg/mL of CD40 antibody: 1-100 µg/mL of OX-40 antibody: 1×10⁸-5×10⁸/mL of autologous platelets. Preferably, the culture composition comprises components in the following proportion: 200-500 IU/mL of IL-2: 5-50ng/mL of IL-7: 5-50ng/mL of IL-15: 1-50 µg/mL of PD-1 antibody: CD3 antibodies coupled to a matrix: CD28 antibodies coupled to a matrix: CD137 antibodies coupled to a matrix: 1-50 µg/mL of TIGIT antibody: 1-50 µg/mL of CD40 antibody: 1-50 µg/mL of OX-40 antibody: 1×10⁸-3×10⁸/mL of autologous platelets. More preferably, the culture composition comprises components in the following proportion: 500 IU/mL of IL-2: 5ng/mL of IL-7: 25 ng/mL of IL-15: 3 µg/mL of PD-1 antibody: CD3 antibodies coupled to a cell culture dish or a cell culture bag: CD28 antibodies coupled to a cell culture dish or a cell culture bag: CD137 antibodies coupled to a cell culture dish or a cell culture bag: 2.5 µg/mL of TIGIT antibody: 2 µg/mL of CD40 antibody: 3 µg/mL of OX-40 antibody: 3×10⁸/mL of autologous platelets.

In another specific embodiment, the culture composition comprises components in the following proportion: 200-6000 IU/mL of IL-2: 5-100 ng/mL of IL-7: 5-100 ng/mL of IL-15: 1-100 µg/mL of PD-1 antibody: CD3 antibodies coupled to a matrix: CD28 antibodies coupled to a matrix: CD137 antibodies coupled to a matrix: 1-100 µg/mL of TIGIT antibody: 1-100 µg/mL of CD40 antibody: 1-100 µg/mL of OX-40 antibody: 1×10⁸-5×10⁸/mL of autologous platelets, or the proportional concentration of these components. Preferably, the culture composition comprises components: 200-500 IU/mL of IL-2, 5-50ng/mL of IL-7, 5-50ng/mL of IL-15, 1-50 µg/mL of PD-1 antibody, CD3 antibodies coupled to a matrix, CD28 antibodies coupled to a matrix, CD137 antibodies coupled to a matrix, 1-50 µg/mL of TIGIT antibody, 1-50 µg/mL of CD40 antibody, 1-50 µg/mL of OX-40 antibody and 1×10⁸-3×10⁸/mL of autologous platelets, or the proportional concentration of these components. More preferably, the culture composition comprises components: 500 IU/mL of IL-2, 5 ng/mL of IL-7, 25 ng/mL of IL-15, 3 µg/mL of PD-1 antibody, CD3 antibodies coupled to a matrix, CD28 antibodies coupled to a matrix, CD137 antibodies coupled to a matrix, 2.5 µg/mL of TIGIT antibody, 2 µg/mL of CD40 antibody, 3 µg/mL of OX-40 antibody and 3×10⁸/mL of autologous platelets, or the proportional concentration of these components.

In another or more embodiments, the culture composition comprises components in the following proportion: 200-6000 IU/mL of IL-2: 5-100 ng/mL of IL-7: 5-100 ng/mL of IL-15: 1-100 µg/mL of PD-1 antibody: 1-100 µg/mL of CTLA-4 antibody: CD3 antibodies coupled to a matrix: CD28 antibodies coupled to a matrix: 1-100 µg/mL of CD137 antibody: 1-100 µg/mL of GITR antibody: 200-5000 U/mL of GM-CSF: 1×10⁸-5×10⁸/mL of autologous platelets. Preferably, the culture composition comprises components in the following proportion: 200-500 IU/mL of IL-2: 5-50ng/mL of IL-7: 5-50ng/mL of IL-15: 1-50 µg/mL of PD-1 antibody: 1-50 µg/mL of CTLA-4 antibody: CD3 antibodies coupled to a matrix: CD28 antibodies coupled to a matrix: 1-50 µg/mL of CD137 antibody: 1-50 µg/mL of GITR antibody: 200-500 U/mL of GM-CSF: 1×10⁸-3×10⁸/mL of allogeneic platelets. More preferably, the culture composition comprises components in the following proportion: 300 IU/mL of IL-2: 10 ng/mL of IL-7: 30 ng/mL of IL-15: 1 µg/mL of PD-1 antibody: 2 µg/mL of CTLA-4 antibody: CD3 antibodies coupled to a matrix: CD28 antibodies coupled to a matrix: 5 µg/mL of CD137 antibody: 2 µg/mL of GITR antibody: 200 U/mL of GM-CSF: 1×10⁸/mL of allogeneic platelets.

In another specific embodiment, the culture composition comprises component: 200-6000 IU/mL of IL-2, 5-100 ng/mL of IL-7, 5-100 ng/mL of IL-15, 1-100 µg/mL of PD-1 antibody, 1-100 µg/mL of CTLA-4 antibody, CD3 antibodies coupled to a matrix, CD28 antibodies coupled to a matrix, 1-100 µg/mL of CD137 antibody, 1-100 µg/mL of GITR antibody, 200-5000 U/mL of GM-CSF, 1×10⁸-5×10⁸/mL of allogeneic platelets, or the proportional concentration of these components. Preferably, the culture composition comprises component: 200-500 IU/mL of IL-2, 5-50ng/mL of IL-7, 5-50ng/mL of IL-15, 1-50 µg/mL of PD-1 antibody, 1-50 µg/mL of CTLA-4 antibody, CD3 antibodies coupled to a matrix, CD28 antibodies coupled to a matrix, 1-50 µg/mL of CD137 antibody, 1-50 µg/mL of GITR antibody, 200-500 U/mL of GM-CSF, 1×10⁸-3×10⁸/mL of allogeneic platelets, or the proportional concentration of these components. More preferably, the culture composition comprises component: 300 IU/mL of IL-2, 10 ng/mL of IL-7, 30 ng/mL of IL-15, 1 µg/mL of PD-1 antibody, 2 µg/mL of CTLA-4 antibody, CD3 antibodies coupled to a matrix, CD28 antibodies coupled to a matrix, 5 µg/mL of CD137 antibody, 2 µg/mL of GITR antibody, 200 U/mL of GM-CSF, 1×10⁸/mL of allogeneic platelets, or the proportional concentration of these components.

The antibodies described herein may be antibodies from any species or recombinant antibodies, preferably monoclonal antibodies. These antibodies are well known in the art, such as PD-1 antibody Tislelizumab, PD-1 antibody Sintilimab, PD-1 antibody Toripalimab, PD-1 antibody Camrelizumab, CD3 antibody ab86883, CD28 antibody MAB342, GITR antibody shown in SEQ ID NO: 104 and SEQ ID NO: 105 in CN103951753B, CD137 antibody shown in SEQ ID NO: 10 and SEQ ID NO: 11 in CN111182919A. These patent documents or publications are hereby incorporated by reference in their entirety. _{∘}

In the above induction composition or culture medium, the concentration ratio of CD3 antibody to CD28 antibody is between 3:1 and 1:6, between 2:1 and 1:5, and between 1:1 and 1:4. In the above amplification composition or culture medium, the concentration ratio of CD3 antibody to CD28 antibody is between 3:1 and 1:6, between 2:1 and 1:5, and between 1:1 and 1:4; the concentration ratio of CD3 antibody to CD137 antibody is between 3:1 and 1:6, between 2:1 and 1:5, and between 1:1 and 1:4.

The serum described herein may be any serum known in the art that can be used for culturing animal cells (such as TILs), such as human AB serum, autologous serum or animal-derived serum. The concentration of serum is 1-10%, such as 1%, 2%, 3%, 4%, 5%, 6%, 7%, 8%, 9%, 10%, or in a range between any two of these values.

The platelets described herein may be platelets derived from the blood of the subject or platelets derived from allogeneic blood. The density of platelets is 0.5×10⁸/mL-3×10⁸/mL, such as 0.5×10⁸/mL, 1×10⁸/mL, 1.5×10⁸/mL, 2×10⁸/mL, 2.5×10⁸/mL, 3×10⁸/mL, or in a range between any two of these values.

The "basal medium" described herein is any medium known in the art that can be used for TIL culture, including but not limited to AIM-V, X-VIVO, DMEM, RPMI1640, OpTmizer, MHM-C. The components and contents of these media are well known in the art and their sources are described in the examples.

The TIL seed cell medium used in the method herein can be any medium known in the art that can induce TILs to extravasate from tumor tissue. An exemplary TIL seed cell culture medium is described in PCT/CN2021/096585, which is incorporated herein by reference in its entirety.

For example, the TIL seed cell culture medium described herein includes: cell culture components, cytokines and immune checkpoint antibodies or antigen-binding fragments thereof, wherein the cytokines include IL-2, the immune checkpoint antibodies or antigen-binding fragments thereof including PD-1 antibody or its antigen-binding fragment, the cell culture components are serum medium or serum-free medium. The concentration of IL-2 is about 3000 IU/mL and below or about 200 IU/mL and below. In one aspect, the present application provides a seed cell culture medium for tumor-infiltrating lymphocytes, which includes the following components: cell culture components, cytokines and immune checkpoint antibodies or antigen-binding fragments thereof, wherein the cytokines include IL -2; the immune checkpoints include: PD-1, LAG-3, TIGIT and/or CTLA-4, and the cell culture components are serum medium or serum-free medium.

In the present application, the cytokines may be human-derived cytokines.

In the present application, the immune checkpoint antibody or antigen-binding fragment thereof may be a humanized and/or fully human antibody or antigen-binding fragment thereof.

For example, the concentration of IL-2 may be about 3000 IU/mL and below (for example, about 2900 IU/mL and below, about 2800 IU/mL and below, about 2700 IU/mL and below, about 2600 IU/mL and below, about 2500 IU/mL and below , about 2400 IU/mL and below, about 2300 IU/mL and below, about 2200 IU/mL and below, about 2100 IU/mL and below, about 2000 IU/mL and below, about 1900 IU/mL and below, about 1800 IU/mL and below, about 1700 IU/mL and below, about 1600 IU/mL and below, about 1500 IU/mL and below, about 1400 IU/mL and below, about 1300 IU/mL and below, about 1100 IU/mL and below, about 1100 IU/mL and below, about 1000 IU/mL and below, about 900 IU/mL and below, about 800 IU/mL and below, about 700 IU/mL and below, about 600 IU/mL and below, about 500 IU/mL and below, about 400 IU/mL and below, about 300 IU/mL and below or lower). For example, the concentration of IL-2 may be about 2000 IU/mL and below, may be about 2500 IU/mL and below, or may be about 3000 IU/mL and below.

For example, the cytokine can include IL-7.

For example, the concentration of IL-7 can be about 5 to about 100 ng/mL (for example, about 5 to about 95 ng/mL, about 5 to about 90 ng/mL, about 5 to about 85 ng/mL, about 5 to about 80 ng/mL, about 5 to about 75 ng/mL, about 5 to about 70 ng/mL, about 5 to about 65 ng/mL, about 5 to about 60 ng/mL, about 5 to about 55 ng/mL, about 5 to about 50 ng/mL, about 5 to about 45 ng/mL, about 5 to about 40 ng/mL, about 5 to about 35 ng/mL, about 5 to about 30 ng/mL, about 5 to about 25 ng/mL, about 5 to about 20 ng/mL or about 5 to about 10 ng/mL). For example, the concentration of IL-7 can be about 200 to about 1000 U/mL (eg, can be at least about 200 U/mL, at least about 500 U/mL, or at least about 1000 U/mL). In the present application, the concentration of IL-7 may be about 200-1000 IU/mL. For example, it can be at least about 200 IU/mL, at least about 500 IU/mL or at least about 1000 IU/mL.

For example, the cytokine can include IL-15.

For example, the concentration of IL-15 can be about 5 to about 100 ng/mL (for example, about 5 to about 95 ng/mL, about 5 to about 90 ng/mL, about 5 to about 85 ng/mL, about 5 to about 80 ng/mL, about 5 to about 75 ng/mL, about 5 to about 70 ng/mL, about 5 to about 65 ng/mL, about 5 to about 60 ng/mL, about 5 to about 55 ng/mL, about 5 to about 50 ng/mL, about 5 to about 45 ng/mL, about 5 to about 40 ng/mL, about 5 to about 35 ng/mL, about 5 to about 30 ng/mL, about 5 to about 25 ng/mL, about 5 to about 20 ng/mL or about 5 to about 10 ng/mL). For example, the concentration of IL-15 can be about 200 to about 500 U/mL (eg, can be at least about 200 U/mL, at least about 250 U/mL, at least about 300 U/mL, or at least about 500 U/mL). In the present application, the concentration of IL-15 may be about 200-500 IU/mL. For example, it can be at least about 200 IU/mL, at least about 250 IU/mL, at least about 300 IU/mL or at least about 500 IU/mL.

For example, the cytokine can include tumor necrosis factor TNF.

For example, the cytokine can include TNF alpha.

For example, the concentration of TNF alpha can be about 10 to about 100 pg/mL (for example, can be about 10 to about 95 pg/mL, about 10 to about 90 pg/mL, about 10 to about 80 pg/mL, about 10 to about 70 pg/mL, about 10 to about 60 pg/mL, about 10 to about 50 pg/mL, about 10 to about 40 pg/mL, about 15 to about 40 pg/mL, about 10 to about 25 pg/mL, about 15 to about 25 pg/mL , about 10 to about 30 pg/mL, about 10 to about 20 pg/mL or about 10 to about 15 pg/mL). In the present application, the concentration of TNF-alpha may be about 10-1000 pg/mL. For example, it can be about 10-100 pg/mL, about 15-100 pg/mL, about 20-100 pg/mL or about 25-100 pg/mL. For example, it can be at least about 10 pg/mL, at least about 15 pg/mL, at least about 20 pg/mL, or at least about 25 pg/mL.

For example, the cytokine can include a colony stimulating factor.

For example, the cytokines may include GM-CSF, G-CSF and/or M-CSF.

For example, the concentration of GM-CSF can be about 200 to about 5000 U/mL (for example, about 200 to about 800 U/mL, about 200 to about 500 U/mL, about 300 to about 5000 U/mL, about 500 to about 5000 U/mL, about 1000 to about 5000 U/mL, about 1500 to about 5000 U/mL, about 2000 to about 5000 U/mL, about 2500 to about 5000 U/mL, about 3000 to about 5000 U/mL, about 3500 to about 5000 U /mL, about 4000 to about 5000 U/mL or about 4500 to about 5000 U/mL). For example, it can be about 200 to about 1000 U/mL, it can be about 200 to about 800 U/mL, it can be about 200 to about 500 U/mL. For example, it can be at least about 200 U/mL, at least about 500 U/mL or at least about 800 U/mL.

For example, the concentration of G-CSF may be about 300 U/mL to about 1000 U/mL. For example, the concentration of G-CSF may be about 500-1000 U/mL. For example, it may be at least about 500 U/mL or at least about 1000 U/mL.

For example, the concentration of M-CSF may be about 300 U/mL to about 800 U/mL. For example, the concentration of M-CSF may be about 300 U/mL to about 500 U/mL. For example, it can be at least about 300 U/mL, at least about 500 U/mL or at least about 800 U/mL.

For example, the cytokine can include interferons.

For example, the cytokines may include IFN-gamma, IFN-alpha and/or IFN-beta.

For example, the concentration of IFN-gamma can be about 10 to about 1000 ng/mL (for example, about 15 to about 1000 ng/mL, about 20 to about 1000 ng/mL, about 30 to about 1000 ng/mL, about 40 to about 1000 ng/mL, about 50 to about 1000 ng/mL, about 60 to about 1000 ng/mL, about 70 to about 1000 ng/mL, about 80 to about 1000 ng/mL, about 90 to about 1000 ng/mL, about 100 to about 1000 ng/mL, about 200 to about 1000 ng/mL, about 300 to about 1000 ng/mL, about 400 to about 1000 ng/mL, about 500 to about 1000 ng/mL, about 600 to about 1000 ng/mL, about 700 to about 1000 ng/mL , about 800 to about 1000 ng/mL or about 900 to about 1000 ng/mL). For example, the concentration of IFN-gamma may be about 10-1000 ng/mL. For example, the concentration of IFN-gamma can be about 300 to about 1000 ng/mL, about 300 to about 800 ng/mL or about 300 to about 500 ng/mL. For example, it can be at least about 300 ng/mL, at least about 500 ng/mL, at least about 800 ng/mL or at least about 1000 ng/mL.

For example, the concentration of IFN-alpha may be about 500 U/mL to about 1000 U/mL. For example, it can be at least about 500 ng/mL.

For example, the concentration of IFN-beta may be about 200 U/mL to about 500 U/mL. For example, the concentration of IFN-beta may be about 250-500 ng/mL. For example, it can be at least about 200 ng/mL, at least about 250 ng/mL or at least about 500 ng/mL.

For example, the cytokines may also include IL-4, IL-1 alpha, IL-1 beta, IL-6, IL-9, IL-18, IL-12, IL-21 and/or IL-10.

For example, the concentration of IL-4 may be about 200 to about 500 U/mL. For example, it may be at least about 200 IU/mL or at least about 500 IU/mL.

For example, the concentration of IL-1 alpha may be about 200 to about 500 U/mL. For example, it may be at least about 200 IU/mL or at least about 500 IU/mL.

For example, the concentration of IL-1 beta may be about 200 to about 500 U/mL. For example, it may be at least about 200 IU/mL or at least about 500 IU/mL.

For example, the concentration of IL-6 may be about 200 to about 500 U/mL. For example, it may be at least about 200 IU/mL or at least about 500 IU/mL.

For example, the concentration of IL-9 may be about 200 to about 500 U/mL. For example, it may be at least about 200 IU/mL or at least about 500 IU/mL.

For example, the concentration of IL-18 may be about 200 to about 500 U/mL. For example, it may be at least about 200 IU/mL or at least about 500 IU/mL.

For example, the concentration of IL-12 may be about 200 to about 1000 U/mL. For example, the concentration of IL-12 can be about 200 to about 1000 U/mL, about 200 to about 500 U/mL, or about 300 to about 1000 U/mL. For example, it can be at least about 200 IU/mL, at least about 250 IU/mL, at least about 300 IU/mL or at least about 500 IU/mL.

For example, the concentration of IL-21 may be about 200 to about 500 U/mL. For example, the concentration of IL-21 may be about 250 to about 1000 U/mL. For example, it can be at least about 200 IU/mL, at least about 250 IU/mL or at least about 500 IU/mL.

For example, the concentration of IL-10 may be about 200 to about 500 U/mL. For example, it can be at least about 200 IU/mL, at least about 250 IU/mL, at least about 300 IU/mL or at least about 500 IU/mL.

For example, the immune checkpoint antibody or antigen-binding fragment thereof may be a PD-1 antibody or antigen-binding fragment thereof.

For example, the PD-1 antibody or antigen-binding fragment thereof can be a human PD-1 antibody or antigen-binding fragment thereof.

For example, the concentration of the PD-1 antibody or antigen-binding fragment thereof may be about 1 to about 100 µg/mL (for example, about 3 to about 100 µg/mL, about 5 to about 100 µg/mL, about 10 to about 100 µg/mL, can be about 15 to about 100 µg/mL, about 16 to about 100 µg/mL, about 17 to about 100 µg/mL, about 18 to about 100 µg/mL, about 19 to about 100 µg/mL, about 20 to about 100µg/mL, about 25 to about 100µg/mL, about 30 to about 100µg/mL, about 36 to about 100µg/mL, about 40 to about 100µg/mL, about 45 to about 100 µg/mL, about 50 to about 100 µg/mL, about 55 to about 100 µg/mL, about 60 to about 100 µg/mL, about 65 to about 100 µg/mL, about 70 to about 100 µg/mL, about 80 to about 100 µg/mL, or about 90 to about 100 µg /mL). In the present application, the concentration of PD-1 mAb may be about 3-100 µg/mL. For example, it can be at least about 3 µg/mL, at least about 5 µg/mL, at least about 10 µg/mL, at least about 15 µg/mL, at least about 20 µg/mL, at least about 25 µg/mL, at least about 36 µg/mL, at least about 50 µg/mL or at least about 100 µg/mL.

For example, the immune checkpoint antibody or antigen-binding fragment thereof may be a human LAG-3 antibody or antigen-binding fragment thereof.

For example, the immune checkpoint antibody or antigen-binding fragment thereof can be a LAG-3 antibody or antigen-binding fragment thereof, and the concentration of the LAG-3 antibody or antigen-binding fragment thereof is about 3 to about 10 µg/mL. For example, it can be at least about 5-10 µg/mL. For example, it can be at least about 3 µg/mL, at least about 5 µg/mL or at least about 10 µg/mL.

For example, the TIGIT antibody or antigen-binding fragment thereof can be a human TIGIT antibody or antigen-binding fragment thereof.

For example, the immune checkpoint antibody or antigen-binding fragment thereof can be a TIGIT antibody or antigen-binding fragment thereof, and the concentration of the TIGIT antibody or antigen-binding fragment thereof is about 1 to about 25 µg/mL. In the present application, the concentration of TIGIT mAb may be about 2.5-20 µg/mL. For example, it can be at least about 5-20 µg/mL. For example, it can be at least about 2.5 µg/mL, at least about 5 µg/mL or at least about 20 µg/mL.

For example, the immune checkpoint antibody or antigen-binding fragment thereof may be a human CTLA-4 antibody or antigen-binding fragment thereof.

For example, the immune checkpoint antibody or antigen-binding fragment thereof can be a CTLA-4 antibody or antigen-binding fragment thereof, and the concentration of the CTLA-4 antibody or antigen-binding fragment thereof is about 1 to about 100 µg/mL. In the present application, the concentration of the CTLA-4 mAb may be about 3-50 µg/mL, about 5-50 µg/mL or about 10-50 µg/mL. For example, it can be at least about 3 µg/mL, at least about 5 µg/mL, at least about 10 µg/mL or at least about 50 µg/mL.

In the present application, the seed cell culture medium may also include a costimulatory receptor antibody or an antigen-binding fragment thereof, wherein the costimulatory receptor immune checkpoint antibody or an antigen-binding fragment thereof may include: a CD40 antibody or an antigen-binding fragment thereof, an OX-40 antibody or an antigen-binding fragment thereof, a CD137 antibody or an antigen-binding fragment thereof, and/or a CD28 antibody or an antigen-binding fragment thereof.

For example, the co-stimulatory antibody or antigen-binding fragment thereof can include a CD137 antibody or antigen-binding fragment thereof.

For example, the CD137 antibody or antigen-binding fragment thereof can be a human CD137 antibody or antigen-binding fragment thereof.

For example, the concentration of the CD137 antibody or antigen-binding fragment thereof may be about 1 to about 100 µg/mL (e.g., about 2 to about 100 µg/mL, about 3 to about 100 µg/mL, about 3 to about 20 µg/mL mL, about 4 to about 100 µg/mL, about 5 to about 100 µg/mL, about 6 to about 100 µg/mL, about 7 to about 100 µg/mL, about 8 to about 100 µg/mL, about 9 to about 100 µg/mL, about 10 to about 100 µg/mL, about 12 to about 100 µg/mL, about 20 to about 100 µg/mL, about 10 to about 90 µg/mL, about 10 to about 80 µg/mL, about 10 to about 70 µg/mL, about 10 to about 50 µg/mL, about 10 to about 20 µg/mL or about 3 to about 12 µg/mL). In the present application, the concentration of CD137 mAb may be about 3-20 µg/mL. For example, it can be at least about 3 µg/mL, at least about 5 µg/mL, at least about 10 µg/mL, at least about 12 µg/mL or at least about 20 µg/mL.

For example, the co-stimulatory antibody or antigen-binding fragment thereof can include a OX40 antibody or antigen-binding fragment thereof.

For example, the OX-40 antibody or antigen-binding fragment thereof can be a human OX-40 antibody or antigen-binding fragment thereof.

For example, the concentration of OX-40 antibody or antigen-binding fragment thereof may be about 3 to about 10 µg/mL. In the present application, the concentration of OX-40 mAb may be about 5-10 µg/mL. For example, it can be at least about 3 µg/mL, at least about 5 µg/mL or at least about 10 µg/mL.

For example, the co-stimulatory antibody or antigen-binding fragment thereof can include a CD28 antibody or antigen-binding fragment thereof.

For example, the immune checkpoint antibody or antigen-binding fragment thereof can include a CD28 antibody or antigen-binding fragment thereof.

For example, the CD28 antibody or antigen-binding fragment thereof can be a human CD28 antibody or antigen-binding fragment thereof.

For example, the concentration of CD28 antibody or antigen-binding fragment thereof may be about 1-about 10 µg/mL. (For example, about 2 to about 10 µg/mL, about 3 to about 10 µg/mL, about 3 to about 5 µg/mL, about 4 to about 10 µg/mL, about 5 to about 10 µg/mL, about 6 to about 10 µg /mL, about 7 to about 10 µg/mL, or about 8 to about 10 µg/mL). In the present application, the concentration of CD28 mAb may be about 3-10 µg/mL. For example, it can be at least about 3 µg/mL, at least about 5 µg/mL or at least about 10 µg/mL.

For example, the co-stimulatory antibody or antigen-binding fragment thereof can include a CD40 antibody or antigen-binding fragment thereof.

For example, the CD40 antibody or antigen-binding fragment thereof can be a human CD40 antibody or antigen-binding fragment thereof.

For example, the concentration of CD40 antibody or antigen-binding fragment thereof may be about 5 to about 10 µg/mL. In the present application, the concentration of CD40 mAb may be about 6-10 µg/mL or about 5-8 µg/mL. For example, it can be at least about 5 µg/mL.

For example, the serum medium can include serum.

For example, the serum can include human AB serum.

For example, the concentration of the serum can be about 1 to about 10% (v/v) (e.g., about 1.5 to about 10%, about 2 to about 10%, about 3 to about 10%, about 4 to about 10%, about 5 to about 10%, about 1 to about 9%, about 1 to about 8%, about 1 to about 7%, or about 1 to about 6%).

For example, the serum medium can include AIM-V medium.

For example, the serum-free medium can include X-VIVO medium or CTS^{™} OpTmizer^{™} medium.

For example, the cell culture components can include antibiotics.

For example, the cell culture components may include penicillin-streptomycin mixture PS.

For example, the concentration of the penicillin-streptomycin mixture PS can be about 1 to about 200 U/mL (for example, it can be about 1 to about 5 U/mL, about 1 to about 10 U/mL, about 1 to about 20 U/mL mL, about 1 to about 30 U/mL, about 1 to about 40 U/mL, about 1 to about 50 U/mL, about 1 to about 60 U/mL, about 1 to about 70 U/mL, about 1 to about 80 U/mL, about 1 to about 90 U/mL, about 1 to about 100 U/mL, about 1 to about 120 U/mL, about 1 to about 140 U/mL, about 1 to about 160 U/mL, about 1 to about 180 U/mL, or about 1 to about 200 U/mL). For example, the concentration of the penicillin-streptomycin mixture PS may be about 100 U/mL.

For example, the seed cell culture medium may also include an M2 macrophage inhibitor, and the M2 macrophage inhibitor may include RRx001 and/or CNI-1493.

For example, the concentration of the M2 macrophage inhibitor can be about 0.1 to about 100 µM (for example, about 0.5 to about 100 µM, about 1 to about 100 µM, about 2 to about 100 µM, about 2.5 to about 100 µM, about 3 to about 100 µM, about 2 to about 3 µM, about 5 to about 100 µM, about 10 to about 100 µM, about 20 to about 100 µM, about 0.1 to about 90 µM, about 0.5 to about 90 µM, about 1 to about 90 µM, about 5 to about 90 µM, about 0.1 to about 80 µM).

For example, the seed cell culture medium may also include a regulatory T cell (Treg) inhibitors, and the regulatory T cell inhibitors may include CAL-101, dasatinib, imatinib and/or Panobinostat.

For example, the concentration of regulatory T cell inhibitor can be about 0.1 to about 100 µM (e.g., about 0.5 to about 100 µM, about 1 to about 100 µM, about 5 to about 100 µM, about 10 to about 100 µM, about 20 to about 100 µM, about 0.1 to about 90 µM, about 0.5 to about 90 µM, about 1 to about 90 µM, about 5 to about 90 µM, about 0.1 to about 80 µM). For example, the concentration of regulatory T cell inhibitor can be about 1 to about 5 µM, about 1 to about 3 µM, about 1.5 to about 5 µM, about 2 to about 5 µM, about 2.5 to about 5 µM or about 3 to about 5 µM.

For example, the seed cell culture medium may also include a myeloid-derived suppressor cell inhibitor, which may include AG490, Decitabine, Sunitinib and/or BBI608.

For example, the concentration of myeloid-derived suppressor cell inhibitor can be about 0.1 to about 100 µg/mL (e.g., about 0.5 to about 100 µg/mL, about 1 to about 100 µg/mL, about 2 to about 100 µg/mL mL, about 5 to about 100 µg/mL, about 6 to about 100 µg/mL, about 7 to about 100 µg/mL, about 8 to about 100 µg/mL, about 9 to about 100 µg/mL, about 10 to about 100 µg/mL, about 20 to about 100 µg/mL, about 10 to about 90 µg/mL, about 10 to about 80 µg/mL, about 10 to about 70 µg/mL, or about 10 to about 50 µg/mL). For example, the concentration of the myeloid-derived suppressor cell inhibitor can be about 1 to about 3 µg/mL, or can be about 1.5 to about 3 µg/mL.

In the present application, the seed cell culture medium may include at least one, at least two and at least three selected from the group consisting of the regulatory T cell (Treg) inhibitor, the myeloid-derived suppressor cell inhibitor and the M2 macrophage inhibitor.

In the present application, the seed cell culture medium may also include a T cell activator, and the T cell activator includes LYC-55716, GNE-1858 and/or methylene blue.

For example, the concentration of the T cell activator can be about 1 to about 10 µM. For example, can be about 1 to about 9 µM, about 1 to about 8 µM, about 1 to about 7 µM, about 1 to about 6 µM, about 2 to about 10 µM, about 3 to about 10 µM, about 4 to about 10 µM, or about 5 to about 10 µM.

In the present application, the seed cell culture medium may also include a T cell differentiation inhibitor, and the T cell differentiation inhibitor may include TWS119.

For example, the concentration of the T cell activator can be about 1 to about 10 µM. For example, can be about 1 to about 9 µM, about 1 to about 8 µM, about 1 to about 7 µM, about 1 to about 6 µM, about 2 to about 10 µM, about 3 to about 10 µM, about 4 to about 10 µM, or about 5 to about 10 µM.

In the present application, the concentration of RRX-001 may be about 0.1-100 µM. For example, it can be at least about 3 µM.

In the present application, the concentration of Sunitinib may be about 1.5-3 µM. For example, it may be at least about 1.5 µM or at least about 3 µM.

In the present application, the concentration of CNI-1493 may be about 2-3 µM. For example, it may be at least about 2 µM, at least 2.5 µM or at least about 3 µM.

In the present application, the concentration of Imatinib may be about 2-5 µM. For example, it may be at least about 2 µM or at least about 5 µM.

In the present application, the concentration of CAL-101 may be about 1.5-5 µM. For example, it may be at least about 1.5 µM or at least about 5 µM.

In the present application, the concentration of dasatinib may be about 2-3 µM. For example, it may be at least about 2 µM, at least 2.5 µM or at least about 3 µM.

In the present application, the concentration of LYC-55716 may be about 1-10 µM. For example, it may be at least about 1 µM or at least about 10 µM.

In the present application, the concentration of GNE-1858 may be about 4-5 nM. For example, it may be at least about 4 nM, at least 4.5 nM or at least about 5 nM.

In the present application, the concentration of methylene blue may be about 1 to about 2 µM. For example, it may be at least about 1 µM, at least 1.5 µM or at least about 2 µM.

In the present application, the concentration of TWS119 may be about 1-10 µM. For example, it may be at least about 1 µM, at least 5 µM or at least about 10 µM.

In this application, the seed cell culture medium may include IL-2, IL-7, PD-1 antibody or its antigen-binding fragment thereof, TNF alpha, human serum, serum-free medium X-VIVO 15 and penicillin-streptomycin mixture PS dual antibiotics.

For example, the seed cell culture medium may include at least about 3000 U/mL of IL-2, at least about 1000 U/mL of IL-7, at least about 100 µg/mL of PD-1 antibody or antigen-binding fragment thereof, at least about 10 pg/mL of TNF alpha, at least about 5% human serum, serum-free medium X-VIVO 15 and 100U/mL of penicillin-streptomycin mixture PS dual antibiotics.

In this application, the seed cell culture medium may include IL-2, IL-9, IL-15, PD-1 antibody or its antigen-binding fragment thereof, CTLA-4 antibody or its antigen-binding fragment thereof, human serum, serum-free medium X-VIVO 15 and penicillin-streptomycin mixture PS dual antibiotics.

For example, the seed cell culture medium may include at least about 2000 U/mL of IL-2, at least about 500 U/mL of IL-9, at least about 500 U/mL of IL-15, at least about 50 µg/mL of CTLA-4 antibody or its antigen-binding fragment thereof, at least about 50 µg/mL of PD-1 antibody or its antigen-binding fragment thereof, at least about 3% human serum, serum-free medium AIM-V and 100 U/mL of penicillin-streptomycin mixture PS dual antibiotics.

In this application, the seed cell culture medium may include IL-2, IL-7, G-CSF, PD-1 antibody or its antigen-binding fragment thereof, TNF alpha, human serum, serum-free medium X-VIVO 15 and penicillin-streptomycin mixture PS dual antibiotics.

For example, the seed cell culture medium may include at least about 2000 U/mL of IL-2, at least about 500 U/mL of IL-7, at least about 1000 U/mL of G-CSF, at least about 36 µg/mL of PD-1 antibody or its antigen-binding fragment thereof, at least about 20 pg/mL of TNF alpha, at least about 3% human serum, serum-free medium AIM-V and 100 U/mL of penicillin-streptomycin mixture PS dual antibiotics.

In this application, the seed cell culture medium may include IL-2, IL-12, IL-21, GM-CSF, LAG3 antibody or its antigen-binding fragment thereof, CTLA-4 antibody or its antigen-binding fragment thereof, human serum, serum-free medium X-VIVO 15 and penicillin-streptomycin mixture PS dual antibiotics.

For example, the seed cell culture medium may include at least about 3000 U/mL of IL-2, at least about 200 U/mL of IL-12, at least about 500 U/mL of IL-21, at least about 800 U/mL of GM-CSF, at least about 10 µg/mL of LAG-3 antibody or its antigen-binding fragment thereof, at least about 10 µg/mL of CTLA-4 antibody or its antigen-binding fragment thereof, at least about 5% human serum, serum-free medium CTS^{™}OpTmizer^{™} and 100U/mL of penicillin-streptomycin mixture PS dual antibiotics.

In this application, the seed cell culture medium may include IL-1 beta, IL-2, IL-6, IL-21, IFN-gamma, TIGIT antibody or its antigen-binding fragment thereof, PD-1 antibody or its antigen-binding fragment thereof, TNF alpha, human serum, serum-free medium X-VIVO 15 and penicillin-streptomycin mixture PS dual antibiotics.

For example, the seed cell culture medium may include at least about 500 U/mL of IL-1 beta, at least about 2500 U/mL of IL-12, at least about 200 U/mL of IL-6, at least about 200 U/mL of IL-21, at least about 1000 µg/mL of IFN-gamma, at least about 20 µg/mL of TIGIT antibody or its antigen-binding fragmentat, at least about 50 µg/mL of PD-1 antibody or its antigen-binding fragment thereof, at least about 25 pg/mL of TNF alpha, at least about 5% human serum, serum-free medium X-VIVO 15 and 100 U/mL of penicillin-streptomycin mixture PS dual antibiotics.

In this application, the seed cell culture medium may include IL-1 alpha, IL-2, IL-9, IL-18, IFN-alpha, IFN-beta, LAG-3 antibody or its antigen-binding fragment thereof, PD-1 antibody or its antigen-binding fragment thereof, TNF alpha, human serum, serum-free medium X-VIVO 15 and penicillin-streptomycin mixture PS dual antibiotics.

For example, the seed cell culture medium may include at least about 500 U/mL of IL-1 alpha, at least about 2000 U/mL of IL-2, at least about 500 U/mL of IL-9, at least about 200 U/mL of IL-18, at least about 500 U/mL of IFN-alpha, at least about 500 U/mL of IFN-beta, at least about 5 µg/mL of LAG-3 antibody or its antigen-binding fragment thereof, at least about 25 µg/mL of PD-1 antibody or its antigen-binding fragment thereof, at least about 25 pg/mL of TNF alpha, at least about 5% human serum, serum-free medium X-VIVO 15 and 100 U/mL of penicillin-streptomycin mixture PS dual antibiotics.

In this application, the seed cell culture medium may include IL-2, IL-4, IL-10, IL-21, CD137 antibody or its antigen-binding fragment thereof, LAG-3 antibody or its antigen-binding fragment thereof, PD-1 antibody or its antigen-binding fragment thereof, human serum, TNF alpha, human serum, serum-free medium X-VIVO 15 and penicillin-streptomycin mixture PS dual antibiotics.

For example, the seed cell culture medium may include at least about 3000 U/mL of IL-2, at least about 200 U/mL of IL-4, at least about 200 U/mL of IL-10, at least about 250 U/mL of IL-21, at least about 10 µg/mL of CD137 antibody or its antigen-binding fragment thereof, at least about 3 µg/mL of LAG-3 antibody or its antigen-binding fragment thereof, at least about 20 µg/mL of PD-1 antibody or its antigen-binding fragment thereof, at least about 10 pg/mL of TNF alpha, at least about 5% human serum, serum-free medium X-VIVO 15 and 1 U/mL of penicillin-streptomycin mixture PS dual antibiotics.

In this application, the seed cell culture medium may include IL-2, IL-6, IL-12, IL-21, CD137 antibody or its antigen-binding fragment thereof, CD28 antibody or its antigen-binding fragment thereof, PD-1 antibody or its antigen-binding fragment thereof, human serum, TNF alpha, human serum, serum-free medium X-VIVO 15 and penicillin-streptomycin mixture PS dual antibiotics.

For example, the seed cell culture medium may include at least about 3000 U/mL of IL-2, at least about 500 U/mL of IL-6, at least about 500 U/mL of IL-12, at least about 200 U/mL of IL-21, at least about 20 µg/mL of CD137 antibody or its antigen-binding fragment thereof, at least about 10 µg/mL of CD28 antibody or its antigen-binding fragment thereof, at least about 10 µg/mL of PD-1 antibody or its antigen-binding fragment thereof, at least about 10 pg/mL of TNF alpha, at least about 3% human serum, serum-free medium CTS^{™}OpTmizer^{™} and 100 U/mL of penicillin-streptomycin mixture PS dual antibiotics.

In this application, the seed cell culture medium may include IL-1 beta, IL-2, IL-7, IL-21, G-CSF, GM-CSF, IFN-gamma, LAG-3 antibody or its antigen-binding fragment thereof, PD-1 antibody or its antigen-binding fragment thereof, TNF alpha, human serum, serum-free medium X-VIVO 15 and penicillin-streptomycin mixture PS dual antibiotics.

For example, the seed cell culture medium may include at least about 200 U/mL of IL-1 beta, at least about 3000 U/mL of IL-2, at least about 200 U/mL of IL-7, at least about 200 U/mL of IL-21, at least about 500 U/mL of G-CSF, at least about 500 U/mL of GM-CSF, at least about 1000 U/mL of IFN-gamma, at least about 5 µg/mL of LAG-3 antibody or its antigen-binding fragment thereof, at least about 3 µg/mL of PD-1 antibody or its antigen-binding fragment thereof, at least about 15 pg/mL of TNF alpha, at least about 5% human serum, serum-free medium CTS^{™}OpTmizer^{™} and 100 U/mL of penicillin-streptomycin mixture PS dual antibiotics.

In this application, the seed cell culture medium may include IL-2, IL-4, IL-12, GM-CSF, M-CSF, IFN-beta, IFN-gamma, TIGIT antibody or its antigen-binding fragment thereof, CTLA-4 antibody or its antigen-binding fragment thereof, human serum, serum-free medium X-VIVO 15 and penicillin-streptomycin mixture PS dual antibiotics.

For example, the seed cell culture medium may include at least about 2500 U/mL of IL-2, at least about 500 U/mL of IL-4, at least about 500 U/mL of IL-12, at least about 500 U/mL of GM-CSF, at least about 300 U/mL of M-CSF, at least about 200 U/mL of IFN-beta, at least about 800 U/mL of IFN-gamma, at least about 5 µg/mL of TIGIT antibody or its antigen-binding fragment thereof, at least about 10 µg/mL of CTLA-4 antibody or its antigen-binding fragment thereof, at least about 5% human serum, serum-free medium X-VIVO 15 and 100 U/mL of penicillin-streptomycin mixture PS dual antibiotics.

In this application, the seed cell culture medium may include IL-2, IL-7, IL-15, IL-2, IL-7, IL-15, GM-CSF, CD137 antibody or its antigen-binding fragment thereof, PD-1 antibody or its antigen-binding fragment thereof, TNF alpha, human serum, serum-free medium X-VIVO 15 and penicillin-streptomycin mixture PS dual antibiotics.

For example, the seed cell culture medium may include at least about 3000 U/mL of IL-2, at least about 200 U/mL of IL-7, at least about 200 U/mL of IL-15, at least about 800 U/mL of GM-CSF, at least about 10 µg/mL of CD137 antibody or its antigen-binding fragment thereof, at least about 20 µg/mL of PD-1 antibody or its antigen-binding fragment thereof, at least about 20 pg/mL of TNF alpha, at least about 5% human serum, serum-free medium X-VIVO 15 and 100 U/mL of penicillin-streptomycin mixture PS dual antibiotics.

In this application, the seed cell culture medium may include IL-2, IL-4, IL-10, IL-10, G-CSF, M-CSF, CD137 antibody or its antigen-binding fragment thereof, CD28 antibody or its antigen-binding fragment thereof, OX-40 antibody or its antigen-binding fragment thereof, human serum, PD-1 antibody or its antigen-binding fragment, human serum, serum-free medium X-VIVO 15 and penicillin-streptomycin mixture PS dual antibiotics.

For example, the seed cell culture medium may include at least about 2500 U/mL of IL-2, at least about 500 U/mL of IL-4, at least about 500 U/mL of IL-10, at least about 250 U/mL of IL-10, at least about 500 U/mL of G-CSF, at least about 300 U/mL of M-CSF, at least about 5 µg/mL of CD137 antibody or its antigen-binding fragment thereof, at least about 3 µg/mL of CD28 antibody or its antigen-binding fragment thereof, at least about 10 µg/mL of OX-40 antibody or its antigen-binding fragment thereof, at least about 10 µg/mL of PD-1 antibody or its antigen-binding fragment thereof, at least about 5% human serum, serum-free medium AIM-V and 100 U/mL of penicillin-streptomycin mixture PS dual antibiotics.

In this application, the seed cell culture medium may include IL-2, IL-7, IL-21, IFN-alpha, IFN-gamma, CD137 antibody or its antigen-binding fragment thereof, CD28 antibody or its antigen-binding fragment thereof, OX-40 antibody or its antigen-binding fragment thereof, LAG-3 antibody or its antigen-binding fragment thereof, CTLA-4 antibody or its antigen-binding fragment thereof, TNF alpha, human serum, serum-free medium X-VIVO 15 and penicillin-streptomycin mixture PS dual antibiotics.

For example, the seed cell culture medium may include at least about 3000 U/mL of IL-2, at least about 200 U/mL of IL-7, at least about 200 U/mL of IL-15, at least about of 800 U/mL of GM-CSF, at least about 10 µg /mL of CD137 antibody or antigen-binding fragment thereof, at least about 20 µg/mL of PD-1 antibody or antigen-binding fragment thereof, at least about 20 pg/mL of TNF alpha, at least about 5% human serum, serum-free medium X-VIVO 15 and 100 U/ mL of penicillin-streptomycin mixture PS dual antibiotics.

In this application, the seed cell culture medium may include IL-2, IL-7, IL-15, IFN-beta, IFN-gamma, CD137 antibody or its antigen-binding fragment thereof, CD40 antibody or its antigen-binding fragment thereof, OX-40 antibody or its antigen-binding fragment thereof, TIGIT antibody or its antigen-binding fragment thereof, PD-1 antibody or its antigen-binding fragment thereof, human serum, serum-free medium X-VIVO 15 and penicillin-streptomycin mixture PS dual antibiotics.

For example, the seed cell culture medium may include at least about 3000 U/mL of IL-2, at least about 500 U/mL of IL-7, at least about 500 U/mL of IL-15, at least about 250 U/mL of IFN-beta, at least about 1000 U/mL of IFN-gamma, at least about 5 µg /mL of CD137 antibody or antigen-binding fragment thereof, at least about 5 µg/mL of CD40 antibody or antigen-binding fragment thereof, at least about 5 µg/mL of OX-40 antibody or antigen-binding fragment thereof, at least about 5 µg/mL of TIGIT antibody or antigen-binding fragment thereof, at least about 15 µg/mL of PD-1 antibody or antigen-binding fragment thereof, at least about 5% human serum, serum-free medium X-VIVO 15 and 100 U/mL of penicillin-streptomycin mixture PS dual antibiotics.

In this application, the seed cell culture medium may include IL-2, IL-7, IL-15, GM-CSF, IFN-gamma, CD137 antibody or its antigen-binding fragment thereof, CD28 antibody or its antigen-binding fragment thereof, PD-1 antibody or its antigen-binding fragment thereof, TNF alpha, at least about 5% human serum, human serum, serum-free medium CTS^{™}OpTmizer^{™} and penicillin-streptomycin mixture PS dual antibiotics.

For example, the seed cell culture medium may include at least about 3000 U/mL of IL-2, at least about 200 U/mL of IL-7, at least about 200 U/mL of IL-15, at least about 500 U/mL of GM-CSF, at least about 1000 U/mL of IFN-gamma, at least about 3 µg/mL CD137 antibody or its antigen-binding fragment thereof, at least about 3 µg/mL of CD28 antibody or its antigen-binding fragment thereof, at least about 10 pg/mL of TNF alpha, at least about 5% human serum, serum-free medium CTS^{™}OpTmizer^{™} and 100 U/mL of penicillin-streptomycin mixture PS dual antibiotics.

In this application, the seed cell culture medium may include IL-2, IL-7, IL-12, G-CSF, GM-CSF, IFN-alpha, IFN-gamma, CD28 antibody or its antigen-binding fragment thereof, CD40 antibody or its antigen-binding fragment thereof, TIGIT antibody or its antigen-binding fragment thereof, PD-1 antibody or its antigen-binding fragment thereof, TNF alpha, human serum, serum-free medium X-VIVO 15 and penicillin-streptomycin mixture PS dual antibiotics.

For example, the seed cell culture medium may include at least about 3000 U/mL of IL-2, at least about 200 U/mL of IL-7, at least about 300 U/mL of IL-12, at least about 300 U/mL of G-CSF, at least about 200 U/mL of GM-CSF, at least about 500 U/mL of IFN-alpha, at least about 500 U/mL of IFN-gamma, at least about 3 µg/mL of CD28 antibody or its antigen-binding fragment thereof, at least about 5 µg/mL of CD40 antibody or its antigen-binding fragment thereof, at least about 2.5 µg/mL of TIGIT antibody or its antigen-binding fragment thereof, at least about 3 µg/mL of PD-1 antibody or its antigen-binding fragment thereof, at least about 10 pg/mL of TNF alpha, at least about 5% human serum, serum-free medium X-VIVO 15 and 100 U/mL of penicillin-streptomycin mixture PS dual antibiotics.

In this application, the seed cell culture medium may include IL-2, IL-7, PD-1 antibody or its antigen-binding fragment thereof, RRx-001, CAL-101, human serum, serum-free medium CTS^{™}OpTmizer^{™} and penicillin-streptomycin mixture PS dual antibiotics.

For example, the seed cell culture medium may include at least about 3000 U/mL of IL-2, at least about 1000 U/mL of IL-7, at least about 50 µg/mL of PD-1 antibody or its antigen-binding fragment thereof, at least about 3 µM RRx-001, at least about 1.5 µM CAL-101, at least about 5% human serum, serum-free medium CTS^{™}OpTmizer^{™} and 100U/mL of penicillin-streptomycin mixture PS dual antibiotics.

In this application, the seed cell culture medium may include IL-2, IL-10, IL-18, LAG-3 antibody or its antigen-binding fragment thereof, PD-1 antibody or its antigen-binding fragment thereof, Sunitinib, Imatinib, human serum, serum-free medium X-VIVO 15 and penicillin-streptomycin mixture PS dual antibiotics.

For example, the seed cell culture medium may include at least about 2500 U/mL of IL-2, at least about 200 U/mL of IL-18, at least about 10 µg/mL of LAG-3 antibody or its antigen-binding fragment thereof, at least about 5 µg/mL of PD-1 antibody or its antigen-binding fragment thereof, at least about 3 µM of Sunitinib, at least about 5 µM of Imatinib, at least about 5% human serum, serum-free medium X-VIVO 15 and 100 U/mL of penicillin-streptomycin mixture PS dual antibiotics.

In this application, the seed cell culture medium may include IL-2, IL-1 beta, IL-6, GM-CSF, M-CSF, CNI-1493, human serum, serum-free medium X-VIVO 15 and penicillin-streptomycin mixture PS dual antibiotics.

For example, the seed cell culture medium can include at least about 3000 U/mL of IL-2, at least about 200 U/mL of IL-1 beta, at least about 500 U/mL of IL-6, at least about 500 U/mL of GM-CSF, at least about 500 U /mL of M-CSF, at least about 5 µM of CNI-1493, at least about 5% human serum, serum-free medium X-VIVO 15 and 100 U/mL penicillin-streptomycin mixture PS dual antibiotics.

In this application, the seed cell culture medium may include IL-2, IL-9, IL-10, IL-21, GM-CSF, OX40 antibody or its antigen-binding fragment thereof, PD-1 antibody or its antigen-binding fragment thereof, Sunitinib, human serum, serum-free medium X-VIVO 15 and penicillin-streptomycin mixture PS dual antibiotics.

For example, the seed cell culture medium may include at least about 2000 U/mL of IL-2, at least about 200 U/mL of IL-9, at least about 200 U/mL of IL-10, at least about 500 U/mL of IL-21, at least about 500 U/mL of GM-CSF, at least about 5 µg/mL of OX40 antibody or its antigen-binding fragment thereof, at least about 36 µg/mL of PD-1 antibody or its antigen-binding fragment thereof, at least about 1.5 µM of Sunitinib, at least about 5% human serum, serum-free medium X-VIVO 15 and 100 U/mL of penicillin-streptomycin mixture PS dual antibiotics.

In this application, the seed cell culture medium may include IL-2, IL-6, IL-12, CD28 antibody or its antigen-binding fragment thereof, OX-40 antibody or its antigen-binding fragment thereof, CTLA-4 antibody or its antigen-binding fragment thereof, TNF alpha, Imatinib, human serum, serum-free medium CTS^{™}OpTmizer^{™} and penicillin-streptomycin mixture PS dual antibiotics.

For example, the seed cell culture medium may include at least about 2500 U/mL of IL-2, at least about 500 U/mL of IL-6, at least about 1000 U/mL of IL-12, at least about 10 µg/mL of CD28 antibody or its antigen-binding fragment thereof, at least about 3 µg/mL of OX-40 antibody or its antigen-binding fragment thereof, at least about 5 µg/mL of CTLA-4 antibody or its antigen-binding fragment thereof, at least about 15 pg/mL of TNF alpha, at least about 5 µM of Imatinib, at least about 5% human serum, serum-free medium CTS^{™}OpTmizer^{™} and 100 U/mL of penicillin-streptomycin mixture PS dual antibiotics.

In this application, the seed cell culture medium may include IL-2, IL-7, IL-15, CD137 antibody or its antigen-binding fragment thereof, CD28 antibody or its antigen-binding fragment thereof, LAG-3 antibody or its antigen-binding fragment thereof, PD-1 antibody or its antigen-binding fragment thereof, dasatinib, human serum, serum-free medium X-VIVO 15 and penicillin-streptomycin mixture PS dual antibiotics.

For example, the seed cell culture medium may include at least about 3000 U/mL of IL-2, at least about 200 U/mL of IL-7, at least about 300 U/mL of IL-15, at least about 12 µg/mL of CD137 antibody or its antigen-binding fragment thereof, at least about 5 µg/mL of CD28 antibody or its antigen-binding fragment thereof, at least about 5 µg/mL of LAG-3 antibody or its antigen-binding fragment thereof, at least about 3 µg/mL of PD-1 antibody or its antigen-binding fragment thereof, at least about 3 µM of dasatinib, at least about 5% human serum, serum-free medium X-VIVO 15 and 100 U/mL of penicillin-streptomycin mixture PS dual antibiotics.

In this application, the seed cell culture medium may include IL-2, IL-6, IL-12, G-CSF, M-CSF, IFN-beta, IFN-gamma, CTLA-4 antibody or its antigen-binding fragment thereof, PD-1 antibody or its antigen-binding fragment thereof, dasatinib, LYC-55716, GNE-1858, human serum, serum-free medium X-VIVO 15 and penicillin-streptomycin mixture PS dual antibiotics.

For example, the seed cell culture medium may include at least about 3000 U/mL of IL-2, at least about 500 U/mL of IL-6, at least about 500 U/mL of IL-12, at least about 1000 U/mL of G-CSF, at least about 500 U/mL of M-CSF, at least about 500 U/mL of IFN-beta, at least about 800 U/mL of IFN-gamma, at least about 3 µg/mL of CTLA-4 antibody or its antigen-binding fragment thereof, at least about 5 µg/mL of PD-1 antibody or its antigen-binding fragment thereof, at least about 2.5 µM dasatinib, at least about 10 µM LYC-55716, at least about 4.5 µM GNE-1858, at least about 5% human serum, serum-free medium X-VIVO 15 and 100 U/mL of penicillin-streptomycin mixture PS dual antibiotics.

In this application, the seed cell culture medium may include IL-2, IL-1 alpha, IL-9, GM-CSF, CD137 antibody or its antigen-binding fragment thereof, CD28 antibody or its antigen-binding fragment thereof, LAG-3 antibody or its antigen-binding fragment thereof, TIGIT antibody or its antigen-binding fragment thereof, CNI-1493, methylene blue, TWS119, human serum, serum-free medium AIM-V and penicillin-streptomycin mixture PS dual antibiotics.

For example, the seed cell culture medium may include at least about 2500 U/mL of IL-2, at least about 500 U/mL of IL-1 alpha, at least about 500 U/mL of IL-9, at least about 800 U/mL of GM-CSF, at least about 3 µg/mL CD137 antibody or its antigen-binding fragment thereof, at least about 3 µg/mL of CD28 antibody or its antigen-binding fragment thereof, at least about 5 µg/mL of LAG-3 antibody or its antigen-binding fragment thereof, at least about 2.5 µg/mL of TIGIT antibody or its antigen-binding fragment thereof, at least about 2.5 µM CNI-1493, at least about 1 µM methylene blue, at least about 5 µM TWS119, at least about 5% human serum, serum-free medium AIM-V and 100 U/mL of penicillin-streptomycin mixture PS dual antibiotics.

In this application, the seed cell culture medium may include IL-2, IL-1 alpha, IL-12, IL-21, G-CSF, M-CSF, IFN-alpha, IFN-beta, IFN-gamma, CD28 antibody or its antigen-binding fragment thereof, CD40 antibody or its antigen-binding fragment thereof, LAG-3 antibody or its antigen-binding fragment thereof, PD-1 antibody or its antigen-binding fragment thereof, CNI-1493, dasatinib, GNE-1858, methylene blue, human serum, serum-free medium AIM-V and penicillin-streptomycin mixture PS dual antibiotics.

For example, the seed cell culture medium may include about 3000 U/mL of IL-2, about 200 U/mL of IL-1 alpha, about 500 U/mL of IL-12, about 500 U/mL of IL-21, about 300 U/mL of G-CSF , about 800 U/mL of M-CSF, about 500 U/mL of IFN-alpha, about 300 U/mL of IFN-beta, about 300 U/mL of IFN-gamma, about 3 µg/mL of CD28 antibody or its antigen-binding fragment thereof, about 5 µg/mL of CD40 antibody or antigen-binding fragment thereof, about 5 µg/mL of LAG-3 antibody or antigen-binding fragment thereof, about 3 µg/mL of PD-1 antibody or antigen-binding fragment thereof, about 3 µM of CNI-1493, about 2 µM of dasatinib, about 5 nM of GNE-1858 , about 1.5µM of methylene blue, about 5% human serum, serum-free medium AIM-V and 100 U/mL of penicillin-streptomycin mixture PS dual antibiotics.

In another aspect, the present application provides seed cells or the population of cell of tumor infiltrating lymphocytes obtained by culturing the seed cell medium described in the present application.

On the other hand, the present application provides a pharmaceutical composition, which comprises the seed cells or the population of cell of the tumor infiltrating lymphocytes described in the present application and a pharmaceutically acceptable carrier.

On the other hand, the present application provides the seed cells or the population of cell of the tumor infiltrating lymphocytes thereof and the application of the pharmaceutical composition described in the present application in the preparation of a medicament for treating cancer.

The seed cell culture medium described herein may be suitable for expanding tumor infiltrating lymphocytes derived from different types of cancer. The seed cell culture medium described in this application has broad spectrum and universal applicability to cancer types. In some cases, the seed cell culture medium may be suitable for culturing tumor-infiltrating lymphocytes derived from different types of cancers, and may be suitable for obtaining seed cells or the population of cell thereof derived from tumor-infiltrating lymphocytes of different types of cancers. In some cases, the seed cell culture medium described in this application and/or the pharmaceutical composition described in this application can be used to prepare a medicament for treating cancer. In the present application, the cancer may include: a cervical cancer (for example, may include a recurrent cervical cancer), a ovarian cancer, a pelvic poorly differentiated adenocarcinoma, a intestinal cancer (for example, may include intestinal cancer with metastases, such as liver metastases), a gastric cancer, melanoma, a breast cancer and/or a endometrial stromal sarcoma. For example, the cancer is selected from the group consisting of a melanoma, a glioma, a gastric cancer, a lung cancer, a gastrointestinal stromal tumor, a intestinal cancer, a liver cancer, a cervical cancer, an ovarian cancer, a breast cancer, a uterine Intimal stromal sarcomas, a pelvic poorly differentiated glandular and a cholangiocarcinomas.

In another aspect, the present application provides an use of the seed cell culture medium in expanding tumor infiltrating lymphocytes.

In another aspect, the present application provides a method for culturing seed cells of tumor-infiltrating lymphocytes, which includes the following steps: culturing isolated tumor-infiltrating lymphocytes in the seed cell culture medium described in the present application.

In another aspect, the present application provides a method for expanding tumor-infiltrating lymphocytes, which includes the following steps, culturing isolated tumor-infiltrating lymphocytes in the seed cell culture medium described in the present application, obtaining the seed cell of the tumor-infiltrating lymphocytes.

For example, the isolated tumor infiltrating lymphocytes may be derived from a sample selected from the group consisting of: ascites fluid from a subject in need thereof, surgically resected primary tumor samples, synchronous and metachronous surgical resections metastasis samples, puncture samples and body fluids.

For example, the body fluid may include blood, interstitial fluid, lymph and/or body cavity fluid. For example, the body fluid may include peritoneal effusion (for example, it may be the ascites of a patient with gastric cancer, or the ascites of a breast cancer patient) and/or pleural effusion (for example, it may be the pleural effusion of a patient with endometrial stromal sarcoma).

For example, the isolated tumor infiltrating lymphocytes may be derived from a tumor selected from the group consisting of: a melanoma, a glioma, a gastric cancer, a lung cancer, a gastrointestinal stromal tumor, a intestinal cancer, a liver cancer, a cervical cancer, an ovarian cancer, a breast cancer, a endometrial stromal sarcoma, a pelvic poorly differentiated adenocarcinoma and a cholangiocarcinoma, for example, the isolated tumor infiltrating lymphocytes may be derived from tumor tissue of a cancer selected from the group consisting of a cervical cancer (for example, may include a recurrent cervical cancer), an ovarian cancer, a poorly differentiated pelvic adenocarcinoma, a intestinal cancer (for example, may include a intestinal cancer with metastases, such as liver metastases), a gastric cancer, a melanoma, a breast cancer and/or a endometrial stromal sarcoma.

For example, the isolated tumor-infiltrating lymphocytes may be from a dissected tumor tissue, and the diameter of the dissected tumor tissue is about 1 mm to about 10 mm, for example, about 2 mm to about 10 mm, about 3mm to about 10mm, about 4mm to about 10mm, about 5mm to about 10mm, about 6mm to about 10mm, about 7mm to about 10mm or about 8mm to about 10mm.

For example, the culture time may be about 3-20 days. For example, the culture time can be about 3-15 days, for example, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days or about 20 days.

For example, the culture temperature may be about 30-42 °C. For example, it may be at least about 30°C, at least about 31°C, at least about 32°C, at least about 33°C, at least about 34°C, at least about 35°C, at least about 36°C, at least about 37°C, at least about 38°C, at least about 39°C, at least about 40°C, at least about 40°C or at least about 42°C.

For example, the CO₂ concentration of the culture is about 1%-10%, for example, can be at least about 1%, at least about 2%, at least about 3%, at least about 4%, at least about 5%, at least about 6%, at least about 7%, at least about 8%, at least about 9% or at least about 10%.

In the present application, using the seed cell culture medium described in the present application can significantly improve the cell activity of the seed cells of tumor infiltrating lymphocytes (for example, can significantly improve the total number of cells of the seed cells of tumor infiltrating lymphocytes, for example, can make the number of seed cells of tumor infiltrating lymphocytes reaches an order of magnitude of at least about 10⁸; for example, the cell viability of seed cells of tumor infiltrating lymphocytes can be significantly increased, for example, the cell viability of seed cells of tumor infiltrating lymphocytes can be increased to at least about 85%; for example, can significantly increase the secretion of cytokines (such as IFN-gamma) by seeded cells of tumor infiltrating lymphocytes). In the present application, using the seed cell culture medium described in the present application can significantly increase the proportion of CD3+ cells in the seed cells of tumor infiltrating lymphocytes.

In this application, using the seed cell culture medium described in this application can significantly improve the TCR clones frequency of the product TIL, that are partially infused after infusing the seed cells of tumor infiltrating lymphocytes into a donor subject (such as a tumor patient), the frequency of TCR in the peripheral blood of the donor subject was significantly increased, and the similarity between the TCR repertoire of the peripheral blood of the donor subject and the TCR repertoire of the TIL infusion product were also significantly increased, and can be maintained for a long time constant. In addition, after the autologous infusion of TILs cultured in the seed cell culture medium described in the present application, the lymphocytes in the donor subjects can produce relatively active proliferation.

The cytokines in the TIL seed cell culture medium may also have the following characteristics: the cytokines include IL-7; the concentration of the IL-7 is, for example, about 5 to about 100 ng/mL. The cytokines include IL-15; the concentration of the IL-15 is, for example, about 5 to about 100 ng/mL. The cytokines include tumor necrosis factor TNF, such as TNF alpha; the concentration of said TNF alpha is, for example, about 10 to about 100 pg/mL. The cytokines include colony-stimulating factors, such as GM-CSF; the concentration of the GM-CSF is, for example, about 200 to about 5000 U/mL. The cytokines include interferon, such as IFN-gamma; the concentration of the IFN-gamma is, for example, about 10 to about 1000 ng/mL. Said cytokines also include interleukins selected from the group consisting of: IL-4, IL-12, IL-21 and IL-10.

The immune checkpoint antibody of the TIL seed cell culture medium can also have the following features: the PD-1 antibody or its antigen-binding fragment thereof is a human PD-1 antibody or its antigen-binding fragment thereof; the concentration of PD-1 antibody or its antigen-binding fragments thereof is, for example, from about 10 to about 100 µg/mL. The immune checkpoint antibody or antigen-binding fragment thereof can include a CD137 antibody or antigen-binding fragment thereof, for example, can be a human CD137 antibody or antigen-binding fragment thereof; and the concentration of the CD137 antibody or antigen-binding fragment thereof is about 1 to about 100 µg/mL. The immune checkpoint antibody or antigen-binding fragment thereof can include a CD28 antibody or antigen-binding fragment thereof, for example, can be a human CD28 antibody or antigen-binding fragment thereof; and the concentration of the CD28 antibody or antigen-binding fragment thereof is about 1 to about 10 µg/mL.

The cell culture components of the TIL seed cell culture medium can also have the following features: the serum medium includes serum, such as human AB serum; the concentration of the serum is, for example, about 1 to about 10% (v/v). The serum medium can include AIM-V medium. The serum-free medium includes X-VIVO medium and MHM-C medium.

The cell culture components of the TIL seed cell culture medium can also have the following features: the serum-free medium includes platelets, such as platelets derived from the subject's own blood or platelets derived from allogeneic blood; the density of the platelets is, for example, about 0.5×10⁸/mL to about 3×10⁸/mL. The serum-free medium includes X-VIVO medium and MHM-C medium.

The TIL seed cell culture medium may also include M2 macrophage inhibitors, and the M2 macrophage inhibitors may include RRx001 and/or CNI-1493. The concentration of the inhibitor of M2 macrophages is about 0.1 to about 100 µM. The TIL seed cell culture medium may also include a regulatory T cell (Treg) inhibitors, and the regulatory T cell inhibitors may include CAL-101, dasatinib, Imatinib and/or Panobinostat. The concentration of the regulatory T cell inhibitor is about 0.1 to about 100 µM. The TIL seed cell culture medium may also include a myeloid-derived suppressor cell inhibitor, which may include AG490, decitabine, Sunitinib and/or BBI608. The concentration of the myeloid-derived suppressor cell inhibitor is about 0.1 to about 100 µg/mL.

The present invention also provides the use of the culture composition for expanding TILs described herein in the manufacture of a culture medium for expanding TILs or a medicament containing TILs. The present invention also provides the use of the culture medium for expanding TILs described herein in the manufacture of a medicament containing TILs. These medicaments are used to treat cancers associated with the tumor tissue from which TILs were obtained.

The invention also provides a method of expanding a population of TILs comprising incubating the population of TILs with a medium comprising a TIL cell culture composition as described herein. The incubation lasts for at least 5 days, at least 6 days, at least 7 days, at least 8 days, at least 9 days, at least 10 days, at least 11 days, at least 12 days, at least 13 days, at least 14 days, at least 15 days; and/or the incubation lasts for at most 20 days, at most 15 days, at most 14 days, at most 13 days, at most 12 days, at most 11 days, at most 10 days, at most 9 days, at most 8 days, at most 7 days, at most 6 days, at most 5 days. The incubation conditions are well known in the art, such as about 30-42°C, about 1%-10% CO₂.

The present invention also provides method I for preparing the population of TILs, comprising:
(1) the tumor tissue is incubated with TIL seed cell culture medium to obtain a first population of TIL cell,
(2) the first population of TILs is incubated with the medium described herein to obtain a second population of TILs.

The present invention also provides method II for preparing the population of TIL cell, comprising:
(1) incubating a tumor cell-containing tissue with a TIL seed cell culture medium to obtain a first population of TILs,
(2) contacting the first population of TILs described in (1) with any one or more of the antibodies coupled to the matrix in the above culture composition herein to obtain a second population of TILs,
(3) culturing the second population of TILs described in (2) in a medium containing any one or more of the components in the above culture composition herein except for the antibodies coupled to the matrix, to obtain the third population of TILs.

The second population of TILs in the method 1 and method 2 contains more than 10¹⁰-10¹¹ of TILs. Typically, the tumor tissue needs to be pretreated, including washing, fragmenting and/or dissociating the tumor tissue. The method of fragmenting and/or dissociating tumor tissue may be mechanical cutting or enzymatic digestion. The size of the fragmented tissue blocks formed by mechanical cutting of the tumor tissue can be any conventional size convenient for subsequent culture and further processing, optionally, 2×2×2 mm³, 3×3×3 mm³, 4 ×4×4 mm³, 5×5×5 mm³ or 6×6×6 mm³.

In order to induce TILs, the incubation of the step (1) of the method I and method II lasts for at least 5 days, at least 6 days, at least 7 days, at least 8 days, at least 9 days, at least 10 days, at least 11 days, at least 12 days days, at least 13 days, at least 14 days, at least 15 days; and/or, the incubation of step (1) lasts at most 20 days, at most 15 days, at most 14 days, at most 13 days, at most 12 days, at most 11 days, at most 10 days, at most 9 days, at most 8 days, at most 7 days, at most 6 days, at most 5 days. In order to expand a sufficient number of TILs, the incubation of the step (2) of the method I lasts for at least 5 days, at least 6 days, at least 7 days, at least 8 days, at least 9 days, at least 10 days, at least 11 days, at least 12 days days, at least 13 days, at least 14 days, at least 15 days; and/or, the incubation of step (2) of the method I lasts at most 20 days, at most 15 days, at most 14 days, at most 13 days, at most 12 days, at most 11 days, at most 10 days, at most 9 days, at most 8 days, at most 7 days, at most 6 days, at most 5 days. The incubation is performed in a condition that is well known in the art, such as about 30-42°C, about 1%-10% CO₂.

In the step (2) of the method II, the antibodies coupled to the matrix include any one or more selected from the group consisting of: a CD3 antibody, a CD28 antibody, a CD137 antibody, a CD40 antibody, a OX-40 antibody and a GITR antibody; preferably, include any one or more selected from the group consisting of a CD3 antibody, a CD28 antibody and a CD137 antibody. The matrix includes any one or more selected from the group consisting of multiwell plates, cell culture dishes, cell culture bags and magnetic beads. The coupling is a covalent coupling or a non-covalent coupling, which can immobilize the above-mentioned antibodies on the matrix. For example, the matrix can be a cell culture dish or a cell culture bag, and the coupling can be the coating by incubating the bottom or inner wall of the a cell culture dish or a cell culture bag with the stock solution containing the above-mentioned antibody, and after coating pretreatment, matrix-immobilized antibodies are formed which attach to the bottom of the culture vessel. Preferably, the above-mentioned antibodies are activating antibodies.

In the step (2) of the method II, the contacting is preferably incubating the first population of TILs in the second step (1) of the method with the antibodies coupled to the matrix, and the temperature of the incubation is 30-42°C, preferably 37°C. Preferably, the concentration of CO₂ of the incubation is 5%. The incubation is preferably carried out in a culture medium containing any one or more of the components in the aforementioned culture composition except for the antibodies coupled to the matrix. Optionally, the culture medium containing any one or more of the components in the aforementioned culture composition except for the antibodies coupled to the matrix comprises any one or more of the following component combinations: 1) IL-2, IL-7, IL-15, a PD-1 antibody; 2) IL-2, IL-7, IL-15, a PD-1 antibody, IL-21, IL-12 and GM-CSF; 3) IL-2, IL-7, IL-15, a PD-1 antibody, a GITR antibody and TWS119; 4) IL-2, IL-7, IL-15, a PD-1 antibody, a TIGIT antibody and GM-CSF; 5) IL-2, IL-7, IL-15, a LAG3 antibody, IL-21, IL-12 and a GITR antibody; 6) IL-2, IL-7, IL-15, a PD-1 antibody, a TIGIT antibody, a CD40 antibody , a OX-40 antibody and autologous platelet; 7) IL-2, IL-7, IL-15, a PD-1 antibody, a CTLA-4 antibody, a CD137 antibody, a GITR antibody, GM-CSF, TWS119 and allogeneic platelet. The contacting lasts for 1-3 days, eg for 1 day, for 2 days, for 3 days.

In step (2) of method II, the density of the first population of TILs is 1.0×10⁵/mL~ 1.0×10⁶/mL; preferably is 2.0×10⁵/mL~1.0 × 10⁶/mL; more preferably is 2.5 × 10⁵/mL~3.5×10⁶/mL. Optionally, 1.0×10⁵/mL, 2.0×10⁵/mL, 2.5×10⁵/mL, 3.0×10⁵/mL, 3.5×10⁵/mL, 4.0×10⁵/mL, 4.5×10⁵/mL, 5.0×10⁵/mL, 5.5×10⁵/mL, 6.0×10⁵/mL, 6.5×10⁵/mL, 7.0×10⁵/mL, 7.5×10⁵/mL, 8.0×10⁵/mL, 8.5×10⁵/mL, 9.0×10⁵/mL , 9.5×10⁵/mL or 10×10⁶/mL.

In the step (3) of the method II,the culturing lasts for at least 5 days, at least 6 days, at least 7 days, at least 8 days, at least 9 days, at least 10 days, at least 11 days, at least 12 days, at least 13 days, at least 14 days, at least 15 days; or, lasts at most 20 days, at most 15 days, at most 14 days, at most 13 days, at most 12 days, at most 11 days, at most 10 days, at most 9 days, at most 8 days, at most 7 days, at most 6 days, at most 5 days. The culture temperature is 30-42°C, preferably is 37°C. The CO₂ concentration of the culture is 5%.

In the step (1) of the method I and method II, the TIL seed cell culture medium can be any one of the aforementioned TIL seed cell culture media. Optionally, the TIL seed cell culture medium may include any one combination of the following combinations of components: 1) IL-2, IL-6, IL-21, IFN-gamma, a TIGIT antibody, a PD-1 antibody, TNF-alpha, serum, dual antibiotics and basal medium; 2) IL-2, IL-4, IL-10, IL-21, a CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, PD-1 antibody, serum, dual antibiotics and basal medium; 4) IL-1beta, IL -2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15 , GM-CSF, a CD137 antibody, a PD-1 antibody, TNF-alpha, serum, dual antibiotics and basal medium; 7) IL-2, IL-4, IL-10, IL-15, G-CSF, M- CSF, a CD28 antibody, a OX-40 antibody, a PD-1 antibody, serum, dual antibiotics and basal medium; 8) IL-2, IL-7, IL-15, IFN-gamma, a CD137 antibody, a CD40 antibody, a OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basal medium; 12) IL-2, IL-7, IL-15, GM-CSF, M-CSF, a PD-1 antibody, CNI-1493, serum, dual antibiotics and basal medium; 13) IL-2, IL-7, IL-15, a CD137 antibody, a CD28 antibody, a LAG3 antibody, a PD-1 antibody, dasatinib, serum, dual antibiotics and basal medium; 14) IL-2 , IL-6, IL-12, G-CSF, M-CSF, IFN-beta, IFN-gamma, a CTLA-4 antibody, a PD-1 antibody, dasatinib, LYC-55716, GENE-1858, serum, dual antibiotics and basal medium; 15) IL-1 alpha, IL-2, IL-9, IL-15, GM-CSF, a CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, dasatinib, GNE-1858, serum, dual antibiotics and basal medium. Preferably, the TIL seed cell culture medium may include any one combination of the following combinations of components: 1) IL-2, IL-6, IL-21, IFN-gamma, a TIGIT antibody, a PD-1 antibody, TNF-alpha, human AB serum, PS dual antibiotics and X-VIVO 15 medium; 2) IL-2, IL-4, IL-10, IL-21, a CD137 antibody, a LAG3 antibody, a PD-1 antibody, TNF-alpha, human AB serum, PS dual antibiotics and X-VIVO 15 medium; 3) IL-2, IL-7, IL-12, IL-21, a CD137 antibody, a CD28 antibody, a PD-1 antibody, human AB serum, PS dual antibiotics and CTS^{™}OpTmizer^{™} medium; 4) IL-1 beta, IL-2, IL-7, G-CSF, GM-CSF, IFN-gamma, a LAG3 antibody, a PD-1 antibody, TNF-alpha, human AB serum, PS dual antibiotics and CTS^{™}OpTmizer^{™} medium; 5) IL-2, IL-4, IL-12, GM-CSF, M-CSF, IFN-beta, IFN-gamma, a TIGIT antibody, a CTLA-4 antibody, human AB serum, PS dual antibiotics and X-VIVO 15 medium; 6) IL-2, IL-7, IL-15 , GM-CSF, a CD137 antibody, a PD-1 antibody, TNF-alpha, human AB serum, PS dual antibiotics and X-VIVO 15 medium; 7) IL-2, IL-4, IL-10, IL-15, G-CSF, M- CSF, a CD28 antibody, a OX-40 antibody, a PD-1 antibody, human AB serum, PS dual antibiotics and AIM-V medium; 8) IL-2, IL-7, IL-15, IFN-gamma, a CD137 antibody, a CD40 antibody, a OX-40 antibody, a TIGIT antibody, a PD-1 antibody, human AB serum, PS dual antibiotics and X-VIVO 15 medium; 9) IL-2, IL-7, IL-15, GM-CSF, IFN-gamma, a CD137 antibody, a CD28 antibody, a PD-1 Antibody, TNF-alpha, human AB serum, PS dual antibiotics and X-VIVO 15 medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, human AB serum, PS dual antibiotics and X-VIVO 15 medium; 11) IL-2, IL-7, IL-15, GM-CSF, a PD-1 antibody, RRx-001, CAL-101, human AB serum, PS dual antibiotics and CTS^{™}OpTmizer^{™} medium; 12) IL-2, IL-7, IL-15, GM-CSF, M-CSF, a PD-1 antibody, CNI-1493, human AB serum, PS dual antibiotics and X-VIVO 15 medium; 13) IL-2, IL-7, IL-15, a CD137 antibody, a CD28 antibody, a LAG3 antibody, a PD-1 antibody, dasatinib, human AB serum, PS dual antibiotics and X-VIVO 15 medium; 14) IL-2 , IL-6, IL-12, G-CSF, M-CSF, IFN-beta, IFN-gamma, a CTLA-4 antibody, a PD-1 antibody, dasatinib, LYC-55716, GENE-1858, human AB serum, PS dual antibiotics and X-VIVO 15 medium; 15) IL-1 alpha, IL-2, IL-9, IL-15, GM-CSF, a CD137 antibody, a CD28 antibody, a LAG3 antibody, a TIGIT antibody, CNI-1493, human AB serum, PS dual antibiotics and X-VIVO 15 medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, dasatinib, GNE-1858, human AB serum, PS dual antibiotics and AIM-V medium.

The population of TILs prepared by the method of the present invention can be used to prepare drugs for treating cancer. Therefore, the present invention also includes a pharmaceutical composition comprising the population of TILs obtained by the method described in any embodiment herein, and a pharmaceutically acceptable excipient. Herein, the pharmaceutically acceptable excipient refers to a carrier, a diluent and/or an adjuvant that are pharmacologically and/or physiologically compatible with the subject and the active ingredient, including but not limited to: pH regulators, surfactant, carbohydrates, adjuvants, antioxidants, chelating agents, ionic strength enhancers and preservatives. More specifically, the suitable pharmaceutically acceptable excipient can be the excipient commonly used in TIL infusion (e.g., intravenous infusion) in the art.

Typically, a therapeutically effective amount of TILs is included in the pharmaceutical composition. A therapeutically effective amount refers to a dose that can achieve treatment, prevention, alleviation and/or attenuation of a disease or condition in a subject. The therapeutically effective dose can be determined according to factors such as the patient's age, sex, disease and its severity, and other physical conditions of the patient. Herein, a subject or a patient generally refers to a mammal, especially a human.

The present invention also provides a cell cryopreservant preparation, including the aforementioned population of TILs obtained by the method described in any embodiment herein and a cryopreservant.

The cryopreservant herein may be a tissue cryopreservant or a cell cryopreservant, which can keep the TILs of the present invention alive under low temperature conditions, such as cryopreservation by dry ice or liquid nitrogen. Preferably, the cryopreservant may be a serum-free cryopreservant, and the cell cryopreservant preparation can be directly infused into the individual without removal of the cryopreservant after reconstitution from low temperature, without obvious toxic and side effects on the individual.

The invention also includes a cell therapy comprising preparing TILs by the methods described herein, and administered the TILs to a subject. For example, the administered TILs can kill tumor cells in the subject. Additionally, TIL-mediated immune responses can be part of an adoptive immunotherapy procedure.

The present invention will be illustrated by way of specific examples below. It should be understood that these examples are merely explanatory and is not intended to limit the scope of the present invention. Unless otherwise specified, the methods and materials used in the examples are conventional materials and methods in the art.

### Example

### Example 1: The Preparation of Culture Medium

TIL seed cell culture medium was prepared with the following components.

| | Source of purchase | | Source of purchase |
|---|---|---|---|
| IL-1 alpha | R&D 200-LA-010 | OX-40 mAb | R&D MAB10543-100 |
| CD3 mAb | abcam (ab86883) | | |
| IL-1 beta | R&D 201-LB-025 | LAG-3 mAb | R&D MAB23193 |
| IL-2 | R&D 201-GMP-01M | TIGIT mAb | R&D MAB7898 |
| IL-4 | R&D 204-GMP-01M | CTLA-4 mAb | R&D MAB325-500 |
| IL-6 | R&D 206-GMP-01M | PD-1 mAb | R&D MAB10861 |
| IL-7 | R&D 207-GMP-01M | TNF-alpha | R&D 210-GMP-100 |
| IL-9 | R&D 209-ILB-050/CF | RRx-001 | MCE HY-16438 |
| IL-10 | R&D 217-IL-025/CF | sunitinib | MCE HY-10255A |
| IL-12 | R&D 219-GMP-01M | CNI-1493 | MCE HY-15509A |
| IL-15 | R&D 247-GMP-01M | imatinib | MCE HY-15463 |
| IL-18 | Biotechne 9124-IL-500 | CAL-101 | MCE HY-13026 |
| IL-21 | R&D 8879-GMP-01M | dasatinib | MCE HY-10181 |
| G-CSF | R&D 214-CS-025 | LYC-55716 | MCE HY-104037 |
| GM-CSF | R&D 215-GMP-01M | GNE-1858 | MCE HY-135892 |
| M-CSF | R&D 216-GMP-500 | methylene blue | MCE HY-14536 |
| IFN-alpha | R&D 11100-1 | TWS119 | MCE HY-10590 |
| IFN-beta | R&D 8499-IF-010 | Human AB serum(v/v%) | Sigmaaldrich H4522 |
| IFN-gamma | R&D 285-GMP-01M | 100*PS Dual antibiotics (penicillin-streptomycin) | Thermo Fisher15140122 |
| CD137 mAb | R&D AF838 | AIM-V | Thermo Fisher 0870112BK |
| CD28 mAb | R&D MAB342-500 | X-VIVO 15 | Lonza BE02-060F |
| CD40 mAb | R&D MAB6321-500 | CTS^{™} OpTmizer^{™} | Thermo Fisher A1048501 |

GITR antibody was synthesized by GenScript (sequences were from SEQ ID NO:104 and SEQ ID NO: 105 of CN103951753B).

Different TIL cell culture media were prepared according to Table 1-Table 3. The basal medium can be stored at 4°C for no more than 1 month after adding serum and dual antibiotics in proportion. Before use, the medium was taken out from 4°C, placed in a 37°C water bath to pre-warm, and then, other components (interleukins, colony-stimulating factors, interferon, TNF-alpha, various antibodies and other molecules) of working concentrations were added for TIL cell culture. The components corresponding to "coating" in Table 3 (CD3 mAb, CD28 mAb, and CD137 mAb) were not present in the medium in the form of solution components; rather, the stock solution containing these components was added to the bottom or inner walls of cell culture vessels (such as multiwell plates, plates or cell culture bags) and incubated before cell culture. After coating pretreatment, the components became matrix-immobilized and are attached to the bottom of the culture vessel. The method of coating pretreatment can be conventional protein coating process well known in the art. For example, a specific treatment method is as follows: coating stock solutions were prepared with the components to be coated (CD3 mAb, CD28 mAb and/or CD137 mAb) and PBS, the concentration of components contained therein being 5 µg/mL each. Then the coating stock solution was added to the above-mentioned cell culture vessel, and the bottom or inner wall of the cell culture vessel was completely covered by the coating stock solution. The cell culture vessel was incubated at 4°C overnight, then the coating stock solution was discarded, and the cell culture vessel was washed with PBS 5 times for later use. The autologous platelets in Table 3 came from the patient's own blood, and the allogeneic platelets came from the blood of healthy adults, the preparation of the platelets was carried out according to the preparation method disclosed in CN105107233B, which is incorporated herein by reference in its entirety. The mass-activity conversion relationship of some cytokines used in the following media is IL-7: 1mg=1.21 × 10⁷U; IL-15: 1mg=1.14 × 10⁷U; IL-12: 1mg=1.02×10⁷U; IL-21 : 1mg= 1.07×10⁷U.

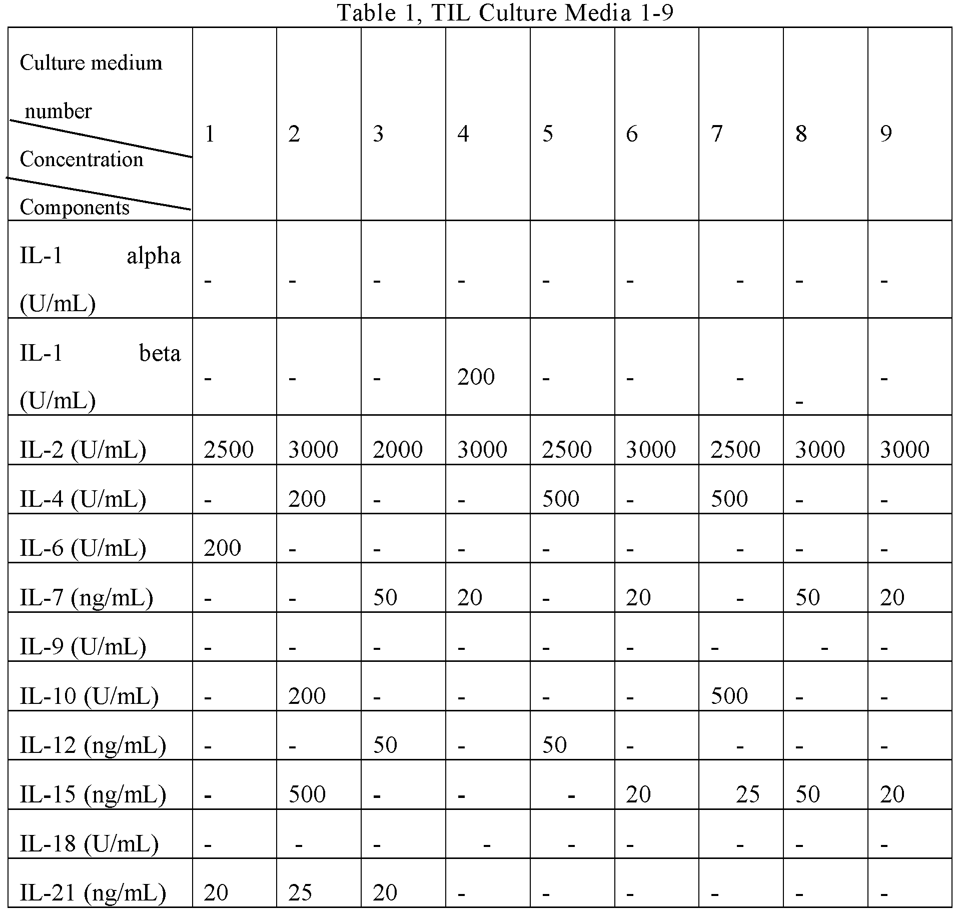

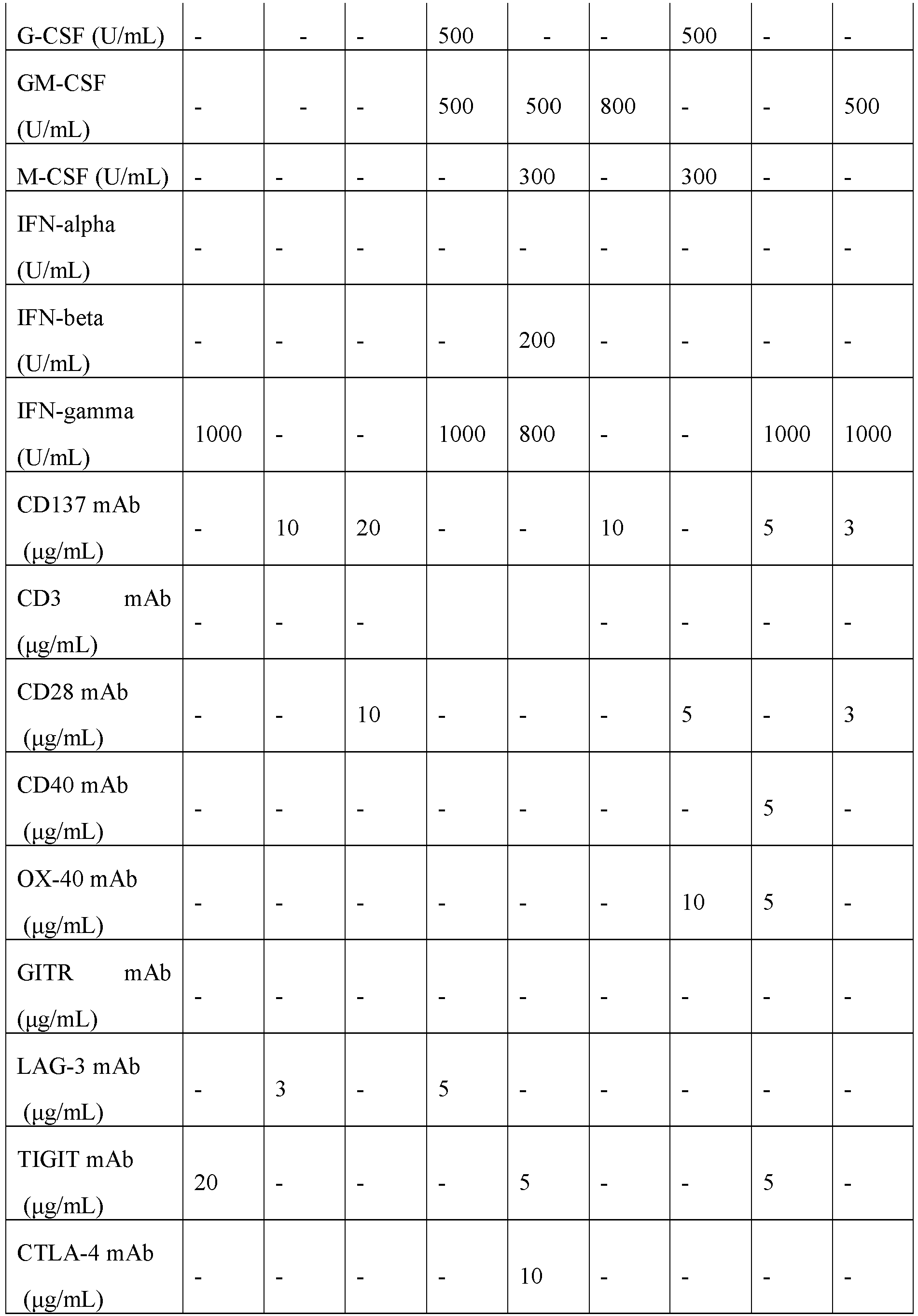

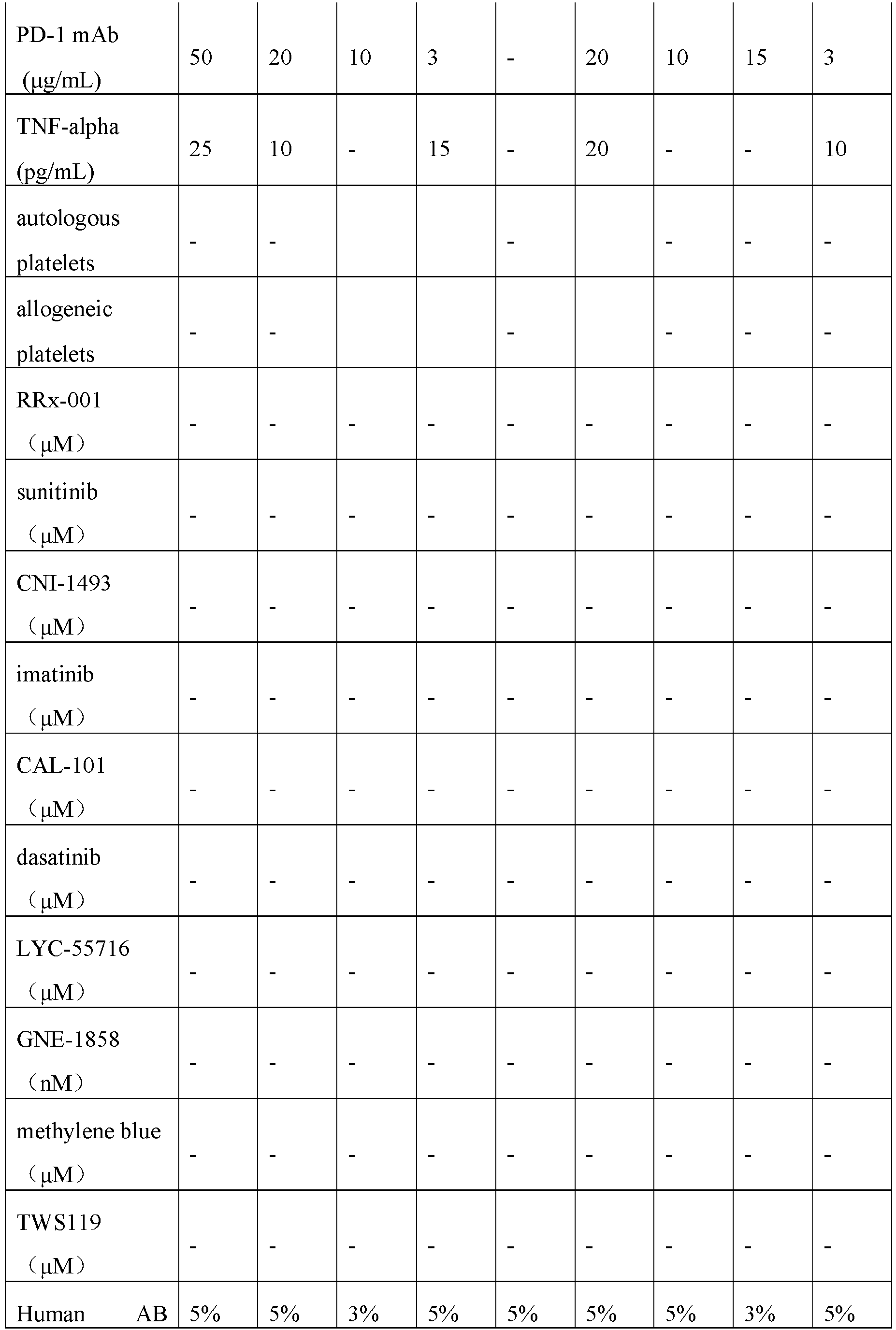

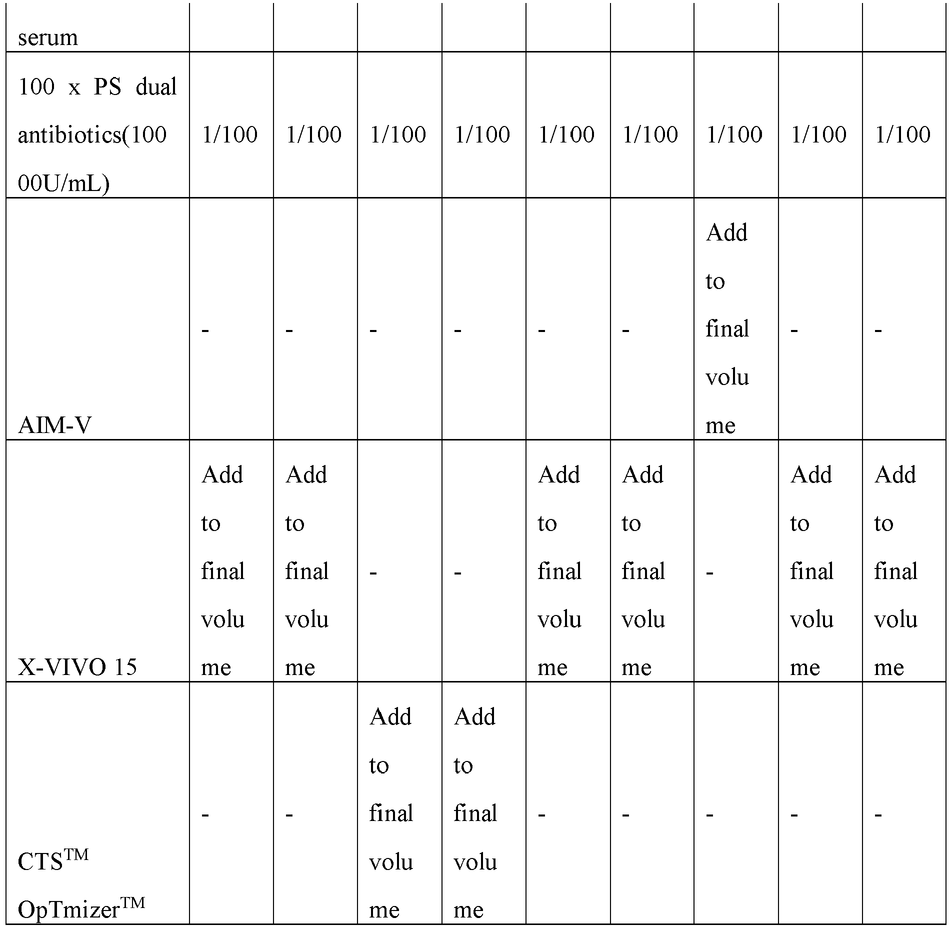

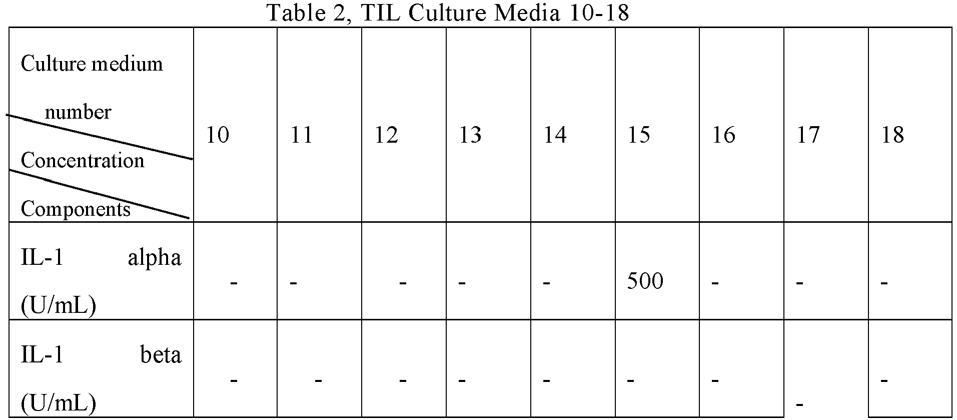

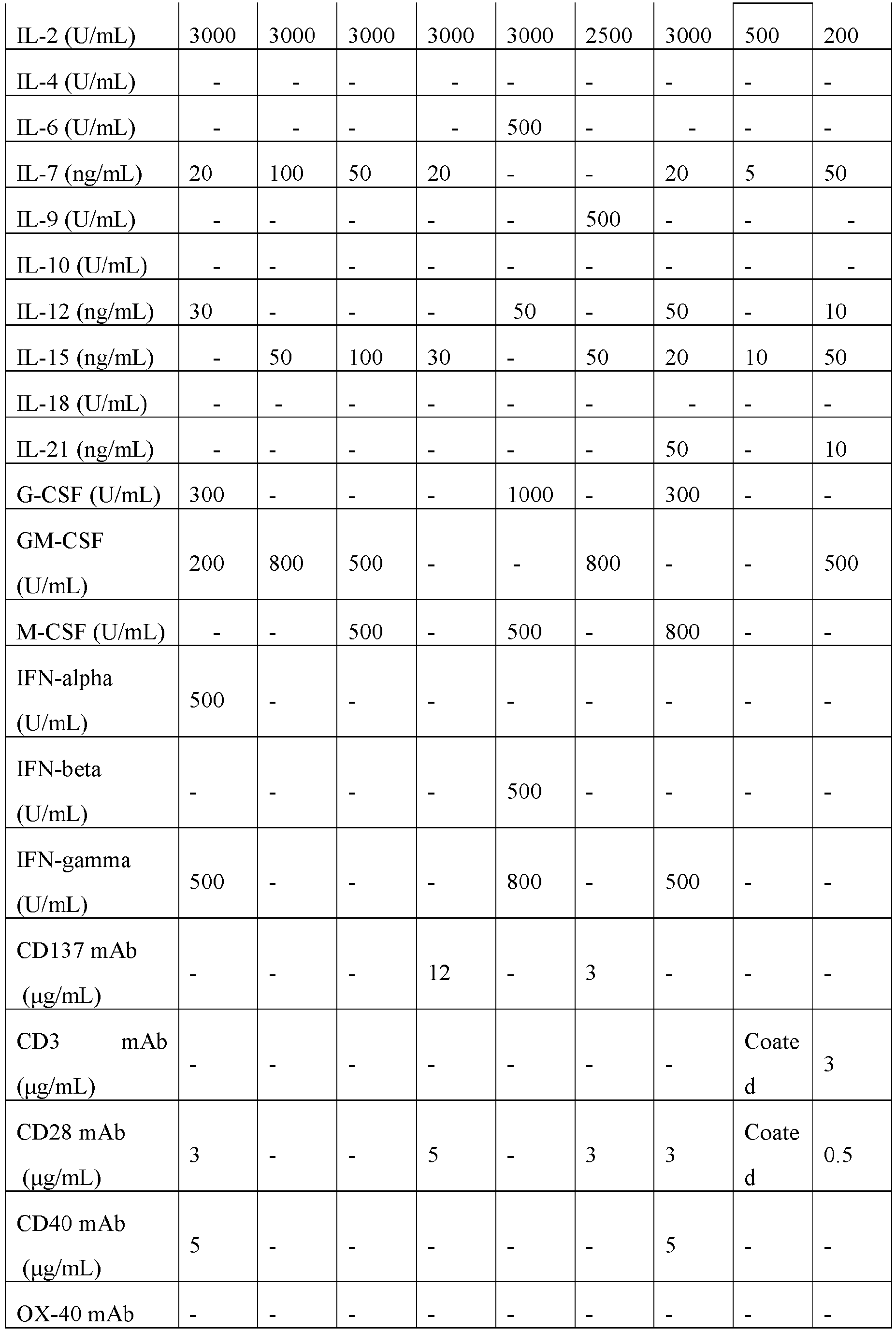

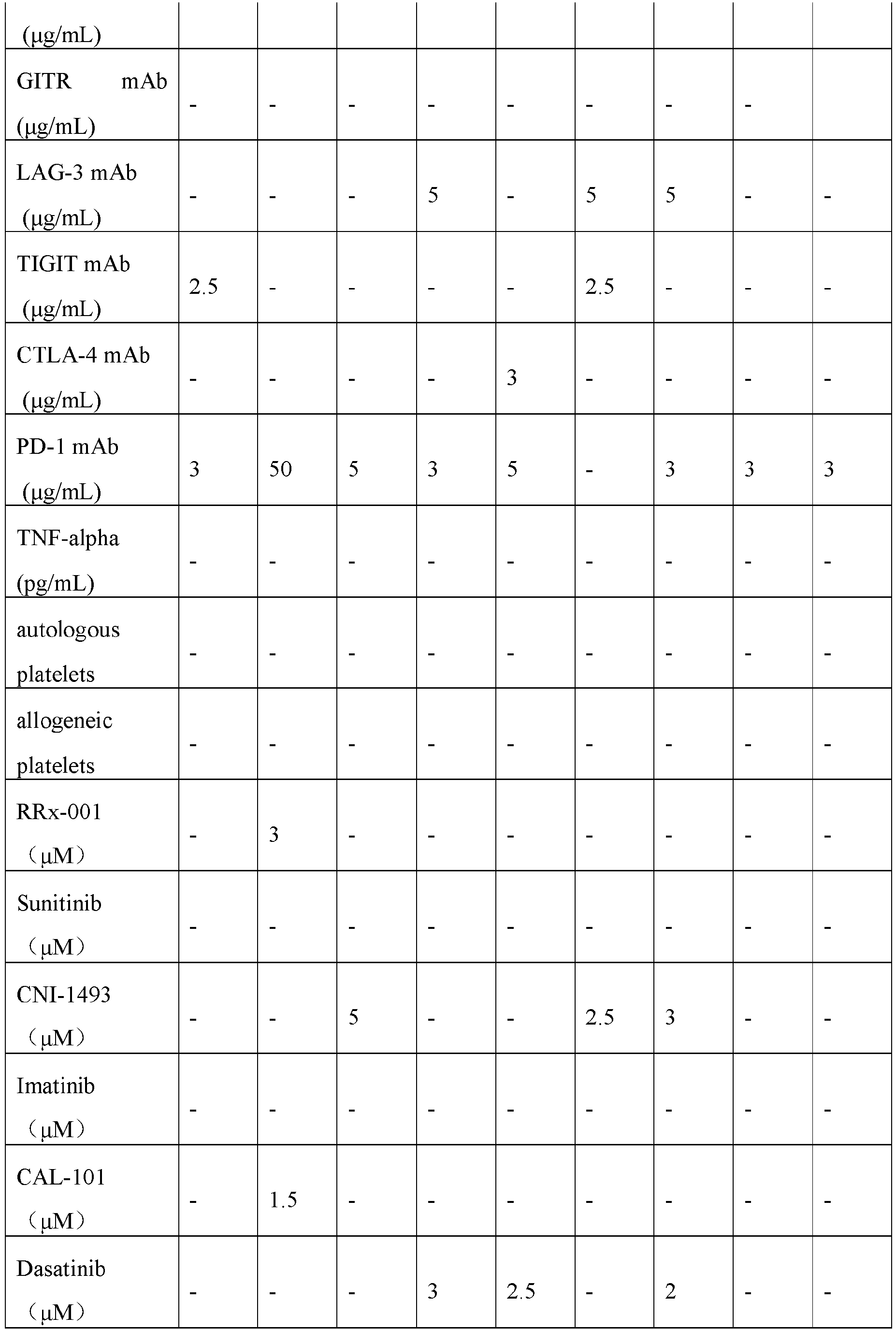

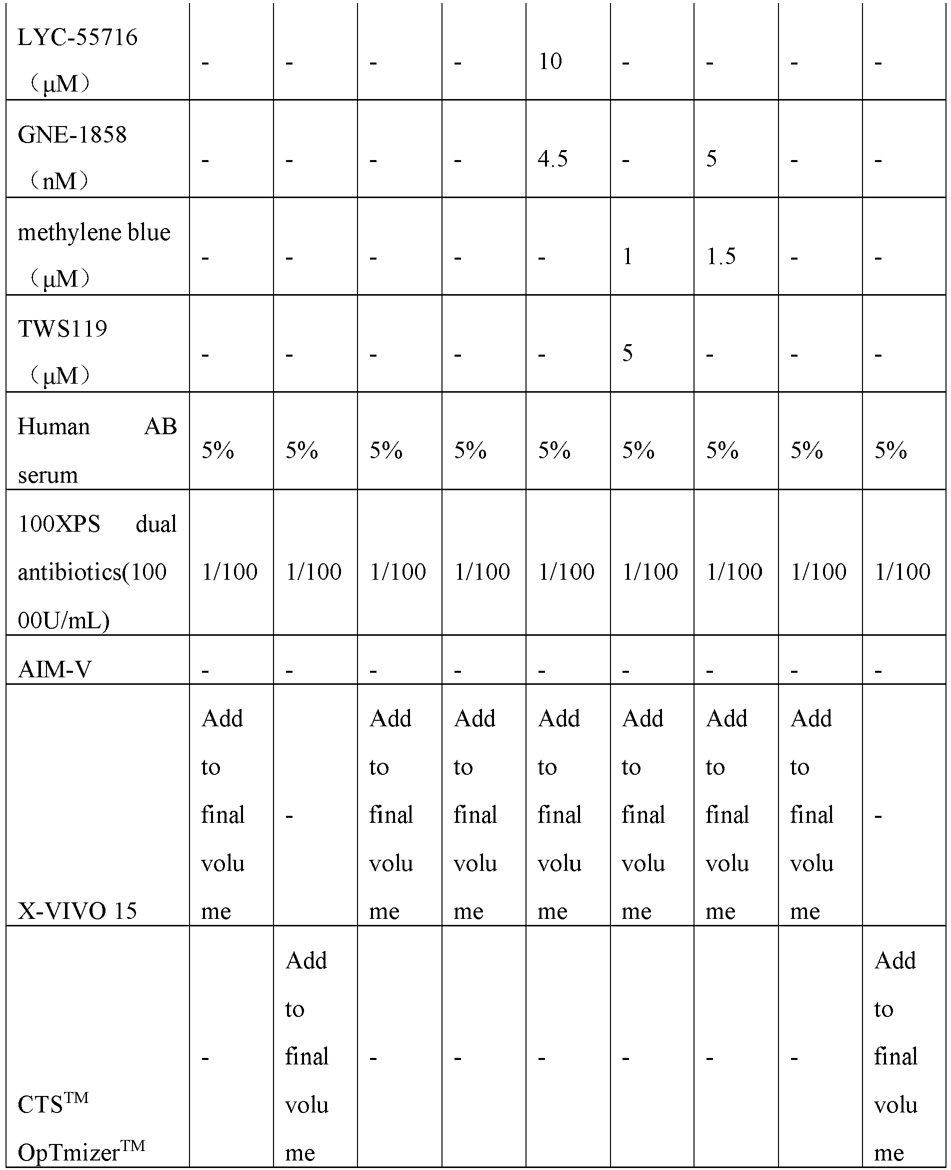

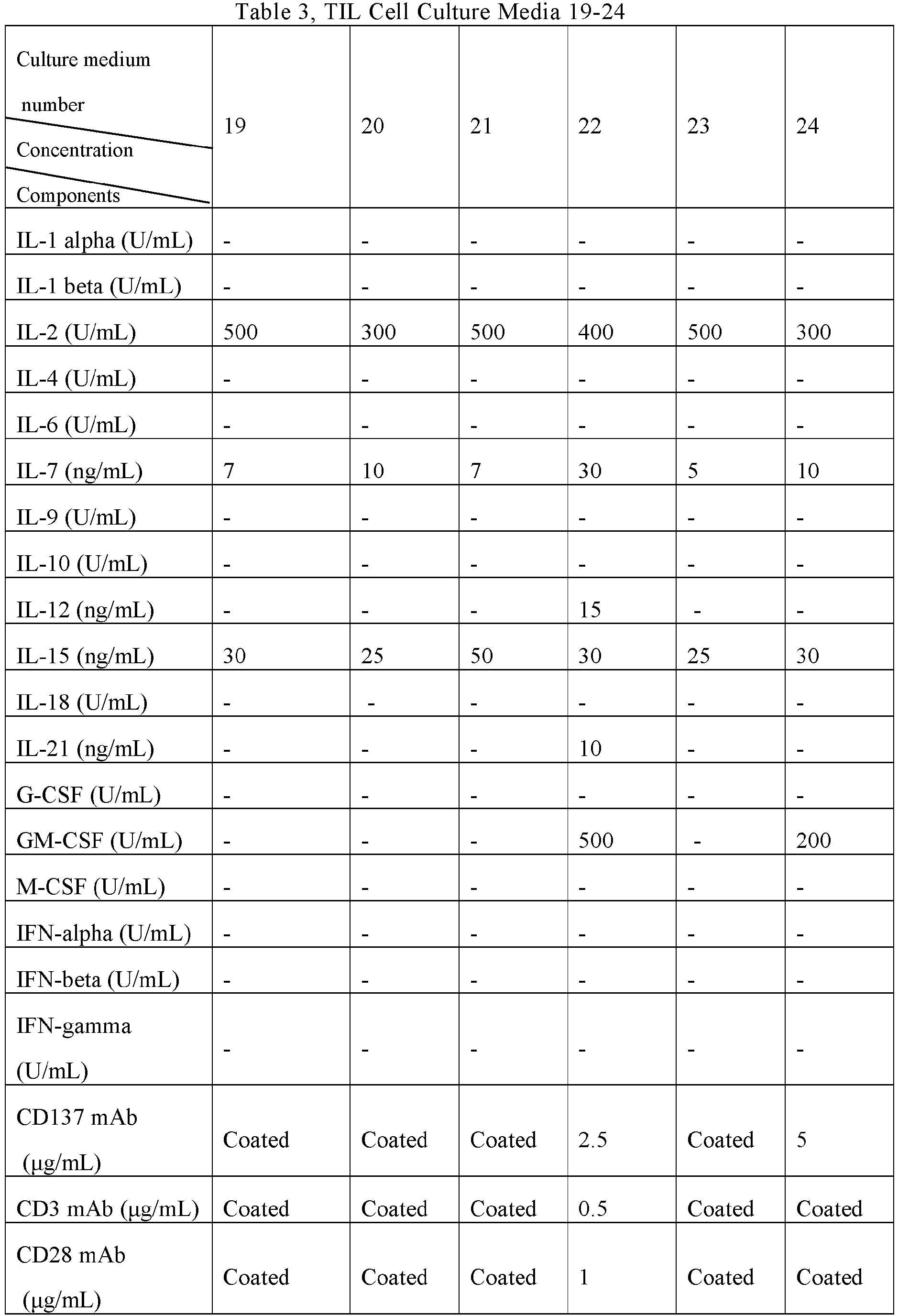

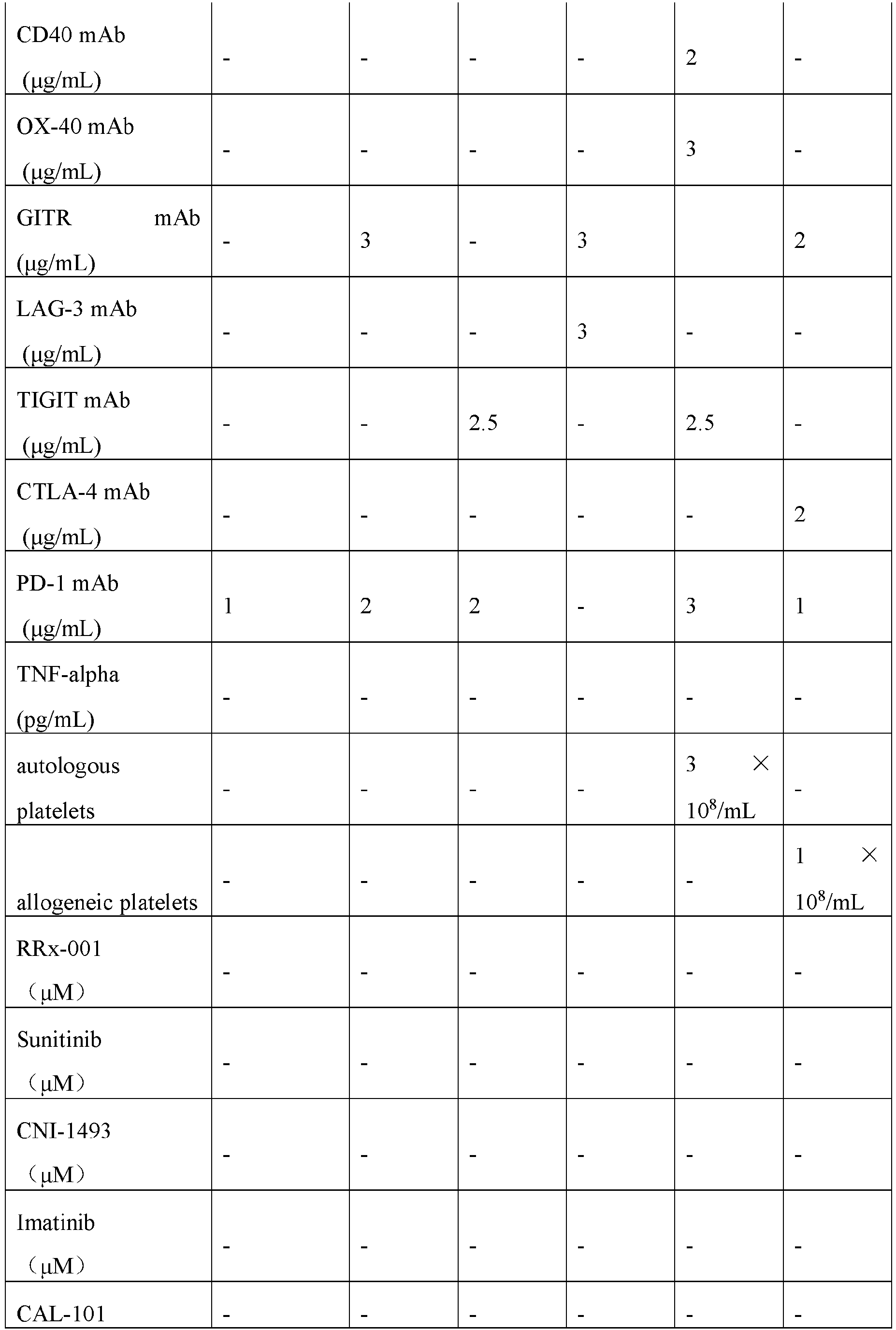

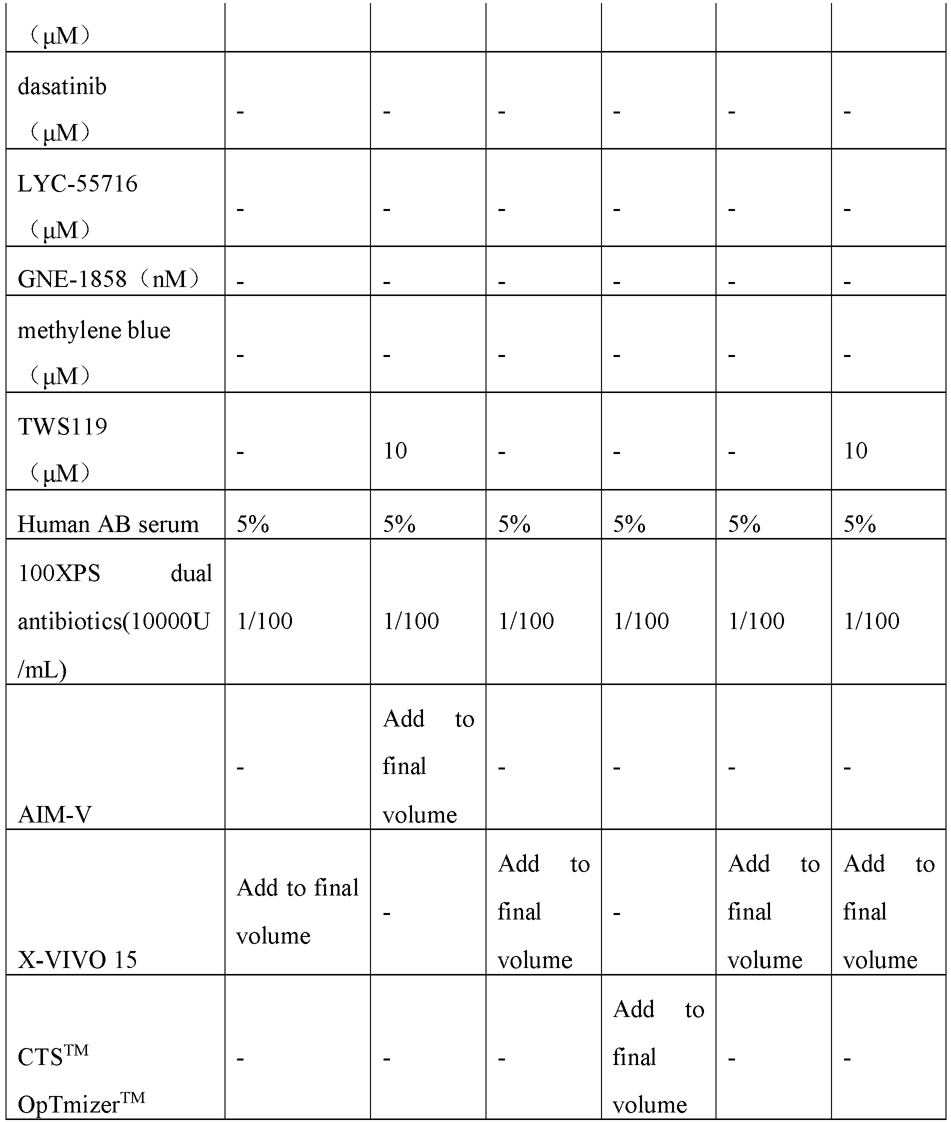

### Example 2: Processing of Patient Solid Tumor Samples and TIL Culture

1) Physiological saline containing final concentrations of 100 U/mL of penicillin, 100 µg/mL of streptomycin and 50 µg/mL of gentamicin was prepared for use;
2) In a sterile environment of a BSL-2 cabinet, the freshly tumor tissue samples isolated from tumor patients were placed and washed in a 10 cm petri dish to which 30 mL of physiological saline prepared in step 1) has been added, then transferred to a new 10cm petri dish to which 30mL of physiological saline prepared in step 1) has been added, and washed for 3 times;
3) Adipose tissue and necrotic tissue were removed with a sterile scalpel blade. The tumor tissues were cut into small fragments with a diameter of 3×3×3mm³, two G-REX100 culture tanks (purchased from Wilsonwolf) were prepared, and 42 randomly selected tumor tissue fragments were placed in each G-REX100 culture tank. One of the TIL culture media prepared in Example 1 was added to the culture tank as the seed cell culture medium; the excess tumor tissue fragments were cryopreserved with cryopreservant CryoStor10 (purchased from BioLifeSolutions) in liquid nitrogen by a controlled-rate freezer;
4) After 1 L of seed cell culture medium was added to the G-REX100 culture tank containing tumor tissue fragments of 3), the tumor tissue fragments were cultured at 37°C with 5% CO₂. Half of the old seed cell culture medium was removed every 4 days, and same volume of the fresh seed cell medium was added, and TIL seed cells were centrifuged and harvested on the 11th-15th day, then total number of cells were counted and viability measured;
5)
   a: For the expansion media (corresponding to media No.18 and 22 in Table 2-Table 3) in which all components are medium solution components, 5 L of the pre-warmed expansion medium was added to the G-REX500M culture tank (purchased from Wilsonwolf), the seed cells obtained in step 4) were inoculated at a seeding density of 2.0 ×10⁵/cm²~5.0×10⁵/cm², and cultured at 37° C with 5% CO₂. After cell count was performed every 4 days, half of the old expansion medium was removed and the same volume of fresh expansion medium was added. After the total number of cells in each G-REX500M tank reached 1.0×10¹⁰, the cells were aliquoted to multiple tanks at the ratio of 1:2, 5L of fresh expansion medium was added and cell culture continued. The cells were cultured in the expansion medium for a total of 12-15 days before they were harvested, and product TILs were thus obtained;
   b:For the expansion medium containing some components which have been coated in advance as described above (corresponding to the media No.17, 19, 20, 21, 23, 24 in Table 2-Table 3), the seed cells harvested in 4) were collected and re-suspended to 1.0× 10⁵/mL~1.0×10⁶/mL with the expansion medium containing all other components in the corresponding solution except for the coating components, added to the cell culture vessel pretreated with the coating components and activated at 37°C with 5% CO₂ for 2-3 days. Then the activated cells were collected by centrifugation and inoculated into a G-REX500M culture tank with pre-warmed expansion medium. The expansion medium in the culture G-REX500M tank is the same expansion medium containing all other components in the corresponding solution except for the coating components. The expansion medium volume in each G-REX500M is 5L. The activated seed cells were inoculated at the seeding density of 2.0X 10⁵/cm²~5.0×10⁵/cm², and cultured at 37° C with 5% CO₂. After cell count was performed every 4 days, half of the old expansion medium was discarded, and fresh expansion medium of the same volume was added. When the total number of cells in each G-REX500M tank reached 1.0×10¹⁰, the cells were aliquoted into multiple tanks at the ratio of 1:2, 5L of fresh expansion medium was added to each tank and cell culture continued. The cells were cultured in the expansion medium in the G-REX500M culture tank for a total of 10-12 days before they were harvested, and product TILs were thus obtained;
6) The phenotypes of the product cells obtained in 5) were detected by flow cytometer, and the IFN-gamma secretion level was measured by HTRF IFN-gamma detection kit (Cisbio Human IFN gamma kit, catalog number: 62HIFNGPET) according to the method described in the instruction.

The following table 4 shows tumor tissue sample numbering, cancer type, seed cell culture medium and expansion medium used in the culture process corresponding to the numbering in Table 1-Table 3, the respective culture time of each tissue sample-matched seed cell culture medium and expansion culture medium and G-REX500M cell seeding density; Table 5 shows the types of coated vessels, re-suspended cell density for coated activation and coated activation time of the corresponding tumor tissue samples in step 5) b. Table 4, TIL-derived tissue sample information and the corresponding number of the medium used

| | Cancer type | Seed cell medium No. | Culture time of the seed cell medium (days) | Expansi on medium No. | Cell seeding density in G-REX500M (cells/cm²) | Culture time of the expansion medium (days) |
|---|---|---|---|---|---|---|
| T001 | gastric cancer | 12 | 12 | 17 | 2.5×10⁵ | 12 |
| T002 | intestin al metasta sis of melano ma | 14 | 12 | 20 | 2.5×10⁵ | 12 |
| T003 | cervical cancer | 5 | 12 | 19 | 2.0×10⁵ | 12 |
| T004 | gastric cancer | 11 | 11 | 21 | 2.5×10⁵ | 12 |
| T005 | gastric cancer | 2 | 12 | 23 | 2.5×10⁵ | 11 |
| T006 | intestin al cancer | 8 | 12 | 19 | 2.5×10⁵ | 10 |
| T007 | ovarian cancer | 6 | 14 | 18 | 3.0×10⁵ | 15 |
| T008 | cervical cancer | 7 | 12 | 24 | 2.5×10⁵ | 12 |
| T009 | bladder metasta | 9 | 12 | 19 | 2.5×10⁵ | 12 |
| | sis of re current cervical cancer | | | | | |
| T010 | ovarian metasta sis of gastric cancer | 15 | 11 | 20 | 4.5×10⁵ | 12 |
| T011 | glioma | 1 | 15 | 17 | 5.0×10⁵ | 12 |
| T012 | cervical cancer (recurre nt) | 3 | 12 | 23 | 2.5×10⁵ | 12 |
| T013 | melano ma | 16 | 13 | 24 | 2.5×10⁵ | 12 |
| T014 | cervical cancer | 4 | 12 | 20 | 2.5×10⁵ | 12 |
| T015 | lymph atic meta stasis of endome trial cancer | 10 | 12 | 22 | 2.5×10⁵ | 15 |
| T016 | lymph node metasta sis of intestin al cancer | 13 | 12 | 21 | 2.5×10⁵ | 12 |
| T017 | ovarian cancer | 9 | 12 | 20 | 2.5×10⁵ | 12 |

**Table 5, Coated stimulation conditions of TIL seed cells of some of the samples**

| | Cancer type | Types of Coated Vessels | Activated re-suspended cell density (/mL) | Activating time (days) |
|---|---|---|---|---|
| T001 | gastric cancer | cell culture bag | 2.0×10⁵ | 2 |
| T002 | intestinal metastasis of melanoma | cell culture bag | 2.5×10⁵ | 3 |
| T003 | cervical cancer | cell culture bag | 1.0×10⁵ | 3 |
| T004 | gastric cancer | 245mm cell culture dish | 5.2×10⁵ | 3 |
| T005 | gastric cancer | cell culture bag | 2.5×10⁵ | 3 |
| T006 | intestinal cancer | cell culture bag | 2.5×10⁵ | 3 |
| T008 | cervical cancer | cell culture bag | 2.5×10⁵ | 3 |
| T009 | bladder metastasis of recurrent cervical cancer | cell culture bag | 2.5×10⁵ | 3 |
| T010 | ovarian metastasis of gastric cancer | cell culture bag | 2.5×10⁵ | 2 |
| T011 | glioma | 245mm cell culture dish | 1.0×10⁶ | 3 |
| T012 | cervical cancer (recurrent) | cell culture bag | 3.5×10⁵ | 2 |
| T013 | melanoma | cell culture bag | 2.5×10⁵ | 3 |
| T014 | cervical cancer | 245mm cell culture dish | 2.5×10⁵ | 3 |
| T016 | lymph node metastasis of intestinal cancer | 245mm cell culture dish | 2.5×10⁵ | 2 |
| T017 | ovarian cancer | cell culture bag | 2.5×10⁵ | 2 |

245mm cell culture dishes were purchased from Corning (Cat.431111), and cell culture bags were purchased from Takara (CultiLife^{™}215 Culture Bag, Cat.FU0005). The whole process of preparation of the above-mentioned product TILs was carried out in a GMP-level workshop in accordance with the requirements of relevant regulations. After harvest the cells were centrifuged, washed with D-PBS, and re-suspended in normal saline. The cells passed the quality control tests (CD3+ ratio, CD4+ and CD8+ ratio, IFN-gamma secretion level, sterility, endotoxin, osmotic pressure, etc.) and all indicators met the requirements of the "Pharmacopoeia of the People's Republic of China" or the corresponding general standards of immune cell therapy products that have been marketed globally before they were released.

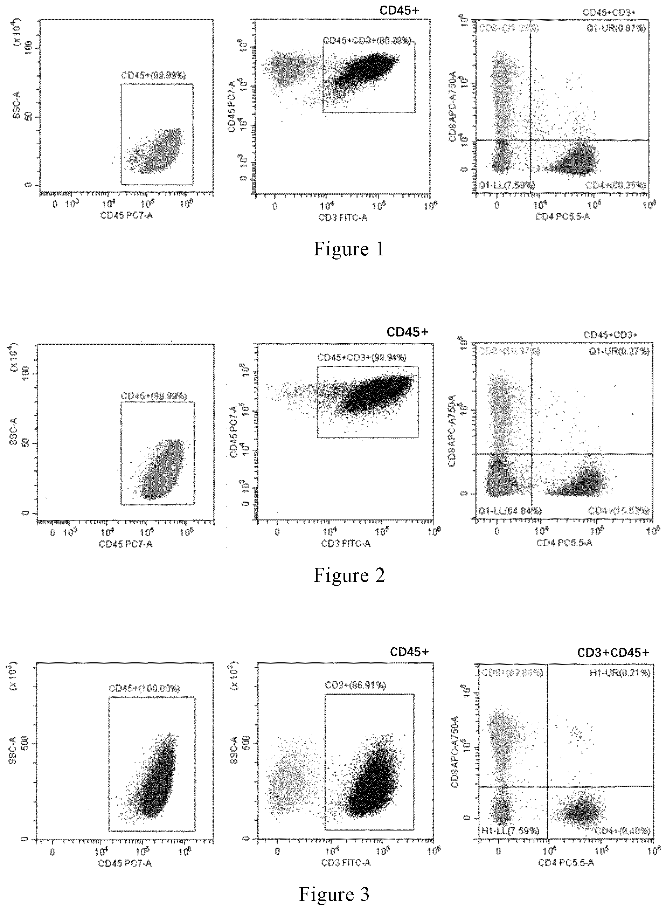

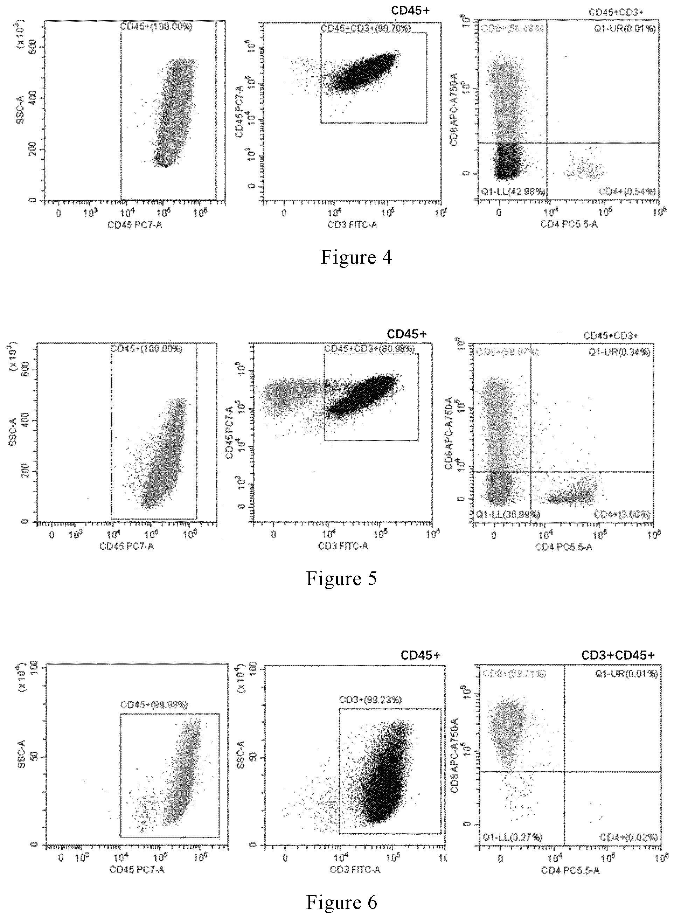

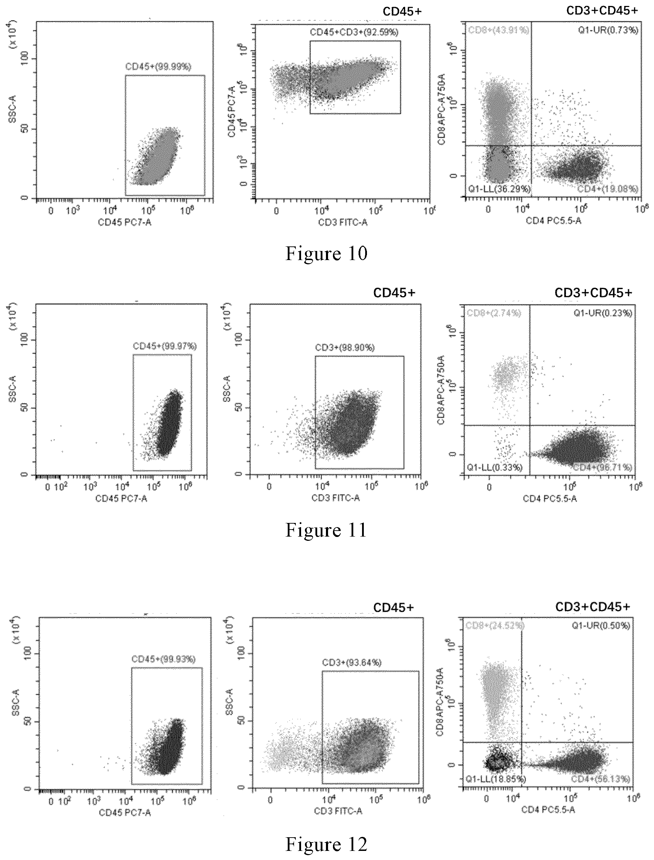

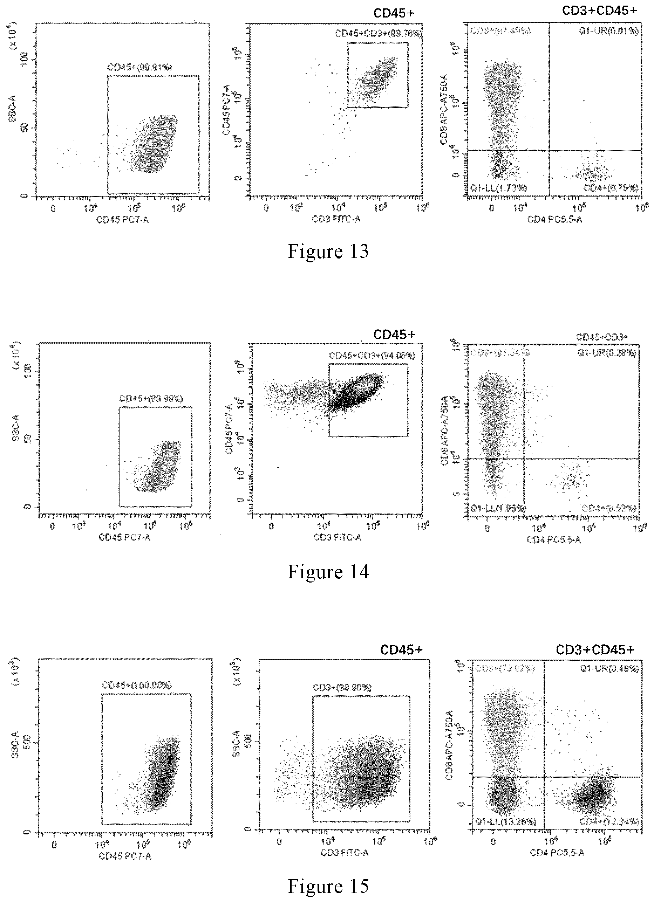

The results were shown in Table 6 and Figures 1-17. Table 6 showed the seed cell number, product cell number, product cell viability, product cell phenotype and IFN-gamma secretion levels of product cells of the tumor-infiltrating lymphocytes (TIL) obtained from each tissue sample in Table 4 using the culture medium and culture method of the present invention. Figures 1 to 17 correspond to the results of flow cytometric analysis of product TILs derived from tissue samples numbered T001, T002, T003, T004, T005, T006, T007, T008, T009, T010, T011, T012, T013, T014, T015, T016 and T017.

**Table 6, TIL culture results of different sources of tissue**

| | Seed cell number | Product cell number | Product cell viability | IFN-γ (pg/mL) | The ratio of CD45+ cells | The percenta ge of CD3+ cells in CD45+ cells |
|---|---|---|---|---|---|---|
| T001 | 2.01×10⁸ | 1.58×10¹¹ | 95.41% | 12173.47 | 99.99% | 86.39% |
| T002 | 2.48×10⁸ | 2.75×10¹¹ | 96.93% | 8812.52 | 98.94% | 98.94% |
| T003 | 1.27×10⁸ | 1.25×10¹¹ | 94.86% | 9712.25 | 100% | 86.91% |
| T004 | 1.93×10⁸ | 2.32×10¹¹ | 92.59% | 10039.71 | 100% | 99.70% |
| T005 | 0.94×10⁸ | 1.32×10¹¹ | 98.48% | 8969.41 | 100% | 80.98% |
| T006 | 1.52×10⁸ | 1.53×10¹¹ | 92.03% | 7298.34 | 99.98% | 99.23% |
| T007 | 1.35×10⁸ | 0.58×10¹¹ | 95.61% | 8015.98 | 99.96% | 97.36% |
| T008 | 1.45×10⁸ | 1.47×10¹¹ | 98.94% | 8730.66 | 99.94% | 97.53% |
| T009 | 1.53×10⁸ | 2.19×10¹¹ | 97.03% | 10213.74 | 99.99% | 87.75% |
| T010 | 2.57×10⁸ | 2.97×10¹¹ | 91.33% | 7523.71 | 99.99% | 92.59% |
| T011 | 0.89×10⁸ | 0.76×10¹¹ | 88.76% | 8789.58 | 99.97% | 98.90% |
| T012 | 1.11×10⁸ | 1.46×10¹¹ | 97.29% | 9910.74 | 99.93% | 93.64% |
| T013 | 3.09×10⁸ | 4.30×10¹¹ | 98.96% | 11203.06 | 99.91% | 99.76% |
| T014 | 1.16×10⁸ | 1.39×10¹¹ | 92.94% | 9811.08 | 99.99% | 94.06% |
| T015 | 1.92×10⁸ | 0.55×10¹¹ | 97.75% | 10196.23 | 100% | 98.90% |
| T016 | 2.03×10⁸ | 2.49×10¹¹ | 93.28% | 9481.61 | 99.90% | 79.25% |
| T017 | 1.18×10⁸ | 1.63×10¹¹ | 98.47% | 12016.55 | 100% | 97.69% |

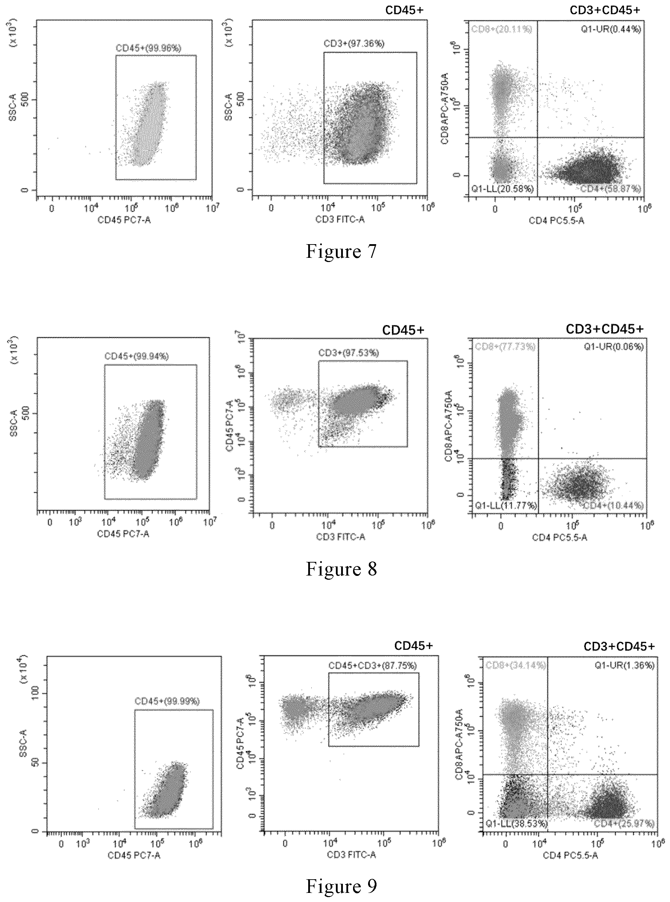

The results in Table 6 shows that the solid tumor tissues cultured with various groups of seed cell culture media of the present invention can generate a total number of at least about 10⁸ cells at the time of harvest on day 12, and the total number of TIL seed cells could be as high as more than 2×10⁸ and more than 3×10⁸ for some tumor samples, such as T002, T010 and T013. After the seed cells of each group were cultured in different expansion media of the present application, over 10¹⁰-10¹¹ product TILs could be obtained when finally harvested on about day 24, and in most groups were more than 10¹¹. The survival rate of harvested product TILs in all groups was relatively high. Except for a few samples, most of them exceeded 90%, and some were close to 99%, such as T005, T008 and T013. The secretion of IFN-gamma of the product TILs in each group tested was also at a relatively high level, indicating that all product TILs obtained by the medium and culture method of the present application are in a relatively highly activated state. Figures 1 to 17 show that all groups of product TILs were high-purity lymphocytes, and the proportion of CD45+ cells was 100% or close to 100%. Among all product TILs, the lowest proportion of CD3+ cells was close to 80% (79.25%), and most groups of product TILs had a CD3+ cell ratio of more than 90%, and in some groups this was close to 100% (such as T006, T013, T002, T015). Among the product TILs of all sample groups, the proportion of CD8+ T cells in the CD3+ cell population of most groups was close to or exceeded 50%, which showed an absolute advantage over CD4+ T cells, as shown in Figures 3, 4, 5, 6, 8, 10, 13, 14, 15, 16 and 17; the proportion of CD8+T cells and CD4+T cells in some groups was close to 1:1, as shown in Figures 2 and 9. The above results show that the TILs obtained from different tumor tissue samples by various combinations of seed cell culture medium and expansion medium according to the present invention had superior phenotypes.

### Example 3: Killing effect of TIL derived from T001 on target cells

The gastric cancer cell line HGC27 (all purchased from Merck Cat.94042256) was used as the target cell, and the *in vitro* killing effect of the T001 tissue-derived TILs prepared in Example 2 was measured by the real-time label-free cell function analyzer (RTCA) of Essen Company. The specific steps were as follows:
(1) Zero adjustment: 50 µL of DMEM culture solution or 1640 culture solution were added to each well which was put into the instrument. Step 1 was selected, and zero adjustment was performed ;
(2) Plating target cells: HGC27 cells were plated at the density of 10⁴ cells/50 µL per well on a plate containing detecting electrodes, sat for a few minutes to settle, and then were placed in the instrument. Starting step 2, cell culture;
(3) Adding effector cells: After the target cells were cultured for 19 hours until the cell index reached 1.0, step 2 paused and 50 µL of effector cells were added into each well. The effector cells were added at the effector-to-target ratio of 2:1, 4:1 and 8:1 according to the viability of TIL, then starting step 3, and the cells continued to co-culture for more than 50 hours, and the cell proliferation curve was monitored.

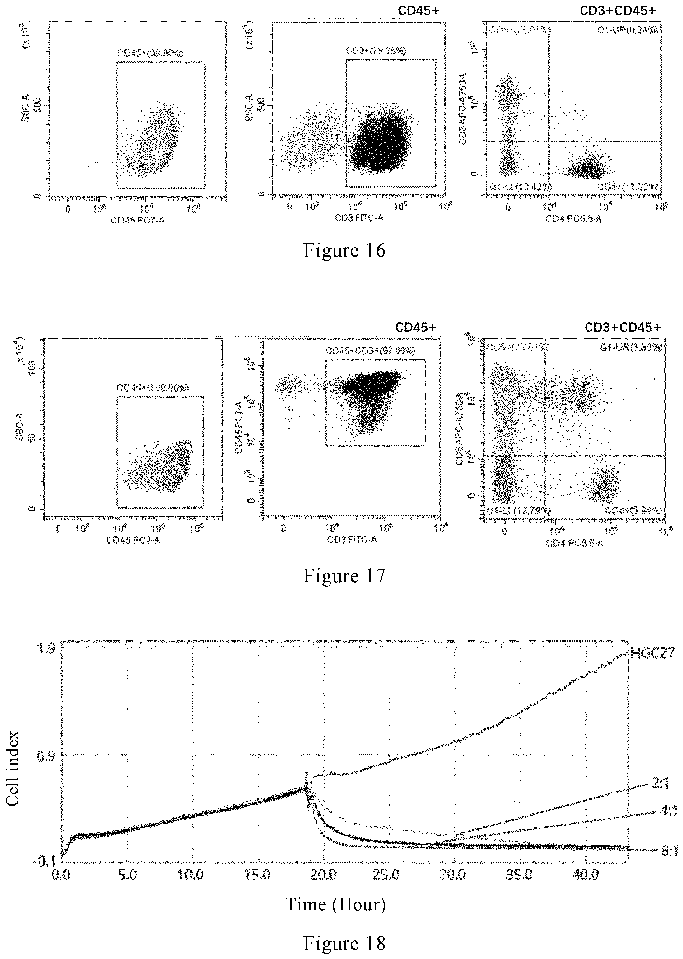

The results were shown in Figure 18. When the effect-to-target ratio was 2:1, compared with the tumor cell control group, T001-derived TILs had shown a significant killing effect on tumor cell HGC27. With the increase in the effector-to-target ratio, the killing effect of TILs on HGC27 significantly improved as well.

### Example 4: Killing effect of TIL derived from T002 on target cells

The melanoma cell line A375 (all purchased from ATCC) was used as the target cell, and the *in vitro* killing effect of the T002 tissue-derived TIL product cells prepared in Example 2 was measured by the real-time label-free cell function analyzer (RTCA) of Essen Corporation. Specific steps were as follows:
(1) Zero adjustment: 50 µL of DMEM culture solution or 1640 culture solution were added to each well, which was put into the instrument. Step 1 was selected, and zero adjustment was performed;
(2) Plating target cells: target cells A375 were plated at the density of 10⁴ cells/50 µL per well on a plate containing detecting electrodes, sat for a few minutes to settle, and then were placed in the instrument. Starting step 2, cell culture;
(3) Adding effector cells: after the target cells were cultured for 20 hours until the cell index reached 1.5, step 2 paused and 50 µL of effector cells were added into each well. The effector cells were added at the effect-to-target ratio of 2:1, 4:1 and 8:1 according to the viability of TIL, then starting step 3, and the cells continued to co-culture for more than 50 hours, and the cell proliferation curve was monitored.

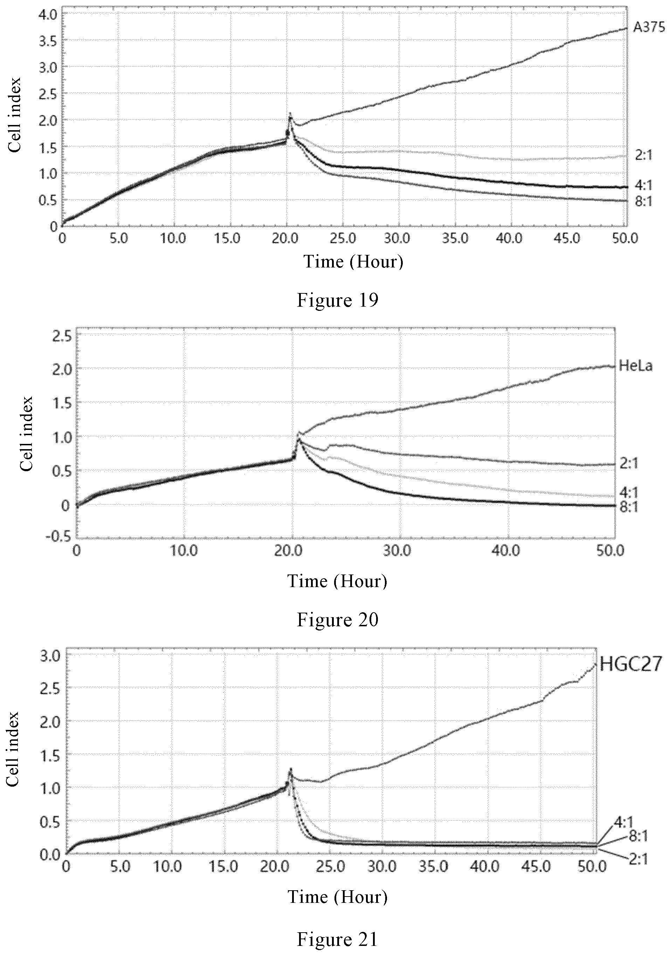

The results are shown in Figure 19. When the effector-to-target ratio was 2:1, T002-derived TILs showed a significant killing effect on tumor cell A375 compared with the tumor cell control group. With the increase in the effector-to-target ratio, the killing effect of TILs on A375 significantly improved as well.

### Example 5: Killing effect of TIL derived from T003 on target cells

The cervical cancer cell line HeLa (all purchased from ATCC) was used as the target cell, and the in vitro killing effect of the T003 tissue-derived product TILs prepared in Example 2 was measured by the real-time label-free cell function analyzer (RTCA) of Essen Company. Specific steps were as follows:
(1) Zero adjustment: 50 µL of DMEM or 1640 culture solution were added to each well, which was put into the instrument. Step 1was selected, and zero adjustment was performed;
(2) Plating target cells: target cells Hela were plated at a density of 10⁴ cells/50 µL per well on a plate containing detecting electrodes, sat for a few minutes to settle, then placed in the instrument. Starting step 2, cell culture;
(3) Adding effector cells: after the target cells were cultured for 20 hours until cell index reached 1.0, step 2 paused and 50 µL of effector cells were added into each well. The effector cells were added at the effector-to-target ratio of 2:1, 4:1 and 8:1, respectively according to the viability of TIL, then starting step 3, and the cells continued to co-culture for more than 50 hours, and the cell proliferation curve was monitored.

The results were shown in Figure 20. When the effector-to-target ratio was 2:1, T003-derived TILs showed a significant killing effect on tumor cell Hela compared with the tumor cell control group. With the increase in the effector-to-target ratio, the killing effect of TILs on Hela also significantly improved.

### Example 6: Killing effect of TIL derived from T005 on target cells

The gastric cancer cell line HGC27 (all purchased from Merck Cat.94042256) was used as the target cell, and the in vitro killing effect of the T005 tissue-derived product TILs prepared in Example 2 was measured by the real-time label-free cell function analyzer (RTCA) of Essen Company. Specific steps were as follows:
(1) Zero adjustment: 50 µL of DMEM or 1640 culture solution were added to each well, which was put into the instrument. Step 1 was selected, and zero adjustment was performed;
(2) Plating target cells: target cells HCG27 were plated at a density of 10⁴ cells/50 µL per well on a plate containing detecting electrodes, sat for a few minutes to settle, and then placed in the instrument. Starting step 2, cell culture;
(3) Adding effector cells: after the target cells were cultured for 21 hours until the cell index reached about 1.0, step 2 paused and 50 µL of effector cells were added into each well. The effector cells were added at the effector-to-target ratio of 2:1, 4:1 and 8:1 according to the viability of TIL, then starting step 3, and the cells continued co-culture for more than 50 hours, and the cell proliferation curve was monitored.

The results are shown in Figure 21. When the effector-to-target ratio was 2:1, , T005-derived TILs showed a stronger killing effect on tumor cell HGC27compared with the tumor cell control group. And with the increase in the effector-to-target ratio, the killing effect of TILs on HGC27 did not significantly further improve, indicating that the killing effect of TILs derived from T005 on the target cell HGC27 had reached the upper limit even under low effector-to-target ratio.

### Example 7: Killing effect of TIL derived from T004 on target cells

The gastric cancer cell line HGC27 (all purchased from Merck Cat.94042256) was used as the target cell, and the *in vitro* killing effect of the T004 tissue-derived product TILs prepared in Example 2 was measured by the real-time label-free cell function analyzer (RTCA) of Essen Company. Specific steps were as follows:
(1) Zero adjustment: 50 µL of DMEM or 1640 culture solution were added to each well, which was put into the instrument. Step 1 was selected, and zero adjustment was performed;
(2) Plating target cells: target cells HCG27 were plated at a density of 10⁴ cells/50 µL per well on a plate containing detecting electrodes, sat for a few minutes to settle, and then placed in the instrument. Starting step 2, cell culture;
(3) Adding effector cells: after the target cells were cultured for 21 hours until the cell index was about 1.5, step 2 paused and 50 µL of effector cells were added into each well. The effector cells were added at the effector-to-target ratio of 2:1, 4:1 and 8:1 according to the viability of TIL, then starting step 3, and the cells continued to co-culture for more than 80 hours, and the cell proliferation curve was monitored.

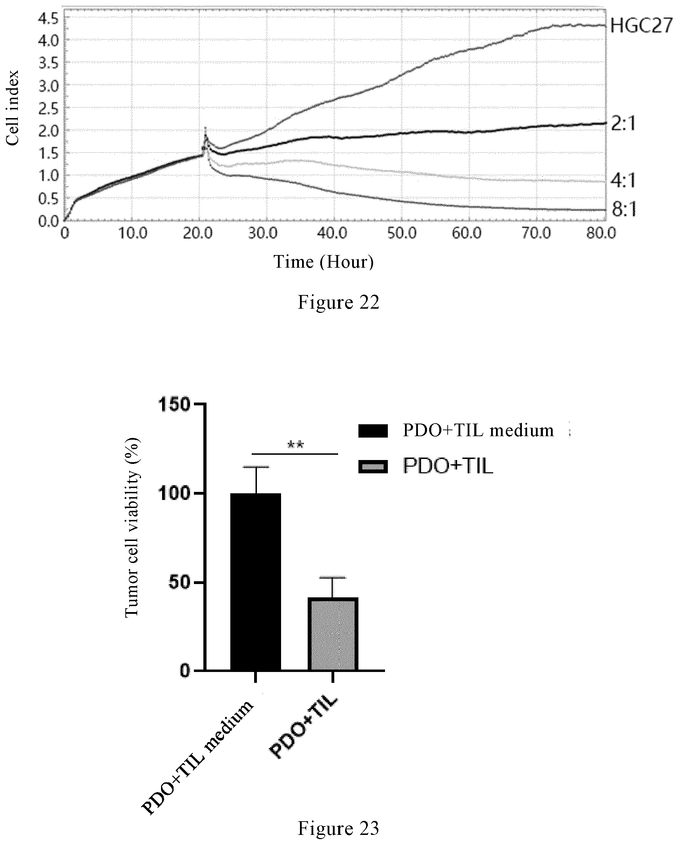

The results are shown in Figure 22. When the effector-to-target ratio was 2:1, T004-derived TILs showed partial killing effect on tumor cell HGC27 compared with the tumor cell control group. With the increase in the effector-to-target ratio, the killing effect of TILs on HGC27 showed a gradual and obvious increase as well.

### Example 8: Killing effect of TILs on syngeneic tumor organoids

Fresh T006 tumor tissue in Example 2 was treated according to the method described on page 10 of published reference (Chiara M Cattaneo, Krijn K Dijkstra, Lorenzo F Fanchi, Sander Kelderman, Sovann Kaing, Nienke van Rooij, Stieneke van den Brink, Ton N Schumacher, Emile E Voest. Tumor organoid-T-cell coculture systems Nat Protoc.2020 Jan; 15(1):15-39, which was incorporated herein by reference in its entirety), and organoids (Patient-derived organoid, PDO) culture was performed. The kits and instruments used for the culture were purchased according to the records in the published reference. Co-culture and killing assay of the syngeneic TILs (T006 product TILs) and PDO which had been successfully cultured and passaged for 2-3 times) were performed according to the following procedure:
Day 1:
   1. The PDO medium in the cultured T006 PDO system was discarded, the PDOs were treated with an appropriate amount of pre-warmed dispase (2 mg/mL dissolved in PBS), gently pipetted, and placed at 37°C for 5-15min to avoid single cells;
   2. The PDOs were transferred to a centrifuge tube, 100 µL of EDTA (0.5M) was added to 1 mL of dispase, and PBS was added to reach a volume of 10 mL and the tube was centrifuged;
   3. The PDOs were transferred to a 12-well plate in 2 mL of PDO medium.10 µM Y-27632 (purchased from MCE Cat.HY10071) was added to avoid anoikis, and the PDOs were cultured at 37°C with 5% CO₂ for 24 hours;
Day 2:
   1. 200ng/mL IFN gamma was added to the PDO and treated overnight to enhance antigen presentation;
   2. 96-well plates were coated with 5 µg/mL CD28 mAb in PBS (50 µL per well) at 4°C for 24 hours;
   3. 1×10⁷ TILs were plated in a 24-well G-REX, and 150 U/mL IL-2 and 20 µg/mL PD1 mAb were added for overnight treatment;
Day 3:
   1. The PDOs were collected from the 12-well plate. The suspension of organoids and medium was transferred to a 15 mL tube. The total volume was divided into two aliquots: 1/11 of the total volume was used for dissociation into single cells and counting, and 10/11 of the total volume was used for experiments;
   2. 1/11 of the total volume was digested into single cells. The PDOs were resuspend in 500 µL TrypLE and incubated at 37°C for 5-15 minutes. Organoids were checked every few minutes to see if they began to dissociate. When the organoids broke down into single cells, stopping termination and cell count was performed;
   3. 10/11 of the total volume of PDOs was washed twice with PBS to remove the IFN-gamma added in the culture medium;
   4. The organoids were re-suspended at 1×10⁵ single cells/mL in TIL culture medium (medium No.8 in Table 1). PD-1 mAb was added to a concentration of 40 µg/mL and Y-27632 was added to a concentration of 20 µM;
   5. TILs were collected, centrifuged at 800g for 3min, and re-suspended with No.8 medium in Table 1; the density was adjusted to 5×10⁵/mL;
   6. The cells were divided equally into 2 groups and added to the 96-well plate:
      Group 1: 100 µL PDO+100 µL No.8 medium;
      Group 2: 100 µL PDO+100 µL No.8 medium containing 5×10⁴ TILs;

Group 1 and Group 2 each was in triplicates. After incubating at 37°C for 48 hours, the mixed suspension of TIL and PDO in each triplicate well was enzymatically digested to form single-cell suspension, and the tumor cells were stained with the flow cytometric antibody against the intestinal cancer cell marker EpCAM, and the dye kit Zombie NIR^{™}Fixable Viability Kit (purchased from Biolegend) was used to stain the tumor cells to distinguish dead cells from living cells, and flow cytometry detection was performed after staining.

The results are shown in Figure 23. **: p<0.01. After co-culturing with syngeneic TILs for 48 hours, the viability of EpCAM+ tumor cells in the PDO+TIL group was significantly reduced compared with the PDO+TII, medium group. This shows that organoids derived from T006 tumor tissue can be effectively killed by their syngeneic TILs.

### Example 9: Killing effect of T001 tissue sample-derived product TILs on mouse cell line-derived xenograft (CDX) tumor tissue

### Experimental animals

Immunodeficient B-NDG mice (purchased from Biocytogen) were used as experimental animals to establish CDX models.

Experimental design and grouping: as shown in Table 7 as below

**Table 7, Mice dosing regimen and grouping**

| Group | Drug | Animal numbers | Administr ation route | Frequency of administra tion | Administra tion volume (µL) | Time (days) |
|---|---|---|---|---|---|---|
| 1 | PBS | 6 | tail vein | once | 200 | 20-30 |
| 2 | TIL (0.4×10⁷ /animal) | 6 | tail vein | once | 200 | 20-30 |

The TILs were the product TILs derived from the tissue sample T001 prepared in Example 2. Before tail vein injection, the cells were centrifuged and resuspended in PBS to prepare a PBS cell suspension with a cell density of 2×10⁷/mL.

### Animal husbandry

The required amount of B-NDG mice were purchased and housed in an SPF-level experimental animal room for an acclimatization period of 7-10 days.

Environment: Mice will be housed in transparent resin plastic cages in the animal house. Cage litter is autoclaved wood chips and corncob litter, which are replaced periodically. The Animal house is equipped with high-efficiency air filters and maintain at a temperature between 20-26°C (68-79°F) with a relative humidity of 40-70%. Temperature and humidity are monitored and recorded continuously. The illumination condition is 12 hours of daylight lamp illumination and 12 hours of darkness.

Food and drinking water: Experimental mice are allowed to access special mouse food (sterilized by irradiation, SLAC Laboratory Animal, Shanghai, China) and sterilized clean drinking water *ad libitum.*

### Tumor cell inoculation

HGC-27 tumor cells were inoculated subcutaneously in each mouse at right side (2×10⁶ cells per mouse), and the inoculation was in 0.1 mL medium containing 50% matrix to promote tumor growth.

### Animal grouping and administration

When the tumor volume reached ~50 mm³, 12 animals were randomly divided into groups according to the tumor volume with n=6, ensuring that all groups were comparable on the baseline. The grouping day was recorded as P0. During the experiment, the body weight and tumor volume of the animals were measured 3 times a week, and the clinical symptoms of the animals were observed every day. The tumor volume was expressed in mm³ according to the formula: V=0.5×a×b², wherein a and b were the long diameter and short diameter of the tumor, respectively.

### Result

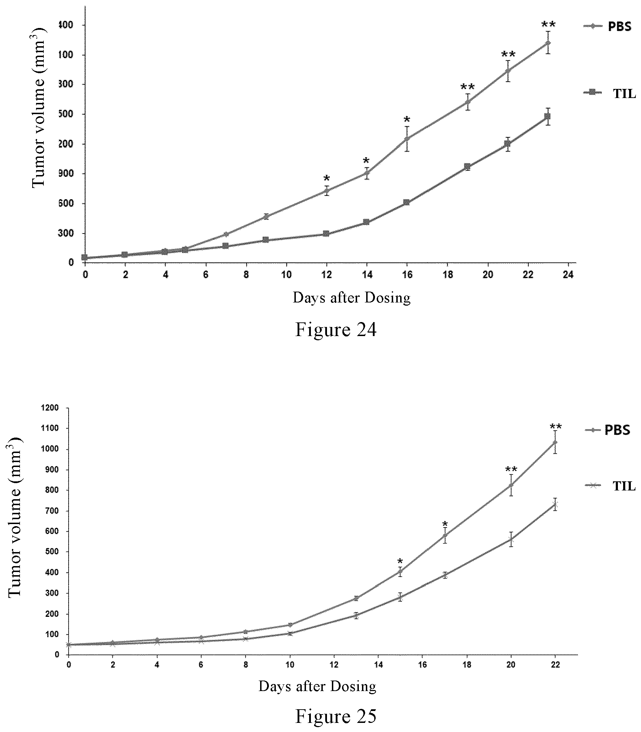

The experimental results are shown in Figure 24. Differences in tumor volume between groups were statistically analyzed by unpaired two-tailed t-test. *: p<0.05, **: p<0.01. The results in Figure 24 show that, as of Day 23 post-dosing, the tumor volume growth of the mice of the TIL-dosing group was significantly inhibited as compared with the control group in which the tumor-bearing mice were injected with PBS, indicating that the product TILs derived from T001 have a significant killing effect on gastric cancer tumors *in vivo.*

### Example 10: Killing effect of product TILs derived from T002 tissue samples on mouse CDX tumor tissue

### Experimental animals

Immunodeficient B-NDG mice (purchased from Biocytogen) were used as experimental animals to establish CDX models.

Experimental design and grouping were shown in Table 8 as below

**Table 8, Mice dosing regimen and grouping**

| Group | Drug | Animal numbers | Administr ation route | Frequency of administrat ion | Administra tion volume (µL) | Time (days) |
|---|---|---|---|---|---|---|
| 1 | PBS | 6 | tail vein | once | 200 | 20-30 |
| 2 | TIL (0.4×10⁷ /animal) | 6 | tail vein | once | 200 | 20-30 |

The TILs were the product TILs derived from the tissue sample T002 prepared in Example 2. Before tail vein injection, the cells were centrifuged and resuspended in PBS to prepare a PBS cell suspension with a cell density of 2×10⁷/mL.

### Tumor cell inoculation

Melanoma cells A375 were inoculated subcutaneously in each mouse at right side (2×10⁶ cells per mouse), and the inoculation was in 0.1 mL medium containing 50% matrix to promote tumor growth.

Animal feeding, grouping and administration of drug were the same as in Example 9,

### Result

The experimental results are shown in Figure 25. Differences in tumor volume between groups were statistically analyzed by unpaired two-tailed t-test. *: p<0.05, **: p<0.01. The results in Figure 25 show that, as of Day 22 post-dosing, the tumor volume growth of the mice in the TIL-dosing group was significantly inhibited as compared with the tumor-bearing mice injected with PBS control group, indicating that the product TILs derived from T002 had a significant killing effect on melanoma *in vivo.*

### Example 11: Killing effect of T008 tissue sample-derived product TILs on syngeneic patient-derived xenograft (PDX) tumor tissue

### Experimental animals

Immunodeficient B-NDG mice (purchased from Biocytogen) were used as experimental animals to establish PDX models.

Experimental design and grouping were shown in Table 9 as below

**Table 9, Mice dosing regimen and grouping**

| Group | Drug | Animal numbers | Administr ation route | Frequency of administra tion | Administra tion volume (µL) | Time (days) |
|---|---|---|---|---|---|---|
| 1 | PBS | 8 | tail vein | once | 200 | 30∼42 |
| 2 | TIL (2X10⁷/ animal) | 8 | tail vein | once | 200 | 30∼42 |

The TILs were the product TILs derived from the tissue sample T008 prepared in Example 2. Before tail vein injection, the cells were centrifuged and re-suspended in PBS to prepare a PBS cell suspension with a cell density of 1×10⁸/mL.

### Animal husbandry

The required amount of B-NDG mice were purchased and housed in an SPF-level experimental animal room for an acclimatization period of 7-10 days.

Environment: Mice will be housed in transparent resin plastic cages in the animal house. Cage litter is autoclaved wood chips and corncob litter, which are replaced periodically. The animal house is equipped with high-efficiency air filters and maintain at a temperature between 20-26°C (68-79°F) with a relative humidity of 40-70%. Temperature and humidity are observed and recorded continuously. The illumination condition is 12 hours of daylight lamp illumination and 12 hours of darkness per day.

Food and drinking water: Experimental mice are allowed to access special mouse food (sterilized by irradiation, SLAC Laboratory Animal, Shanghai, China) and sterilized clean drinking water *ad libitum.*

### Establishment of PDX model

1) Treatment of tumor tissue samples from patients: a portion of T008 tumor tissue was used, and necrotic tissue, adipose tissue and connective tissue, etc. were removed from the tumor tissue in sterile condition. After wash, the tumor tissue was dissociated into a number of 5×5×5mm³ tissue fragments with a scalpel, put into the tumor sample transportation preservation buffer, UW, and the tumor fragments were ready to be inoculated into B-NDG mice;
2) Tumor tissue sample inoculation: a number of B-NDG mice were used. After the skin of the mouse scapula were prepared, the mouse was fixed with a mouse subcutaneous tumor inoculation fixer, disinfected with iodophor, locally anesthetized with lidocaine, and the tumor fragments of 1) was inoculated to the right groin of the mice by the PDX model tumor fragment inoculation trocar. The day of inoculation was recorded as P0, and the tumor was measured twice a week. The formula for calculating tumor volume is: V=0.5×a×b², where a and b are the long and short diameters of the tumor, respectively;
3) PDX tissue passaging: the growth of tumor tissue in each inoculated mouse was observed. When the volume of tumor tissue reached over 300mm³, the mice were anesthetized, the tumor fragments taken out, and cut into 5×5×5mm³ tissue fragments with a scalpel under sterile conditions. Then step 2) was repeated, tissue fragments were inoculated into the right groin of a new mouse, and the next generation of PDX tumor began to grow;
4) Step 3) was repeated, and after 2-3 passages, some PDX tissues in the mice were sampled for tissue section pathological analysis. Upon confirming the human nature of the PDX tissues (not murine), they were used to inoculate 1.5 times the number of mice according to the experimental plan in Table 9 (i.e., 24 mice inoculated with PDX tissue fragments). The tumorigenicity in mice was monitored, and the tumor was measured twice a week until tumors began to form.

### Animal grouping and dosing

When the tumor volume of PDX-inoculated mice reached ~50 mm³, 16 animals with appropriate tumor volume were selected from 24 animals, and randomly grouped according to tumor volume, n=8, to ensure that all groups were comparable at baseline. The grouping day was recorded as D0, and the dosing was carried out according to the scheme in Table 9. During the experiment, the body weight and tumor volume of the animals were measured 3 times a week, and the clinical symptoms of the animals were monitored daily. The tumor volume is expressed in mm³, and the formula used to calculate tumor volume is the same as above.

### Result

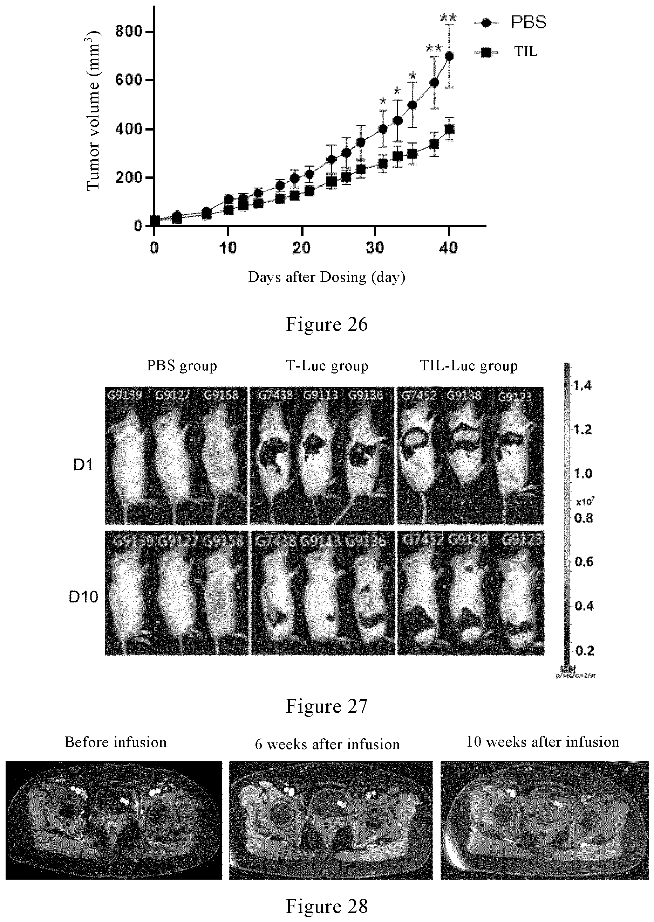

The experimental results are shown in Figure 26. Differences in tumor volume between groups were statistically analyzed by unpaired two-tailed t-test. *: p<0.05, **: p<0.01. The results in Figure 26 show that, as of Day 40 post-dosing, the tumor volume growth of the mice in the TIL-dosing group was significantly inhibited as compared with the control group tumor-bearing mice injected with PBS, indicating that the product TILs derived from T008 had a significant killing effect *in vivo* on its syngeneic PDX tumor tissue.

### Example 12: Detection of tumor tropism of product TILs derived from T007 tissue samples in syngeneic PDX mice

### 1. Preparation of T cells from peripheral blood PBMCs of patients

Peripheral blood mononuclear cells (PBMCs) were isolated from 20 mL of peripheral blood of patients corresponding to T007 tissue samples by Ficoll leukapheresis. PBMCs were subjected to adherent culture for 2-4 hours, and the non-adherent floating cells were initial T cells. The floating cells were collected into a 15ml centrifuge tube, centrifuged at 1200rmp for 3min, the supernatant was discarded, and physiological saline was added. The centrifuge tube was centrifuged at 1200rmp for 3min, the physiological saline was discarded, and this step was repeated. Harvested T cells were re-suspended with X-VIVO 15 containing 200 IU/mL of IL-2 and 5% human AB serum, placed in a cell culture dish pre-coated with CD3 mAb and CD28 mAb, and activated in a 37°C, 5% CO₂ cell incubator. The cell suspension was transferred to a new culture dish for culture, and fresh medium was replaced every 1-2 days until at least 1.0×10⁸ T cells were obtained for later use.

### 2. Preparation of T cells and TILs with integrated luciferase expression.

Lentiviral particles expressing the luciferase gene were purchased from GeneChem, Cat.No. LVCON101. T cells derived from the peripheral blood of the T007 patient prepared in 1 (1×10⁸ cells were used) and product TILs derived from T007 tumor tissue samples prepared in Example 2 (1×10⁸ cells were used) were transduced with luciferase-expressing lentivirus according to the following steps:
Step 1: preparation before infection: the virus was taken from refrigerator and was thawed on ice and diluted 10 times with FBS-free DMEM.
Step 2: infection: T cells and TILs were centrifuged at 800g for 3 minutes, and the cell pellet was re-suspended with serum-free X-VIVO 15 basal medium. Then the corresponding amount of virus was added according to the cell MOI and virus titer, and the calculation formula: virus volume = (MOI × number of cells)/virus titer. Peripheral blood T cells and TILs were transduced at the MOI value = 50. After 8-12 hours of transduction, human AB serum was replenished to a final concentration of 5% v/v and IL-2 was replenished to a final concentration of 200 IU/mL. The cells continued to culture for 24-48 hours.
Step 3: cell culture and passaging: the culture medium can be replenished during culture to maintain cell viability.
Step 4: Confirmation of the transducing effects: 72 hours after transduction, a small amount of cells were taken and added to one of the wells in a 96-well plate, and 20 microliters of salt dilution of the luciferase substrate luciferin were added at the same time. the fluorescence was monitored using the in vivo imaging system.

The results show that fluorescence values could be clearly detected in both T cells and TILs, indicating that the luciferase-expressing lentivirus had been successfully transduced into T cells and TILs of T007, designated as T-Luc cells and TIL-Luc cells, respectively.

### 3. Establishment of PDX model

Referring to the method described in the "Establishment of PDX model" in Example 11, some T007 tumor tissues were taken, and a PDX model of T007 tumor tissue based on immunodeficient B-NDG mice was established. After stably passaging for 2-3 generations, pathological examination was conducted to confirm that the PDX tissues were still of human nature (rather than murine). B-NDG mice were prepared and inoculated with tumor fragments according to the experimental requirements, and the inoculation site of the tumor fragments was the right groin of mice.

### 4. Detection of tumor tropism of TILs in vivo

Experimental design and grouping are shown in Table 10 as below

**Table 10, Mice administration regimen and grouping**

| Group | Drug | Animal number s | Administrati on route | Frequency of administrat ion | Administrati on volume (µL) | Time (days) |
|---|---|---|---|---|---|---|
| 1 | PBS | 3 | tail vein | once | 200 | 10 |
| 2 | T-Luc cell (2x10⁷/ animal) | 3 | tail vein | once | 200 | 10 |
| 3 | TIL-Luc cell (2x10⁷/ animal) | 3 | tail vein | once | 200 | 10 |

The T-Luc cells and TIL-Luc cells prepared in 2 were centrifuged, and the cell pellet was re suspended with PBS to prepare a cell suspension with a cell density of 1×10⁸/mL. The three groups of T007 PDX tumor-bearing mice were dosed according to the experimental scheme in Table 10. Day 1 (D1) and Day 10 (D10) after dosing, the three groups of mice were injected with the luciferase substrate luciferin and then subjected to live fluorescence imaging on an *in vivo* imaging system to observe the *in vivo* distribution of the T-Luc cells and the TII,-Luc cells in tumor-bearing mice.

The results are shown in Figure 27. Figure 27 shows that one day after tail vein infusion, both peripheral blood-derived T cells and TILs were significantly enriched in lungs as a result of blood circulation. Ten days after infusion, fluorescence *in vivo* imaging shows that both T cells and TILs demonstrated a tumor tropic tendency. But when compared with peripheral blood-derived T cells, the tumor tropism of TILs was more pronounced.

### Example 13: Clinical infusion of TIL and proliferation of TILs in vivo

In order to validate the clinical safety and effectiveness of the TIL obtained by the culture method of the present application, a clinical trial of TIL infusion therapy for solid tumors was carried out. This clinical trial had been approved by the Ethics Committee of Shanghai Tenth People's Hospital. For details, please refer to http://www.chictr.org.cn/, registration number: ChiCTR2100044705; or refer to www.clinicaltrials.gov, registration number: NCT04766320. In addition, this trial also involved cooperation with Tong Ren Hospital Shanghai Jiaotong University School of Medicine and the Second Affiliated Hospital of Soochow University, www.clinicaltrials.gov registration numbers being NCT04967833 and NCT04943913, respectively.

### Study plan

### 1. Inclusion and exclusion criteria and grouping methodology of test subjects

Inclusion criteria:
1) 18 years old ≤ age ≤ 75 years old;
2) Patients pathologically diagnosed with primary/recurrence/metastasis of malignant tumors;
3) Life expectancy > 3 months;
4) Karnofsky ≥ 60% or ECOG scoring 0-2 points;
5) The subject currently failed in standard treatment, or had no standard treatment plan;
6) The subject must have a tumor area or malignant effusion suitable for puncture or resection of living tissue from which TILs can be isolated;
7) There was at least one assessable tumor lesion;
8) White blood cell count ≥ 2.5×10⁹/L, absolute neutrophil count ≥1.5×10⁹/L, platelet count ≥100×10⁹; hemoglobin ≥90g/L;
9) Serum creatinine clearance rate was 50 mL/min or higher; creatinine ≤ 1.5× ULN; ALT (alanine aminotransferase)/AST (aspartate aminotransferase) < 3 times of the normal group, ALT/AST of those with liver metastases < 5 times of the normal group; Total bilirubin≤1.5×ULN;
10) Activated partial thromboplastin time (APTT) ≤ 1.5 × ULN; International Normalized Ratio (INR) ≤ 1.5×ULN;
11) Sufficient venous access, no absolute or relative contraindications for surgery or puncture;
12) Subjects with reproductive potential must be willing to implement the approved high-efficiency contraceptive method at the time of signing informed consent, and continue to implement it within one year after the completion of the lymphodepletion regimen;
13) Any treatment for malignant tumors, including radiotherapy, chemotherapy and biologicals must be stopped 28 days before the TILs are obtained;
14)The subject has sufficient comprehensive capability and voluntarily signs the informed consent;
15) The subject has good compliance and be able to adhere to the study visit plan and other protocol requirements.

### Exclusion criteria:

1) Need to use glucocorticoid therapy, with a daily dose of prednisone greater than 15 mg (or equivalent dose of hormone);
2) Require immunosuppressive therapy against autoimmune diseases;
3) Serum creatinine>1.5×ULN; serum glutamic-oxaloacetic transaminase (SGOT)>5 times of the upper limit of normal value; total bilirubin>1.5×ULN;
4) Forced expiratory volume in the 1st second (FEV1) < 2L, diffusing capacity of lung carbon monoxide (DLCO) (corrected) < 40%
5) Has any one of the following definitions of significant cardiovascular abnormality: New York Heart Association (NYHA) class III or IV congestive heart failure, clinically significant hypotension, clinically uncontrolled hypertension, uncontrolled symptomatic coronary arteries disease, or ejection fraction <35%; severe cardiac rhythm or conduction abnormalities, such as ventricular arrhythmia requiring clinical intervention, degree II-III atrioventricular block, etc.;
6) Has human immunodeficiency virus (HIV) infection or HIV antibody test positive, active hepatitis B or hepatitis C virus infection (HBsAg positive and/or anti-HCV positive), syphilis infection or treponema pallidum antibody positive;
7) Severe physical or mental illness;
8) Imaging evidence of positive blood culture or infection;
9) Receiving other medicaments, or other biologicals, chemotherapy or radiotherapy currently or within one month;
10) Has a history of allergic reactions to chemical or biological composition compounds similar to cell therapy;
11) Has received immunotherapy and manifests irAE grade ≥ 3;
12) The adverse reactions of the previous anti-tumor therapy has not recovered to CTCAE version 5.0 grade evaluation ≤ grade 1 (except for toxicities that the investigator judges to bear no safety risk such as alopecia);
13) Pregnant or lactating women;
14) The investigator believes that the subject has a history of other serious systemic diseases, or is not suitable for participating in this clinical study for other reasons.

### Evaluation Criteria for Efficacy

### Main efficacy indicators:

1) Objective response rate (ORR);
2) Disease Control Rate (DCR);
3) Duration of response (DOR);
4) Progression-free survival (PFS);
5) Overall survival (OS).

### Secondary efficacy indicators:

1) Clinical efficacy evaluation: complete remission (CR), partial remission (PR), Stable disease (SD), Progressive disease (PD), etc.;
2) Changes in quality of life before and after treatment.
Efficacy was defined as observable ORR according to the criteria of RECIST1.1.

### 3. Records of adverse events and countermeasures

### 3.1 Definition of Adverse Events

Adverse events (AE): Adverse medical events that occurred from the time of the subject signed the informed consent and was enrolled in the trial, to the last medical follow-up, which can be manifested as symptoms and signs, diseases or abnormal laboratory tests, but was not necessarily related to a causal relationship with the treatment or experimental medicament.

A new condition or deterioration of a pre-existing condition was considered as an AE. Stable chronic diseases that were present before entry into the study and did not worsen during the study period, such as arthritis, were not considered as an AE. Abnormal laboratory test results, clinical symptoms or signs judged by the investigator to be clinically significant were considered as an AE.

Serious Adverse Event (SAE): Refers to adverse events that meet one or more of the following conditions during the trial: (1) leading to death; (2) life-threatening, which means that serious patients were at risk of immediate death, not hypothetical death may occur in severe cases in the future; (3) leading to hospitalization or prolonged hospitalization; (4) permanent or significant loss of function; (5) teratogenic, birth defects; (6) other important medical events.

### 3.2. Criteria for judging the severity of adverse events

The severity of all adverse events that occurred during the study was evaluated according to NCI-CTCAE version 5.0 and categorized into grades 1 to 5:
Grade 1: Mild; asymptomatic or mild; only clinical or diagnostic findings; no treatment required.
Grade 2: Moderate; minor, topical, or noninvasive treatment required; age-appropriate limitation in instrumental activities of daily living.
Grade 3: Severe or medically significant but not immediately life-threatening; resulting in hospitalization or prolonged hospitalization; disabling; limiting personal activities of daily life.
Grade 4: Life-threatening; urgent treatment required.
Grade 5: AE-related death.

### 4. Pretreatment before infusion

Before the infusion of TILs, the patient received a lymphocyte preconditioning regimen (hydroxychloroquine 600-800 mg×1 day, cyclophosphamide 20-25 mg/kg/d×3 days), the drugs, dosages and number of days (dosing frequency) used in the preconditioning regimen will be adjusted by the investigator according to the actual condition of the patient.

The day of infusion was defined as Day 0. Before the infusion of TILs, the investigator needs to perform the following chemotherapy pretreatment on the patient (the patient will experience leukopenia after pretreatment, and infection should be prevented, such as avoiding unnecessary personnel mobility in the ward, and patients wearing masks, etc.):
Day -5: intravenous injection of cyclophosphamide (Cy) 20-25 mg/kg + oral administration of hydroxychloroquine (HCQ) 600-800 mg
Day -4 day: intravenous injection of cyclophosphamide 20-25 mg/kg
Day -3 day: intravenous injection of cyclophosphamide 20-25 mg/kg
Day -2 and Day -1: the patient resting
Day 0, infusion of TILs.

### 5. TIL infusion and therapeutic effect evaluation

The patients were infused on Day 0 after the pretreatment prior to infusion in the aforementioned 4 was completed. Each patient received a single infusion, the dose of one infusion was 2×10¹⁰-5×10¹⁰ TILs, the cell density was 1.0×10⁸/mL~2.0×10⁸/mL, and the volume of the infused cell preparation was 200mL-600mL. The patients were infused with TILs on Day 0, and no cytokines were administered post-infusion. After infusion, the patients were required to stay in hospital for 5-8 days for observation, and patients' conditions after infusion were monitored and recorded. With the patient's informed consent, peripheral blood was drawn from the patient at different time points to detect the composition and changes of PBMCs, and the efficacy was evaluated by imaging 1-3 months after the infusion.

### 6. Results of TIL clinical infusion

A total of 16 subjects had been enrolled so far. Table 11 as shown below records the basic conditions of 11 patients who had undergone clinical efficacy evaluation (evaluation time varies within 1-3 months after infusion):

**Table 11. Comprehensive information on clinical infusion of TILs to tumor patients**

| **Serial number** | **Tumor type** | **Number of infused TILs** | **Pretreatment prior to infusion** | **Clinical efficacy** |
|---|---|---|---|---|
| T009 | cervical cancer | 2.0×10¹⁰ | Cy 20mg/kg+ HCQ 600mg | CR |
| T010 | Ovarian metastasis of gastric cancer | 5.0×10¹⁰ | Cy 25mg/kg+ HCQ 600mg | SD |
| T011 | glioma | 2.5×10¹⁰ | Cy 25mg/kg+ HCQ 600mg | PD |
| T007 | ovarian cancer | 2.0×10¹⁰ | Cy 20mg/kg+ HCQ 800mg | SD |
| T012 | cervical cancer | 2.0×10¹⁰ | Cy 20mg/kg+ HCQ 600mg | PR |
| T003 | cervical cancer | 3.5×10¹⁰ | Cy 25mg/kg+ HCQ 600mg | SD |
| T013 | melanoma | 2.0×10¹¹ | Cy 20mg/kg+ HCQ 700mg | SD |
| T014 | cervical cancer | 4.5×10¹⁰ | Cy 20mg/kg+ HCQ 600mg | SD |
| T015 | endometrial cancer | 4.0×10¹⁰ | Cy 25mg/kg+ HCQ 600mg | SD |
| T016 | colorectal cancer | 2.1×10¹⁰ | Cy 20mg/kg+ HCQ 600mg | SD |
| T017 | ovarian cancer | 3.0×10¹⁰ | Cy 25mg/kg+ HCQ 600mg | PR |

Among the 16 subjects, adverse events included grade 1 chills in 5 cases (31.3%), grade 1 fever in 7 cases (43.8%), and grade 2 fever in 6 cases (37.5%), and all symptoms disappeared over time. No other adverse events were observed.

Patient T017 was evaluated as partial response (PR) based on the imaging evaluation that the tumor shrank significantly. Peripheral blood was drawn from the patient before and after the infusion of TILs, the levels of some tumor markers were detected, and the comparison results are shown in Table 12 as below.

**Table 12. Comparison of the values of some tumor markers before and after infusion of TILs into T017 patients**

| | Carcinoem bryonic antigen(ng /mL) | Alpha-fetoprotein (ng/mL) | CA15 3 < U/ mL) | CA12 5 < U/ mL) | CA19 9 < U/ mL) | human epididy mis protein 4 (pmol /mL) | postmeno pausal ROMA value | premeno pausal ROMA value |
|---|---|---|---|---|---|---|---|---|
| norm al range | <5.2 | <7.0 | <26.4 | <35 | <27 | <82.9 | <29.9% | <43.3% |
| Befor e infusi on | 1.07 | 3.8 | 143 | 60.1 | 12.43 | 124 | 48 | 43.3 |
| 30 days after infusi on | 0.71 | 3.81 | 104 | 30.8 | 14.58 | 87.1 | 28.2 | 24 |

The results in Table 12 show that before infusion in patient T017, the above tumor markers significantly exceeded the normal range,, except for alpha-fetoprotein and CA199 which were within the normal range. The above tumor markers in the peripheral blood of the patient were tested again 30 days after TIL infusion, and it was found that several factors that exceeded the normal standard had returned to or remained within the normal range, except for human epididymis protein 4 which was slightly higher than the normal range.

Figure 28 shows the imaging evaluation results of patient T009 after infusion of TILs. MRI results shows that the size of the tumor lesion before infusion was about 3 cm in diameter. The patient's tumor lesions had shrunk significantly by the 6th week after the TIL infusion (when it was judged as partial response, PR) compared with that before the infusion. After 10 weeks post-infusion, the tumor was no longer detectable by imaging, and it was judged as complete remission (CR) according to the RECIST 1.1 guideline standard.

Blood samples were collected and blood routine tests were performed at different time points from before infusion to 120 days after infusion. With patient T009's informed consent, the peripheral blood of the patient was extracted, and PBMCs were isolated by Ficoll density gradient centrifugation on the day of infusion before infusion was performed (D0) and on Day 2, Day 7, Day 11, Day 13, Day 17, Day 24, Day 29, Day 31, Day 43, Day 49, Day 53 and , Day 59 after infusion (D2, D7, D11, D13, D17, D24, D29, D31, D43, D49, D53 and D59). Then cells were lysed by Trizol (Life Technologies), RNA was extracted from the cell lysate, and RNA sequencing of the CDR3 region of the TCR beta chain was performed (TCR-seq, Hangzhou ImmuQuad Biotech Co., Ltd.). At the same time, T009 product TILs used for clinical infusion for patients were also subjected to TCR-seq of the TCR beta chain CDR3 region. TCR beta chain CDR3 libraries of peripheral blood PBMCs and infused product TILs were obtained at different time points. By comparing the TCR beta chain CDR3 libraries of D0, D2, D7, D11, D13, D17, D24, D29, D31, D43, D49, D53, D59 obtained by TCR-seq with the TCR beta chain CDR3 libraries of product TILs, respectively, the TCRs found in the shared portion were the TCRs corresponding to the clonotypes of the TILs infused into the peripheral blood of patients at different time points before and after TIL infusion (D2, D7, D11, D13, D17, D24, D29, D31, D43, D49, D53 and D59).

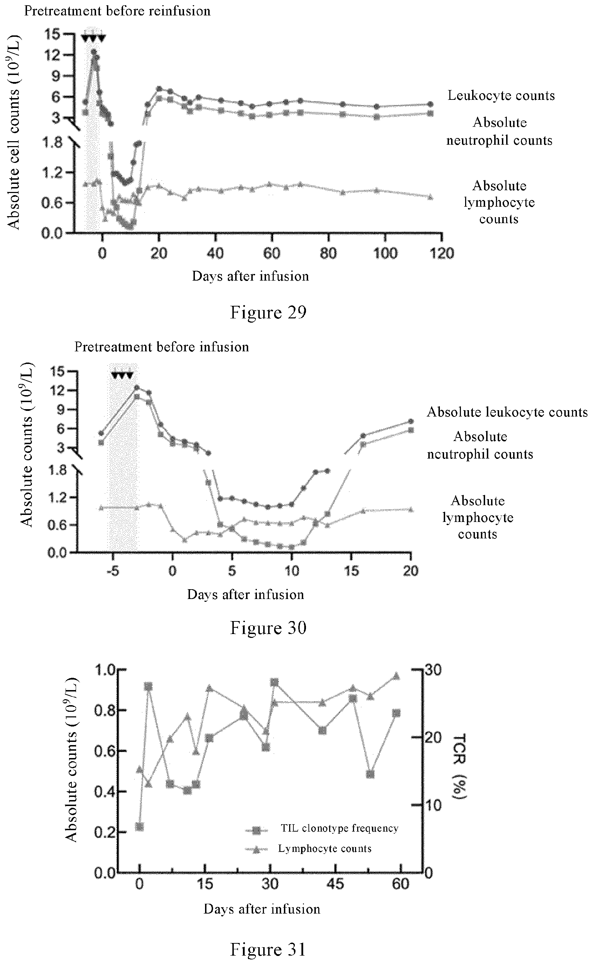

Figure 29 shows the tendency curve of the number of white blood cells, neutrophils and lymphocytes in the peripheral blood of the patients from before the infusion to nearly 120 days after the infusion. Fig. 30 is a partial enlarged view of Fig.29 from before infusion to 20 days after infusion. It can be seen from Figures 29 and 30 that the absolute counts of the above three types of cells all decreased significantly from the time of pretreatment before infusion to the day of infusion (D0). From D0, the absolute counts of leukocytes and neutrophils continued to decrease, and both leukocytes and neutrophils dropped to the lowest level around D10, which was consistent with published reports (M E Gershwin, E J Goetzl, A D Steinberg Cyclophosphamide: use in practice Ann Intern Med.1974 Apr;80 (4):531-40). However, the lymphocyte count began to rise significantly after D0, and this upward trend was particularly pronounced about one week after D0, which was in significant contrast to the downward trend of the absolute counts of white blood cells and neutrophils, indicating that lymphocytes proliferated significantly *in vivo* after the infusion of TILs.

Figure 31 shows the superimposed tendency of the TCR clonotype frequency and the total number of lymphocytes in TILs infused in the peripheral blood of patients over time. It could be clearly observed in Figure 31 that on the day of infusion, the frequency of TCR clonotypes of infused TILs reached its peak instantaneously, then dropped to a low value, and then continued to rise, and the results were highly consistent with the rising trend of the total number of lymphocytes until 30 days after infusion, indicating that the significant increase in the number of lymphocytes in the peripheral blood of patients after infusion was highly correlated with the proliferation of TCR clonotypes of infused TIL.

Combining the results in Figures 29, 30 and 31, it could be seen that the product TILs of the present invention can actively and effectively proliferate in the human body when the patient was infused after pretreatment with low doses of cyclophosphamide + hydroxychloroquine.

### Comparative example

According to the process 2A in the method described in Example 19 (refer to preparation method of Gen2 in the application) on page 142 of the specification of Chinese patent application CN110785486A in conjunction with the technical solution of adding IL-2, IL-15 and IL-21 in the pre-REP culture stage (corresponding to the seed cell culture stage of the present application) disclosed in Example 27 on page 177 of the specification of CN110785486A , product TILs were prepared from T004, T009, T013 and T014 tissue samples in the Table 4 of Example 2, the preparation method was described in Examples 19 and 27 and all other related examples of CN110785486A , and CN110785486A were incorporated herein by reference in its entirety. The feeder cells required in the reference TIL production method were from healthy human PBMCs.

The final product TILs prepared by the preparation method described in CN110785486A (hereinafter referred to as "comparison method") and the preparation method of the present invention (hereinafter referred to as "present method") were respectively tested and compared for the following indicators:
1) Cell count and survival rate;
2) Proportion of CD45+ cells in total cells;
3) Proportion of CD3+ cells in CD45+ cells;
4) Proportions of CD4+ cells and CD8+ cells in CD3+ cells;
5) Proportions of memory T cells in CD3+ cells;
6) IFN-gamma secretion level;
7) The level of T cell exhaustion;
8) Proportion of regulatory T cells (Treg) in CD3+ cells;

The proportions of memory T cells, exhausted T cells and activated T cells were detected by flow cytometry, specifically, Tcm was determined by detecting CD45RO⁺CCR7⁺ cells; Tern was determined by detecting CD45RO⁺CCR7⁻ cells; exhausted T cells were determined by detecting Lag3⁺ cells; The detection of IFN-gamma secretion level was the same as the method described in Example 2 of the present application; Treg was determined by detecting CD4⁺CD25⁺Foxp3⁺ cells.

The results are shown in Table 13.

**Table 13, Comparison of indicators of product TILs prepared by the comparison method and present method**

| | T004 | | T009 | | T013 | | T014 | |
|---|---|---|---|---|---|---|---|---|
| | Comp arison metho d | present method | Comp arison metho d | present method | Comp arison metho d | present method | Compar ison method | presen t metho d |
| Cell number(x 10¹¹) | 2.86 | 2.32 | 1.58 | 2.19 | 3.27 | 4.30 | 1.51 | 1.39 |
| Viability (%) | 95.43 | 92.59 | 93.88 | 97.03 | 97.61 | 98.96 | 96.88 | 92.94 |
| CD45⁺ ratio(%) | 99.85 | 100 | 99.99 | 99.99 | 100 | 99.91 | 99.73 | 99.99 |
| CD3⁺/D45⁺ ratio(%) | 90.63 | 99.70 | 91.23 | 87.75 | 99.14 | 99.76 | 98.96 | 94.06 |
| CD4⁺/CD3⁺ ratio(%) | 46.37 | 0.54 | 52.64 | 25.97 | 5.16 | 0.76 | 26.24 | 0.53 |
| CD8⁺/CD3⁺ ratio(%) | 50.17 | 56.48 | 46.03 | 34.14 | 94.02 | 97.49 | 71.05 | 97.34 |
| Tcm ratio(%) | 10.45 | 8.92 | 6.33 | 9.12 | 1.22 | 5.67 | 5.22 | 0.12 |
| Tern ratio(%) | 88.19 | 91.02 | 91.75 | 90.54 | 96.31 | 90.94 | 92.93 | 99.32 |
| IFN-gamma secretion (pg/mL) | 11257 .71 | 10039. 71 | 9985. 16 | 10213. 74 | 9977. 44 | 11203. 06 | 10541. 98 | 9811. 08 |
| Proportion of exhausted T cells in CD3⁺ (%) | 40.37 | 22.9 | 18.93 | 5.37 | 26.89 | 4.68 | 49.91 | 33.03 |
| Proportion of Treg in CD3+ (%) | 2.43 | N/A | 5.28 | 0.74 | 3.02 | N/A | 5.17 | N/A |

The results in Table 13 show that the product TILs obtained through culture by the comparative method were relatively close to the product TILs obtained through culture by the present method in terms of cell number, viability, CD45⁺ cell proportion and CD3⁺/CD45⁺ proportion. The proportions of CD8⁺ cells in CD3⁺ cells in the product TILs obtained through culture by the two methods were close, and the proportions of CD4⁺ cells in the TILs obtained through culture by the comparative method was relatively higher, while some cells in the TILs obtained through culture by this method were CD4⁻CD8⁻CD3⁺ T cells, that is, double-negative T cells. For the memory T cell phenotype of TILs obtained through culture by these two methods, the highest ratio of Tcm to the total number of T cells was both about 10%, and the IFN-gamma secretion levels of the two TILs were also relatively close. However, compared with the TILs obtained through culture by the comparative method, the product TILs obtained through culture in the four tissue samples by the present method had significantly lower proportions of exhausted T cells and Tregs, showing that the TILs obtained through culture by the method had greater potential advantages in internal and external activation, proliferation and killing effects.

The embodiments of the present application have been described in detail above, but the present application is not limited to the specific details in the above-mentioned embodiments, within the scope of the technical concept of the present application, various simple modifications could be made to the technical solutions of the present application, these simple modifications are all belong to the protection scope of this application. In addition, it should be noted that the various specific technical features described in the above specific embodiments can be combined in any suitable way if there is no contradiction, and the combination method will not be described separately to avoid unnecessary repetition. In addition, any combination of various implementations of the present application can also be made, as long as they do not violate the idea of the present application, they should also be regarded as the content disclosed in the present application.

## Claims

1. A culture composition for expanding TILs, wherein, the culture composition comprises IL-2, IL-7, IL-15 and an immune checkpoint antibody or antigen-binding fragments thereof, preferably, the immune checkpoint antibody includes any one or more selected from the group consisting of a PD-1 antibody, a CTLA-4 antibody, a LAG-3 antibody, a TIM-3 antibody, a TIGIT antibody and a BTLA antibody,
preferably, the culture composition further includes any one, two, three or four selected from the group consisting of IL-21, IL-12, GM-CSF, and small molecules that increase the proportion of memory T cells,
preferably, the culture composition further includes any one or more selected from the group consisting of a CD3 antibody, a CD28 antibody, a CD137 antibody, a OX40 antibody, a GITR antibody, a ICOS antibody, a CD206 antibody and a CD40 antibody,
preferably, the CD3 antibody, CD28 antibody, CD137 antibody, OX40 antibody, GITR antibody, ICOS antibody, CD206 antibody, and CD40 antibody are activating antibodies,
preferably, the culture composition further includes autologous platelets or allogeneic platelets.

2. The culture composition according to claim 1, wherein,
the one or more antibodies are coupled to a matrix, and/or
the matrix is a solid matrix, and the solid matrix includes any one or more selected from a microsphere, a well-plate, a dish, a cell culture flasks and a cell culture bag, and/or
the small molecule that increases the proportion of memory T cells is TWS119, and/or
the culture composition comprises IL-2, IL-7, IL-15, a PD-1 antibody, IL-21, IL-12, a CD3 antibody, a CD28 antibody and GM-CSF, and/or
the culture composition comprises IL-2, IL-7, IL-15, a PD-1 antibody, IL-21, IL-12, a CD3 antibody, a CD28 antibody, a CD137 antibody and a GITR antibody, and/or
the culture composition comprises IL-2, IL-7, IL-15, a PD-1 antibody, a CD3 antibody and a CD28 antibody, preferably, the CD3 antibody and CD28 antibody are coupled to a matrix, and/or
the culture composition comprises IL-2, IL-7, IL-15, a PD-1 antibody, IL-21, IL-12, a CD3 antibody, a CD28 antibody and GM-CSF, preferably, the CD3 antibody and CD28 antibody are coupled to a matrix, and/or
the culture composition comprises IL-2, IL-7, IL-15, a PD-1 antibody, a CD3 antibody, a CD28 antibody and a CD137 antibody, preferably, the CD3 antibody, CD137 antibody and CD28 antibody are coupled to a matrix, and/or
the culture composition comprises IL-2, IL-7, IL-15, a PD-1 antibody, a GITR antibody, a CD3 antibody, a CD28 antibody, TWS119 and a CD137 antibody, preferably, the CD3 antibody, CD137 antibody and CD28 antibody are coupled to a matrix, and/or
the culture composition comprises IL-2, IL-7, IL-15, a PD-1 antibody, IL-21, IL-12, a CD3 antibody, a CD28 antibody and GM-CSF, preferably, the CD3 antibody and CD28 antibody are coupled to a matrix, and/or
the culture composition comprises IL-2, IL-7, IL-15, a LAG3 antibody, IL-21, IL-12, a CD3 antibody, a CD28 antibody, a CD137 antibody and a GITR antibody, preferably, the CD3 antibody, CD137 antibody and CD28 antibody are coupled to a matrix, and/or
the culture composition comprises IL-2, IL-7, IL-15, a PD-1 antibody, a TIGIT antibody, a CD3 antibody, a CD28 antibody, a CD137 antibody, a CD40 antibody, a OX-40 antibody and autologous platelets, preferably, the CD3 antibody, CD137 antibody and CD28 antibody are coupled to a matrix, and/or
the culture composition comprises IL-2, IL-7, IL-15, a PD-1 antibody, a CTLA-4 antibody, a CD3 antibody, a CD28 antibody, a CD137 antibody, a GITR antibody, GM-CSF, TWS119 and allogeneic platelets, preferably, the CD3 antibody and CD28 antibody are coupled to a matrix.

3. The culture composition according to claim 1, wherein,
the culture composition comprises components: 200-6000 IU/mL of IL-2, 5-100 ng/mL of IL-7, 5-100 ng/mL of IL-15 and 3-100 µg/mL of PD-1 antibodies, or the proportional concentration of these components;
the culture composition comprises components: 200-6000 IU/mL of IL-2, 5-100 ng/mL of IL-7, 5-100 ng/mL of IL-15 and 1-100 µg/mL of PD-1 antibodies, or the proportional concentration of these components;
the culture composition comprises components: 200-6000 IU/mL of IL-2, 5-100 ng/mL of IL-7, 5-100 ng/mL of IL-15, 3-100 µg/mL of PD-1 antibodies, 5-100 ng/mL of IL-21, 5-100 ng/mL of IL-12, 1-10 µg/mL of CD3 antibodies, 1-10 µg/mL of CD28 antibodies and 200-5000 U/mL of GM-CSF, or the proportional concentration of these components;
the culture composition comprises components: 200-6000 IU/mL of IL-2, 5-100 ng/mL of IL-7, 5-100 ng/mL of IL-15, 3-100 µg/mL of PD-1 antibodies, 5-100 ng/mL of IL-21, 5-100 ng/mL of IL-12, 1-10 µg/mL of CD3 antibodies, 0.5-10 µg/mL of CD28 antibodies and 200-5000 U/mL of GM-CSF, or the proportional concentration of these components;
the culture composition comprises components: 200-6000 IU/mL of IL-2, 5-100 ng/mL of IL-7, 5-100 ng/mL of IL-15, 3-100 µg/mL of PD-1 antibodies, 5-100 ng/mL of IL-21, 5-100 ng/mL of IL-12, 1-10 µg/mL of CD3 antibodies, 1-10 µg/mL of CD28 antibodies, 1-100 µg/mL of CD137 antibodies and 1-100 µg/mL of GITR antibodies, or the proportional concentration of these components;
the culture composition comprises components: 200-6000 IU/mL of IL-2, 5-100 ng/mL of IL-7, 5-100 ng/mL of IL-15, 1-100 µg/mL of PD-1 antibodies, 5-100 ng/mL of IL-21, 5-100 ng/mL of IL-12, 0.5-10 µg/mL of CD3 antibodies, 1-10 µg/mL of CD28 antibodies, 1-100 µg/mL of CD137 antibodies and 1-100 µg/mL of GITR antibodies, or the proportional concentration of these components;
the culture composition comprises component: 200-6000 IU/mL of IL-2, 5-100 ng/mL of IL-7, 5-100 ng/mL of IL-15, 3-100 µg/mL of PD-1 antibodies, 1-10 µg/mL of CD3 antibodies, 1-10 µg/mL of CD28 antibodies, 1-100 µg/mL of CD137 antibodies, 1-100 µg/mL of GITR antibodies, 200-5000 U/mL of GM-CSF and 1-500 µM of TWS119, or the proportional concentration of these components;
the culture composition comprises components: 200-500 IU/mL of IL-2, 5-50 ng/mL of IL-7, 5-50 ng/mL of IL-15, 1-50 µg/mL of PD-1 antibodies, CD3 antibodies coupled to a matrix, CD28 antibodies coupled to a matrix and 200-1000 U/mL of GM-CSF, or the proportional concentration of these components;
the culture composition comprises components: 200-6000 IU/mL of IL-2, 5-100 ng/mL of IL-7, 5-100 ng/mL of IL-15, 1-100 µg/mL of PD-1 antibodies, 5-100ng/mL of IL-21, 5-100ng/mL of IL-12, CD3 antibodies coupled to a matrix, CD28 antibodies coupled to a matrix, 1-100 µg/mL of CD137 antibodies and 1-100 µg/mL of GITR antibodies, or the proportional concentration of these components;
the culture composition comprises component: 200-6000 IU/mL of IL-2, 5-100ng/mL of IL-7, 5-100ng/mL of IL-15, 1-100 µg/mL of PD-1 antibodies, CD3 antibodies coupled to a matrix, CD28 antibodies coupled to a matrix and CD137 antibodies coupled to a matrix, or the proportional concentration of these components;
the culture composition comprises components: 200-6000 IU/mL of IL-2, 5-100 ng/mL of IL-7, 5-100 ng/mL of IL-15, 1-100 µg/mL of PD-1 antibodies, CD3 antibodies coupled to a matrix, CD28 antibodies coupled to a matrix, CD137 antibodies coupled to a matrix, 1-100 µg/mL of GITR antibody and 1-500 µM of TWS119, or the proportional concentration of these components;
the culture composition comprises component: 200-6000 IU/mL of IL-2, 5-100ng/mL of IL-7, 5-100ng/mL of IL-15, 1-100 µg/mL of PD-1 antibody, CD3 antibodies coupled to a matrix, CD28 antibodies coupled to a matrix, CD137 antibodies coupled to a matrix, 1-100 µg/mL of TIGIT antibodies and 200-5000 U/mL of GM-CSF, or the proportional concentration of these components;
the culture composition comprises components: 200-6000 IU/mL of IL-2, 5-100ng/mL of IL-7, 5-100ng/mL of IL-15, 1-100 µg/mL of LAG-3 antibodies, 5-100ng/mL of IL-21, 5-100ng/mL of IL-12, 0.5-10 µg/mL of CD3 antibodies, 0.5-10 µg/mL of CD28 antibodies, 1-100 µg/mL ofCD137 antibodies and 1-100 µg/mL of GITR antibodies, or the proportional concentration of these components;
the culture composition comprises components: 200-6000 IU/mL of IL-2, 5-100 ng/mL of IL-7, 5-100 ng/mL of IL-15, 1-100 µg/mL of PD-1 antibodies, CD3 antibodies coupled to a matrix, CD28 antibodies coupled to a matrix, CD137 antibodies coupled to a matrix, 1-100 µg/mL of TIGIT antibodies, 1-100 µg/mL of CD40 antibodies, 1-100 µg/mL of OX-40 antibodies and 1×10⁸-5×10⁸/mL of autologous platelets, or the proportional concentration of these components; or
the culture composition comprises component: 200-6000 IU/mL of IL-2, 5-100 ng/mL of IL-7, 5-100 ng/mL of IL-15, 1-100 µg/mL of PD-1 antibodies, 1-100 µg/mL of CTLA-4 antibodies, CD3 antibodies coupled to a matrix, CD28 antibodies coupled to a matrix, 1-100 µg/mL of CD137 antibodies, 1-100 µg/mL of GITR antibodies, 200-5000 U/mL of GM-CSF, 1×10⁸-5×10⁸/mL of allogeneic platelets, or the proportional concentration of these components.

4. A TIL cell expansion medium, comprising serum and/or platelets, a basal medium, and the culture composition for expanding TILs according to any one of claims 1-3,
preferably, the culture composition has one or more features selected from:
the basal medium is selected from the group consisting of AIM-V, X-VIVO, DMEM, RPMI1640, OpTmizer^{™}, and FUJIFILM Irvin MHM-C,
the serum is selected from the group consisting of human AB serum, subject's own serum or animal-derived serum,
the concentration of serum is 1-10%,
the platelets are allogeneic platelets or subject's own platelets,
the platelet density is 0.5×10⁸-1.0×10⁹/mL
the concentration of IL-2 is 200-6000 IU/mL,
the concentration of IL-7 is 5-100 ng/mL,
the concentration of IL-15 is 5-100 ng/mL,
the concentration of PD-1 antibodies is 1-100 µg/mL,
the concentration of IL-21 is 5-100 ng/mL,
the concentration of IL-12 is 5-100 ng/mL,
the concentration of CD3 antibodies is 0.5-10 µg/mL,
the concentration of CD28 antibodies is 0.5-10 µg/mL,
the concentration of CD137 antibodies is 1-100 µg/mL,
the concentration of GITR antibodies is 1-100 µg/mL,
the concentration of GM-CSF is 200-5000 U/mL,
the concentration of TWS119 is 1-500 µM.

5. Use of the culture composition according to any one of claims 1-3 or the medium according to claim 4 in the manufacture of a medicament containing TILs,
preferably, the medicament is for the treatment of cancer,
more preferably, the cancer is selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a brain cancer, a glioma, a pancreatic cancer, a bladder cancer, a prostate cancer, a renal cancer, an osteosarcoma.

6. A method for expanding a population of TIL cell, comprising incubating the population of TIL cell with the medium according to claim 4,
preferably, said incubation lasts at least 5 days, and/or said incubation lasts at most 20 days.

7. A method for preparing a population of TIL cell, comprising the following steps:
(1) incubating a tumor cell-containing tissue with a TIL seed cell culture medium to obtain the first population of TIL cell,
(2) incubating the first population of TILs with the medium described in claim 4 to obtain the second population of TIL cell,
preferably, the method has one or more features selected from:
a) the tumor tissue is pretreated, preferably, the pretreatment includes fragmentation and/or dissociation of the tumor tissue,
b) said tumor cell-containing tissue is a tumor tissue or body fluid of a subject having cancer,
c) the TIL seed cell culture medium described in step (1) includes any one of the combinations of the following components: 1) IL-2, IL-6, IL-21, IFN-gamma, a TIGIT antibody, a PD-1 antibody, TNF-alpha, serum, dual antibiotics and basal medium; 2) IL-2, IL-4, IL-10, IL-21, CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, a PD-1 antibody, serum, dual antibiotics and basal medium; 4) IL-1 beta, IL-2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15 , GM-CSF, a CD137 antibody, a PD-1 antibody, TNF-alpha, serum, dual antibiotics and basal medium; 7) IL-2, IL-4, IL-10, IL-15, G-CSF, M-CSF, CD28 antibody, OX-40 antibody, a PD-1 antibody, serum, dual antibiotics and basal medium; 8) IL-2, IL-7, IL-15, IFN-gamma, a CD137 antibody, a CD40 antibody, a OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 Antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basal medium; 12) IL-2, IL-7, IL-15, GM-CSF, M-CSF, a PD-1 antibody, CNI-1493, serum, dual antibiotics and basal medium; 13) IL-2, IL-7, IL-15, a CD137 antibody, a CD28 antibody, a LAG3 antibody, a PD-1 antibody, dasatinib, serum, dual antibiotics and basal medium; 14) IL-2, IL-6, IL-12, G-CSF, M-CSF, IFN-beta, IFN-gamma, a CTLA-4 antibody, a PD-1 antibody, dasatinib, LYC-55716, GENE-1858, serum, dual antibiotics and basal medium; 15) IL-1 alpha, IL-2, IL-9, IL-15, GM-CSF, a CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, dasatinib, GNE-1858, serum, dual antibiotics and basal medium,
d) the incubation of step (1) lasts at least 5 days,
e) the incubation of step (1) lasts at most 20 days,
f) the temperature of the incubation of step (1) is 30-42°C, preferably 37°C,
g) the concentration of CO₂ in the incubation of step (1) is 5%,
h) the incubation of step (2) lasts at least 5 days,
i) the incubation of step (2) lasts at most 20 days,
j) the temperature of the incubation of step (2) is 30-42°C, preferably 37°C,
k) the concentration of CO₂ in the incubation of step (2) is 5%,
l) the tumor cell-containing tissue is a patient with the group consisting of a gastric cancer, thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a brain cancer, a glioma, a pancreatic cancer, a bladder cancer, a prostate cancer, a renal cancer, an osteosarcoma, etc.

8. A method for preparing a population of TIL cell, comprising the following steps:
(1) incubating a tumor cell-containing tissue with a TIL seed cell culture medium to obtain the first population of TIL cell,
(2) contacting the first population of TILs described in (1) with any one or more of the antibodies coupled to a matrix in the culture composition described in any one of claims 1-3 to obtain a second population of TILs,
(3) culturing the second population of TILs described in (2) in a medium containing any one or more of the components in the culture composition according to any one of claims 1-3 except for the antibodies coupled to the matrix, to obtain the third population of TILs,
preferably, the method has one or more features selected from the group consisting of
a) the tumor tissue is pretreated, preferably, the pretreatment includes fragmentation and/or dissociation of the tumor tissue,
b) said tumor cell-containing tissue is a tumor tissue or body fluid of a subject having cancer,
c) the TIL seed cell culture medium described in step (1) includes any one of the combinations of the following components: 1) IL-2, IL-6, IL-21, IFN-gamma, TIGIT antibody, PD-1 antibody, TNF-alpha, serum, dual antibiotics and basal medium; 2) IL-2, IL-4, IL-10, IL-21, a CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, a PD-1 antibody, serum, dual antibiotics and basal medium; 4) IL-1 beta, IL -2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15 , GM-CSF, a CD137 antibody, a PD-1 antibody, TNF-alpha, serum, dual antibiotics and basal medium; 7) IL-2, IL-4, IL-10, IL-15, G-CSF, M-CSF, a CD28 antibody, a OX-40 antibody, a PD-1 antibody, serum, dual antibiotics and basal medium; 8) IL-2, IL-7, IL-15, IFN-gamma, a CD137 antibody, a CD40 antibody, OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 Antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basal medium; 12) IL-2, IL-7, IL-15, GM-CSF, M-CSF, PD-1 antibody, CNI-1493, serum, dual antibiotics and basal medium; 13) IL-2, IL-7, IL-15, a CD137 antibody, a CD28 antibody, a LAG3 antibody, a PD-1 antibody, dasatinib, serum, dual antibiotics and basal medium; 14) IL-2 , IL-6, IL-12, G-CSF, M-CSF, IFN-beta, IFN-gamma, a CTLA-4 antibody, a PD-1 antibody, dasatinib, LYC-55716, GENE-1858, serum, dual antibiotics and basal medium; 15) IL-1 alpha, IL-2, IL-9, IL-15, GM-CSF, CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, dasatinib, GNE-1858, serum, dual antibiotics and basal medium,
d) the incubation of step (1) lasts at least 5 days,
e) the incubation of step (1) lasts at most 20 days,
f) the temperature of the incubation of step (1) is 30-42°C, preferably 37°C,
g) the concentration of CO₂ in the incubation of step (1) is 5%,
h) the matrix described in step (2) is a multi-well plate, a cell culture dish or a cell culture bag,
i) the coupling described in step (2) is a covalent coupling or a non-covalent coupling,
j) the antibody coupled to the matrix in step (2) includes any one or more of a CD3 antibody, a CD28 antibody and a CD137 antibody; preferably, includes a CD3 antibody and a CD28 antibody; more preferably, includes a CD3 antibody, a CD28 antibody and a CD137 antibody,
k) the contacting in step (2) is to incubate the first population of TILs in step (1) with the antibodies coupled to the matrix; preferably, the temperature of the incubation is 30-42°C, preferably, is 37°C; preferably, the concentration of CO₂ of the incubation is 5%,
l) In step (2), the incubation is carried out in a culture medium containing any one or more of the components in the aforementioned culture composition except for the antibodies coupled to the matrix,
m) the density of the first population of TILs in step (2) is 1.0×10⁵/mL~1.0× 10⁶/mL; preferably is 2.0×10⁵/mL~1.0 × 10⁶/mL; more preferably is 2.5×10⁵/mL~3.5 ×10⁶/mL,
n) the contacting in step (2) lasts for 1-3 days; preferably, lasts for 2-3 days,
o) the incubation of step (3) lasts at least 5 days,
p) the incubation of step (3) lasts at most 20 days,
q) the temperature of the incubation of step (3) is 30-42°C, preferably 37°C,
r) the concentration of CO₂ in the incubation of step (3) is 5%,
s) the tumor cell-containing tissue is a tumor tissue or body fluid of a subject having a cancer selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a brain cancer, a glioma, a pancreatic cancer, a bladder cancer, a prostate cancer, a renal cancer, an osteosarcoma, etc.

9. A population of TILs obtained by the method of any one of claims 6-8.

10. The use of the population of TILs according to claim 9 in the manufacture of a medicament for treating cancer,
preferably, the cancer is selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a brain cancer, a glioma, a pancreatic cancer, a bladder cancer, a prostate cancer, a renal cancer, an osteosarcoma.

11. A pharmaceutical composition, comprises the population of TILs described in claim 9, and a pharmaceutically acceptable excipient.

12. A cell cryopreservation preparation, comprises the population of TILs according to claim 9 and a cryopreservation solution,
preferably, the cryopreservation solution is a cell cryopreservation solution or a tissue cryopreservation solution;
preferably, the cryopreservation solution is a serum-free cryopreservation solution.
